(19) Europäisches Patentamt
European Patent Office
Office européen des brevets

(11) EP 1 403 369 A1

(12) **EUROPEAN PATENT APPLICATION**
published in accordance with Art. 158(3) EPC

(43) Date of publication:
**31.03.2004 Bulletin 2004/14**

(21) Application number: **02778910.6**

(22) Date of filing: **27.05.2002**

(51) Int Cl.[7]: **C12N 15/09**, C12N 5/06, C12N 5/10, C12Q 1/68, G01N 33/53

(86) International application number:
**PCT/JP2002/005106**

(87) International publication number:
**WO 2002/101037 (19.12.2002 Gazette 2002/51)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.05.2001 JP 2001165954**

(71) Applicant: **FUSO PHARMACEUTICAL INDUSTRIES LTD.**
**Osaka-shi, Osaka 541-0045 (JP)**

(72) Inventors:
- **OHNO, Tsuneya**
**Tokyo 158-0081 (JP)**
- **MATSUHISA, Akio**
**Osaka-shi, Osaka 536-0025 (JP)**
- **KESHI, Hiroyuki**
**Osaka-shi, Osaka 558-0042 (JP)**
- **ABE, Kanako**
**Hirakata-shi, Osaka 573-0016 (JP)**
- **SUGIMOTO, Norihiko**
**Yao-shi, Osaka 581-0818 (JP)**
- **UEYAMA, Hiroshi**
**Osaka-shi, Osaka 536-0025 (JP)**
- **EDA, Soji**
**Kyoto-shi, Kyoto 607-8422 (JP)**
- **UEHARA, Hirotsugu**
**Takarazuka-shi, Hyogo 665-0873 (JP)**
- **IWAMI, Takahisa**
**Kaizuka-shi, Osaka 597-0031 (JP)**
- **YAMAMOTO, Seiji**
**Osaka-shi, Osaka 552-0021 (JP)**
- **ARAKI, Hiromasa**
**Yamatokoriyama-shi, Nara 639-1017 (JP)**

(74) Representative: **Gardner, Rebecca**
**Frank B. Dehn & Co.**
**179 Queen Victoria Street**
**London EC4V 4EL (GB)**

(54) **METHOD OF EVALUATING THE FUNCTION OF PHAGOCYTE AND UTILIZATION THEREOF**

(57) A digested phagocyte prepared by contacting *in vitro* a phagocyte with a foreign microorganism and isolating the phagocyte so contacted; a process for producing the same; and a process and a kit in which these are utilized are disclosed. An experimental model, which enables *in vitro* evaluation of a phagocytic function of phagocytes, is provided.

EP 1 403 369 A1

SEQUENCE LISTING

<110> FUSO PHARMACEUTICAL INDUSTRIES, LTD.

<120> Method for Evaluating Function by Phagocyte and Use Thereof

<130> 02P328WO

<150> JP 2001-165954
<151> 2001-05-31

<160> 19

<210> 1
<211> 10207
<212> DNA
<213> Staphylococcus aureus

<220>
<223> Designated as SA-24

<400> 1
```
aagcttatgg acctatttta ggtatattga ttagttggct tggattaatt tctggaacat     60
ttacagtcta tttgatctgt aaacgattgg tgaacactga gaggatgcag cgaattaaac    120
aacgtactgc tgttcaacgc ttgattagtt ttattgatcg ccaaggatta atcccattgt    180
ttattttact ttgttttcct tttacgccaa atacattaat aaattttgta gcgagtctat    240
ctcatattag acctaaatat tatttcattg ttttggcatc atcaaagtta gtttcaacaa    300
ttattttagg ttatttaggt aaggaaatta ctacaatttt aacgcatcct ttaagaggga    360
tattaatgtt agttgtgttg gttgtatttt ggattgttgg aaaaaagtta gaacagcatt    420
ttatgggatc gaaaaaggag tgacatcgtg aaaaaagttg taaaatattt gatttcattg    480
atacttgcta ttatcattgt actgttcgta caaacttttg taatagttgg tcatgtcatt    540
ccgaataatg atatgtcacc aacccttaac aaagggacgt gttattgtaa ataaaattaa    600
agttacattt aatcaattga ataatggtga tatcattaca tataggcgtg gtaacgagat    660
atatactagt cgaattattg ccaaacctgg tcaatcaatg gcgtttcgtc agggacaatt    720
ataccgtgat gaccgaccgg ttgacgcatc ttatgccaag aacagaaaaa ttaaagattt    780
tagtttgcgc aattttaaag aattagatgg agatattata ccgcctaaca attttgttgt    840
gctaaatgat catgataaca atcagcatga ttctagacaa tttggtttaa ttgataaaaa    900
```

```
ggatattatt ggtaatataa gtttgagata ttatcctttt tcaaaatgga cgattcagtt   960
caaatcttaa aaagaggtgt caaaattgaa aaaagaatta ttggaatgga ttatttcaat  1020
tgcagtcgct tttgtcattt tatttatagt aggtaaattt attgttacac catatacaat  1080
taaaggtgaa tcaatggatc caactttgaa agatggcgag cgagtagctg taaacattat  1140
tggatataaa acaggtggtt tggaaaaagg taatgtagtt gtcttccatg caaacaaaaa  1200
tgatgactat gttaaacgtg tcatcggtgt tcctggtgat aaagtagaat ataaaaatga  1260
tacattatat gtcaatggta aaaaacaaga tgaaccatat ttaaactata atttaaaaca  1320
taaacaaggt gattacatta ctgggacttt ccaagttaaa gatttaccga atgcgaatcc  1380
taaatcaaat gtcattccaa aaggtaaata tttagttctt ggagataatc gtgaagtaag  1440
taaagatagc cgtgcgtttg gcctcattga tgaagaccaa attgttggta aagtttcatt  1500
tagattctgg ccatttagtg aatttaaaca taatttcaat cctgaaaata ctaaaaatta  1560
atatgaaaca aatacaacat cgtttgtcgg ttttaatact gataaacgat gttttatttt  1620
gttagtacca caataaaagc taagttcgaa atgaacttat aataaatcaa tcacaatcac  1680
tttgtgttaa aatatgtgtc aaaggaagtg agggtttgtc atgacattac atgcttattt  1740
aggtagagcg ggaacaggta agtctacgaa aatgttgacc gaaataaaac aaaaaatgaa  1800
agcagatccg cttggagatc caatcatttt aattgcgcca actcaaagta catttcaatt  1860
agaacaagcc tttgtcaatg atccggaatt aaatggtagt ttaagaacag aagtgttgca  1920
ttttgaacga ttaagtcatc gtattttcca agaagttggt agttatagcg aacaaaagtt  1980
atctaaagct gcaacggaaa tgatgattta taacattgtt caagaacaac aaaagtattt  2040
aaaactttat caatcacaag caaaatatta tgggtttagt gaaaaattaa cagaacaaat  2100
tcaagatttt aaaaaatatg cagtaacgcc tgaacattta gaacacttta ttgctgataa  2160
aaatatgcaa actcgaacta aaaataagtt agaggatatt gctttaatat accgtgagtt  2220
cgaacaacgc attcaaaacg agtttattac tggtgaggat tcattacaat attttattga  2280
ttgtatgccg aaatcagagt ggctaaaacg tgctgatata tatattgatg gttttcacaa  2340
cttttcaacg attgagtatt taataatcaa aggattaatt aaatatgcga gagtgtcaca  2400
attatattga cgacagatgg taaccacgat caatttagtt ttttagaaaa ccatcggaag  2460
tgttacgaca tattgaagaa atagcaaatg aactcaatat ttctattgaa cgtcaatatt  2520
tcaaccaatt atatcgcttc aataatcaag atttaaagca tcttgaacaa gaatttgatg  2580
tacttcaaat caatcgagtg gcatgtcaag gtcatatcaa tattttagaa tctgcgacta  2640
tgagagagga aataaatgaa attgcgcgac gtatcatcgt tgatattcgt gataagcaat  2700
tacgatatca agatattgca attttatatc gtgacgagtc ttatgcttat ttatttgatt  2760
ccatattacc gctttataat attccttata acattgatac aaagcgttcg atgacacatc  2820
atccggtcat ggaaatgatt cgttcattga ttgaagttat tcaatctaat tggcaagtga  2880
atccaatgct acgcttattg aagactgatg tgttaacggc atcatatcta aaaagtgcat  2940
acttagttga tttacttgaa aattttgtac ttgaacgtgg tatatacggt aaacgttggt  3000
tagatgatga gctatttaat gtcgaacatt ttagcaaaat ggggcgtaaa gcgcataaac  3060
tgaccgaaga tgaacgtaac acatttgaac aagtcgttaa gttaaagaaa gatgtcattg  3120
```

ataaaatttt acattttgaa aagcaaatgt cacaagcgga aactgtaaaa gactttgcaa 3180
ctgcttttta tgaaagtatg gaatatttcg aactgccaaa tcaattgatg acagagcgag 3240
atgaacttga tttaaatggt aatcatgaaa aggcggagga aattgatcaa atatggaatg 3300
gcttaattca aatccttgac gacttagttc tagtatttgg agatgaacca atgtcgatgg 3360
aacgtttctt agaagtattt gatattggtt tagaacaatt agaatttgtc atgattccac 3420
aaacattaga tcaagttagt attggtacga tggatttggc taaagtcgac aataagcaac 3480
atgtttactt agttggaatg aacgacggca ccatgccaca accagtaact gcatcaagtt 3540
taattactga tgaagaaaag aaatattttg aacaacaagc aaatgtagag ttgagtccta 3600
catcagatat tttacagatg gatgaagcat ttgtttgcta tgttgctatg actagagcta 3660
agggagatgt tacattttct tacagtctaa tgggatcaag tggtgatgat aaggagatca 3720
gcccattttt aaatcaaatt caatcattgt tcaaccaatt ggaaattact aacattcctc 3780
aataccatga agttaaccca ttgtcactaa tgcaacatgc taagcaaacc aaaattacat 3840
tatttgaagc attgcgtgct tggttagatg atgaaattgt ggctgatagt tggttagatg 3900
cttatcaagt aattagagat agcgatcatt taaatcaagg tttagattat ttaatgtcag 3960
cattaacgtt tgacaatgaa actgtaaaat taggtgaaac gttgtctaaa gatttatatg 4020
gtaaggaaat caatgccagt gtatctcgtt ttgaaggtta tcaacaatgc ccatttaaac 4080
actatgcttc acatggtctg aaactaaatg aacgaacgaa atatgaactt caaaactttg 4140
atttaggtga tattttccat tccgttttaa aatatatatc tgaacgtatt aatggcgatt 4200
ttaaacaatt agacctgaaa aaaataagac aattaacgaa tgaagcattg gaagaaattt 4260
tacctaaagt tcagtttaat ttattaaatt cttcagctta ctatcgttat ttatcaagac 4320
gcattggcgc tattgtagaa acaacactaa gcgcattaaa atatcaaggc acgtattcaa 4380
agtttatgcc aaaacatttt gagacaagtt ttagaaggaa accaagaacc aaatgtacga 4440
attaattgca caaacattaa cgacaactca aggtattcca attaatatta gagggcaaat 4500
tgaccgtatc gatacgtata caaagaatga tacaagtttt gttaatatca ttgactataa 4560
atcctctgaa ggtagtgcga cacttgattt aacgaaagta tattatggta tgcaaatgca 4620
aatgatgaca tacatggata tcgttttaca aaataaacaa cgccttggat taacagatat 4680
tgtgaaacca ggtggattat tatacttcca tgtacatgaa cctagaatta aatttaaatc 4740
atggtctgat attgatgaag ataaactaga acaagattta attaaaaagt ttaagctgag 4800
tggtttagtg aatgcagacc aaactgttat tgatgcattg gatattcgtt tagaacctaa 4860
attcacttca gatattgtac cagttggttt gaataaagat ggctctttga gtaaacgagg 4920
cagccaagtg gcagatgaag caacaattta taaattcatt cagcataaca aagagaattt 4980
tatagaaaca gcttcaaata ttatggatgg acatactgaa gtgcaccatt aaagtacaaa 5040
caaaaattgc catgtgcttt ttgtagttat caatcggtat gtcatgtaga tggcatgatt 5100
gatagtaagc gatatcgaac tgtagatgaa acaataaatc caattgaagc aattcaaaat 5160
attaacatta atgatgaatt tgggggtgag taatagatga caattccaga gaaaccacaa 5220
ggcgtgattt ggactgacgc gcaatggcaa agtatttacg caactggaca agatgtactt 5280
gttgcagccg cggcaggttc aggtaaaaca gctgtactag ttgagcgtat tatccaaaag 5340

attttacgtg atggcattga tgtcgatcga cttttagtcg taacgtttac aaacttaagc 5400
gcacgtgaaa tgaagcatcg tgtagaccaa cgtattcaag aggcatcgat tgctgatcct 5460
gcaaatgcac acttgaaaaa ccaacgcatc aaaattcatc aagcacaaat atctacactt 5520
catagttttt gcttgaaatt aattcaacag cattatgatg tattaaatat tgacccgaac 5580
tttagaacaa gcagtgaagc tgaaaatatt ttattattag aacaaacgat agatgaggtc 5640
atagaacaac attacgatat ccttgatcct gcttttattg aattaacaga acaattgtct 5700
tcagatagaa gtgatgatca gtttcgaatg attattaaac aattgtattt ctttagcgtt 5760
gcaaatccaa atcctacaaa ttggttggat caattggtga caccatacga agaagaagca 5820
caacaagcgc aacttattca actactaaca gacttatcta aagtatttat cacagctgcc 5880
tatgatgctt taaataaggc gtatgatttg tttagtatga tggatggcgt cgataaacat 5940
ttagctgtta tagaagatga acgacgttta atggggcgtg ttttagaagg tggttttatt 6000
gatatacctt atttaactga tcacgaattt ggcgcgcgtt tgcctaatgt aacagcgaaa 6060
attaaagaag caaatgaaat gatggtcgat gccttagaag atgctaaact tcagtataaa 6120
aaatataaat cattaattga taaagtgaaa aatgattact tttcaagaga agctgatgat 6180
ttgaaagctg atatgcaaca attggcgcca cgagtaaagt accttgcgcg tattgtgaaa 6240
gatgttatgt cagaattcaa tcgaaaaaag cgtagcaaaa atattctgga tttttctgat 6300
tatgaacaat ttgcattaca aattttaact aatgaggatg gttcgccttc agaaattgcc 6360
gaatcatacc gtcaacactt tcaagaaata ttggtcgatg agtatcaaga tacgaaccgg 6420
gttcaagaga aaatactatc ttgcatcaaa acgggtgatg aacataatgg taatttattt 6480
atggttggag atgttaagca atccatttat aaatttagac aagctgatcc aagtttattt 6540
attgaaaagt atcaacgctt tactatagat ggagatggca ctggacgtcg aattgatttg 6600
tcgcaaaact ccgttctcga aaagaagtac tgtcaacgac taactatata tcaaacatat 6660
gatggatgaa caagtcggtg aagtaaaata tgatgaagcg gcacagttgt attatggtgc 6720
accatatgat gaatcggacc atccagtaaa cttaaaagtg cttgttgaag cggatcaaga 6780
acatagtgat ttaactggta gtgaacaaga agcgcatttt atagtagaac aagttaaaga 6840
tatcttagaa catcaaaaag tttatgatat gaaaacagga agctatagaa gtgcgacata 6900
caaagatatc gttattctag aacgcagctt tggacaagct cgcaatttac aacaagcctt 6960
taaaaatgaa gatattccat tccatgtgaa tagtcgtgaa ggttactttg aacaaacaga 7020
agtccgctta gtattatcat ttttaagagc gatagataat ccattacaag atatttattt 7080
agttgggtta atgcgctccg ttatatatca gttcaaagaa gacgaattag ctcaaattag 7140
aatattgagt caaatgatga ctacttctat caatcgattg taaattacat taatgacgaa 7200
gcagcagatg ctattttagt tgataaatta aaaatgtttt tatcagatat tcaaagttac 7260
caacaatata gtaaagatca tccggtgtat cagttaattg ataaatttta taatgatcat 7320
tatgttattc aatactttag tggacttatt ggtggacgtg gacgacgtgc aaacctttat 7380
ggtttattta ataaagctat cgagtttgag aattcaagtt ttagaggttt atatcaattt 7440
attcgtttta tcgatgaatt gattgaaaga ggcaaagatt ttggtgagga aatgtagtt 7500
ggtccaaacg ataatgttgt tagaatgatg acaattcata gtagtaaagg tctagagttt 7560

ccatttgtca tttattctgg attgtcaaaa gattttaata aacgtgattt gaaacaacca 7620
gttattttaa atcagcaatt tggtctcgga atggattatt ttgatgtgga taaagaaatg 7680
gcatttccat ctttagcttc ggttgcatat aaagctgttg ccgaaaaaga acttgtgtca 7740
gaagaaatgc gattagtcta tgtagcatta acaagagcga aagaacaact ttatttaatt 7800
ggtagagtga aaaattgata aatcgttact agaactagag caattgtcta tttctggtga 7860
gcacattgct gtcaatgaac gattaacttc accaaatccg ttccatctta tttatagtat 7920
tttatctaaa catcaatctg cgtcaattcc agatgattta aaatttgaaa aagatatagc 7980
acaagttgaa gatagtagtc gtccgaatgt aaatatttca attatatact ttgaagatgt 8040
gtctacagaa accattttag ataataatga atatcgttcg gttaatcaat tagaaactat 8100
gcaaaatggt aatgaggatg ttaaagcaca aattaaacac caacttgatt atcaatatcc 8160
atatgtaaat gatactaaaa agccatccaa aacaatctgt ttctgaattg aaaaggcaat 8220
atgaaagaag aaagtggcac aagttacgaa cgagtaagac aatatcgtat cggttttcaa 8280
cgtatgaacg acctaaattt ctaagtgaac aaggtaaacg aaaaagcgaa ttgaaattgg 8340
tacgttaatg catacagtga tgcaacattt accattcaaa aaagaacgca tatctgaagt 8400
tgagttacat cagtatatcg atggattaat cgataaacat attatcgaag cagatgcgaa 8460
aaaagatatc cgtatggatg aaataatgac attatcaata gtgagtatat tcgattattg 8520
ctgaagcaga gcaagtttat cgtgaattac cgtttgtagt taaccaagca ttagttgacc 8580
aattgccaca aggagacgaa gacgtctcaa ttattcaagg tatgattgac ttaatctttg 8640
ttaaagatgg tgtgcattat tttgtagact ataaaaccga tgcatttaat cgtcgccgtg 8700
ggatgacaga tgaagaaatt ggtacacaat taaaaaataa atataagata cagatgaaat 8760
attatcaaaa tacgcttcaa acgatactta ataaagaagt taaaggttat ttatacttct 8820
tcaaatttgg tacattgcaa ctgtagtatt ttgattttca aaagaataaa aaataatttc 8880
gattaagtgc aaagtccttg tagcagaatg aacacaactc attttcaaaa ttgtcttact 8940
tatttatttg ttatttgata acgaaaaaag ttataatgtg aattaagata aagatgagga 9000
gttgagaatg aatgaaattc ttatcattca agtataatga caaaacttca tatggcgtta 9060
aagtaaaacg cgaagatgct gtatgggatt taacacaagt atttgctgac tttgcagaag 9120
gagatttcca tcctaaaaca ttgttagctg gtttacaaca aaatcatact ttagattttc 9180
aagaacaagt acgtaaagca gttgtagcag cagaagatag cggcaaagct gaagactata 9240
aaatttcatt taatgacatt gaattcttac caccagtaac acctccgaat aatgtgattg 9300
cttttggtag aaattacaaa gatcatgcga acgaattaaa tcatgaagta gaaaaattat 9360
atgtatttac aaaagcagcg tcatctttaa caggagataa tgcaacaatt ccaaatcata 9420
aagatattac tgatcaatta gattatgaag gtgaattagg tattgttatt ggtaagtctg 9480
gtgaaaagat tccaaaagca ttagctttag attatgttta cggctataca attattaacg 9540
atatcactga tcgcaaagca caaagtgaac aagatcaagc atttttatca aaaagtttaa 9600
ctggcggttg cccaatgggt ccttatatcg ttactaaaga cgaactacca ttacctgaaa 9660
atgtaaatat tgttacaaaa gttaacaatg aaattagaca agatggtaac actggcgaaa 9720
tgattcttaa aattgatgaa ttaatagaag aaatttcaaa atatgttgca ctactaccgg 9780

gagattatta ttgcaactgg tacaccagct ggcgttggtg caggtatgca accacctaaa 9840
tttttacaac caggtgatga agttaaagtg actattgata atattggaac gctgacaact 9900
tatatcgcta aataattatc atttaaaaag ctaaccaggt ctttatatag attggttagt 9960
tttttcttgc ttttctaaaa aggtgttaaa gataaattat ttataatgtt accattttga 10020
gatgaaagtg aaatattgat attaagaagt agttgattat tttacagcag attcacaata 10080
ttctaataag ggcaatgcaa atgtcatgtt cttcctctca aatatagaag tgtggtagaa 10140
tatatattcg tgtataatca aatctagatt aaattacaag caagtgggta ttaatcccaa 10200
gaagctt 10207

<210> 2
<211> 2082
<212> DNA
<213> Staphylococcus aureus

<220>
<223> Designated as SA-36

<400> 2
aagctttcta atctatcgtt aatgatttgc tttaaaattg ggtcgaagtt aattgaaggt 60
gtgaagtgta tatctgtatt aataaccatg tcattcattt gctgcttcac tttgttaaca 120
agtcttccgt catataaaaa taatggtacg acaatcaatt tttgataccg tttcgagatg 180
ctttctaaat catgtgtaaa actaatctct ccatatagcg ttctcgcata agtaggttta 240
ttaatctgca aatgttgagc gcatatttgt aactcttcgt gtgccttagt aaaatttcca 300
ttaatattgc cgtgtgcaac aaccataact ccaacttgtt gttcgtcacc tgctaatgcg 360
tcacaaatac gttgttcaat taatcgtctc attaaaggat gtgtgccaag tggctcgctt 420
acttctacct ttatgtctgg ataccgtcgt ttcatttcat gaacgatatt cggtatatcc 480
ttgagataat gcattgcact aaagattagc aatggtacaa ttttaaaatg gtcaacccca 540
ctttgaatca acgtcgtcat taccgtctct aaatcctgat gctcactttc taaaaacgca 600
atatcatagt gatgtatatc atcttttact aattcagaaa taaatgcttc taacgcttga 660
ttctgtcgtc cgtgcctcat gccatgtgca acaatgatat tcccattcac atttaccaac 720
cctttcacac gtattgtata ccaaatcatt ttgtttttgt gaaaagaatc acattataat 780
gtaaaatcag ggaattccct gatgcctgta gtcatgcata ttccttatac attttccctt 840
tttgttaaat caaaaaaagc gaccgatata tgaatcccta ctcaacattt atttgagcaa 900
gcatcaatat atcggtcgct tgtagtgtat attattatct taaaatggtg gttggcctaa 960
tattgtttcg tcaaagcgct cgggtatcaa tactttgcgc atgatcacac ctaaatcgcc 1020
atcatcattt tcatgttcgc tgtatatttc ataacctctt ttttcataaa ttttaagtaa 1080
ccacggatgc aatcttgcag atgtacctaa agtaactgcc gctgacttta acgtatctcg 1140

caaaaatgct cttcaacata agtaagtaat tggctaccat agcctttccc ttcatactca 1200
ggatttgtcg caaaccacca gacaaaagga tagcccgaaa tacttttcac acttccçcaa 1260
ggatatctaa ccgtaatcgt agatataatt tcatcatcaa ttgtcatgac aaatgtagta 1320
tttttatcta tattttcttt aacagcatct aaattagcat taactgaagg ccaatcaata 1380
cctagttctc ttagaggcgt aaatgcttca tgcatgagtt gttgcaattt ttctgcatct 1440
tgttcacttg cgagtcgaat catcgttttt gtcatattaa tccccactct tttttaaatg 1500
atttaaccat attttatttt taaaataaat atccatcaaa gtgtatcaat aaatttatca 1560
catgtcagaa agtatgcttc atctgaatac accaatactc tcatgaaact tattaaaaat 1620
tactctctca acgtaaaaaa accattcaaa ttcatgaatg gtttggaaga atgattcatt 1680
gttacgctat ttaatcacta catcttaatt attgttgctc taaacgatta cgcttaccat 1740
ttaagaaagc ataaacgaga cctacaaaaa taccgccacc gacaaagtta cctaagaaag 1800
caaaaacgat atttttaaa acatgtaacc atgaaactgc atcaaggtta aagaatacca 1860
tacctgcata tagacctgca ttgaacacaa cgtgctcata tcccatgtat acaaagacca 1920
cgacaccaca agctatgaag aatgcctttg ttaagccgcc tttgaattgc atagagatga 1980
aaataccaat attaataaag aagttacaga aaataccttt tgtaaaaata ttcaaccatg 2040
ttgaatcaac agtctttttc tgaactaaag ctgttaaagc tt 2082

<210> 3
<211> 2885
<212> DNA
<213> Staphylococcus aureus

<220>
<223> Designated as SA-77

<400> 3
aagcttttga ttaatttggg ctttaaagta ttcccaatta taattcttca tgattttctt 60
attggatttc gaatttggtt tcatgcattg ttgcctcaaa gaacatgctg aacagtcatc 120
gcattcatat agcttgaagt cacgtttaaa accatatcta tcattacggt atgcatatct 180
tttaaaacct attcttttgt tattaggaca tataaattca tcattaagtt cgtcatattt 240
ccaattttga gtgttaaaaa tgtcactttt aaactttcta gttttatctt taataaacat 300
gccatacgta ataagtggcg ttttattaaa acatctataa tagccatata gttttgctca 360
ctatcataac tgcatcagct acattaactc tggtaatacc gaggatttga atcattgtta 420
aaaatggaat taaagttcta gtatctgttg gggtttgaaa taggtcatag gataaaaaaa 480
ttgagaattt gtcgctattt gtaaattgta tcctggctta agttggccat ttttcatatg 540
gtcttccttc attctcataa aagttgcatc atgatcagcc cagaaagcta tttctatctt 600
taagaatcca tttttgttct tcatatttat tttttctttc ggaataatca tcaaatttct 660

```
ttttgaactt cttaatctca gttctttttt acgggtctgt tttctaattt gagcactctt    720
cgttctaaat agaatgattt aaatcttcga tttcttttat ctaaatgact accaattaaa    780
tctatttctt ctcgtgattt tgaatacttt tcttccacac aaatgtatat ctattggcat    840
tagcttctac ttatgtacca tcaataaaaa ttgaattatt atcaataaga ttttgcttta    900
aacattgact atggaactga ataaataaag attcaattaa cgcatcagta ttaggattca    960
ctctaaaacg attaatagtt ttataagaag gtgtttgatc ttgagctaac cacatcattc   1020
gaatactgtc atgaagtaat ttctctattc tacgaccaga aaatacagat tgagtatatg   1080
catataagat gatttttaac atcattttg gatgatagga tgttgcgcca cgatgatgtc   1140
tgaattcatc gaattcgcta tcaggtatcg tttcaacaat ttcatttaca tatcgcgaaa   1200
tatcatttta aggaattcta acagaagttt ctattggtag tgtaagttgg gcaaagtgtc   1260
ttattttttt aaagtatgta aaagtaaaat tacatgttaa tacgtagtat taatggcgag   1320
actcctgagg gagcagtgcc agtcgaagac cgaggctgag acggcaccct aggaaagcga   1380
agcattcaat acgaagtatt gtataaatag agaacagcag taagatattt tctaattgaa   1440
aattatctta ctgctgtttt tttagggatt tatgtcccag cctgttttat tttcgactag   1500
tttggagaat ttattgacat tcacattatt taaacggcaa caaagattgt tttattttga   1560
taggcattat atggtgttaa aaaatttgca tgaaaattaa aaaatgcttc gttcaggaag   1620
gtgtcgtaat ttacctattt gctgaatgaa gcattttatt tttaaatatg atagccaata   1680
taacaagcta taaatccaat gatgaattgt aaaagtgaat aattgagaaa aaggttaata   1740
tcaaattttg gtgtcatcat taatgtaagt tccttggcta acgttgagaa agttgttaag   1800
ccacctaaaa aaaccggtga caaagaacgc agggaaccat gagattgaaa ttgataggcc   1860
tatagttaat ccaattaaaa aactaccaac tagatttact atcaatgttg cgataggtaa   1920
ctttgaagta aatttatgat taaaataatc agtaatggca cttctagcaa ttgcgccaaa   1980
accgccgcca atcatgacta aaatgattga tatcatgata aaccaccacc tagttttata   2040
ccgacgtaac ataacaaaat accaaagaca taacttgtta cagcatatag tagtaaagtt   2100
ataaattgtt gatgatcaaa catatgtatt aattctaatt gaaatgttga aaaagtcgtt   2160
aaagcaccaa gaaaaccagt cgtaatagct ttttttaggg tcggatggtt tgaaaaaaat   2220
gcaattgtta aggctgttag caatcccatt acaaaggcac cagtcaaatt ggctatcagt   2280
gttccgattg gaaaacctcc gtcagtattc agaaaagaaa tgaggtaacg taataaagcg   2340
cctaaagcac caccgataaa aatatataca tattgcattt ggttcacctc gaaaagaagt   2400
agtttgaatt taaaaaagag gttttggcaa cacgacgaca aaaattgtcg atgcattatc   2460
aaacctcatt atatgttata tcttgttgta taactatagc gattagatgc atagttatga   2520
tttcgaaaat ctaatatttt ttatacgcaa caacgtcatc aaattgtttt actcattata   2580
gcatgataca ttgtattgtt ttgtattaac gctacattga cattttatct tttttaaata   2640
aaaccgaatg tacgacaatt gaaaagatat gtactaaaat aacaattaga ataatccaag   2700
gcaaactttt actcgcaatt ctaatccaat ctgcatcagg ctttagtgat ttaattgaac   2760
gatctgcaaa aattatagac aaaattagta caattgagtt aataacactg cagaaaagta   2820
ttaatttaat aaaagaatta aaaaatccac ttaggaaaac gttatttgta ttaaagaaaa   2880
```

agctt 2885

<210> 4

<211> 8654

<212> DNA

<213> Staphylococcus epidermidis

<220>

<223> Designated as SE-22

<400> 4

aagcttgttt tattgcttag ttatatttcc aataacactc attttatatg tacgtattgc 60
caaaaaaaat tatctataca gtaataagta tgaaatgaga actggaataa tcattggtat 120
tattgcttta attctagtaa ttatgcaagg gtttcacttt aactgggcta ttattcctat 180
ttctatctat ggtcatcagt ttgtattttt cgctggaatt attttaagtc ttgttggtat 240
attctttaaa cgtatagaat ttgtaggagt tggcttacta ttttgtcaaa aacatagatg 300
caatggtaac tgacccggaa attgcacagt ttttctcttt agcaatttgg attatacttg 360
ttgtgctaat catttttat acgatacgtt tatctgaacg cactaaatca tcatcatata 420
caaagattta aactcagaaa atatgctaga catatctttc tgagtttttt aatttattaa 480
aatatatcat ttgtttacca tataagtttg ttttagaaaa tgaatcacta ttttaatata 540
caaataattt aattacactg aaaataacct aaaagcgtaa cactatttta atatgggtat 600
ataaatgact aaagggaggt gccaagatga ataaaattca aatttgtaat cagattgaac 660
ttaactatat tgatgaaggc gaaggcatcc ccatcatttt aattcatgga ttagatggaa 720
acttggcagg atttaaagat ttaaaaaatg aactcaagaa gcagtataga gtaattactt 780
atgatgtcag aggtcatgga aaatcttcac gaacagaatc atatgaatta aaagatcatg 840
ttgaagattt aaatgattta atgggagcat taaatatcga ttctgcacat attttaggac 900
atgatatggg gggcatcatt gcgagtgaat ttactgaaaa atatcaatat aaagtgatta 960
cattgacaat tgtttcggcc aaaagtgaag acattgcaaa tggtttcaac aaattaatgg 1020
ttgattacca agaagaatta gcaggcttta ataaatctga ggcaatgatt attttattct 1080
ctaaattatt taaagagaaa gataaagcaa tgaaatgggt atcaaagcca aaaattatac 1140
aatagaccaa ctccggaaga aagtgcaatt gcagtacgtg cattgcttaa tattaaagat 1200
ttaactcgtg ttcatcataa tgtgtccata cctactttaa ttgtgaatgg taagtatgac 1260
ccactcatac aaaataaaag tcattatgat atggatcaat attatgatca agttacaaaa 1320
attgtatttg ataattcagg acatgcacca catatcgagg aaccagaaaa attcctgaaa 1380
ctctacttag attttgttag ttaaaaaata agaacataaa taaaaaccct taaatgatta 1440
ttgtcggaaa atcatttgag ggttttgtag tagcagtaaa gtttggactc agatcactat 1500
cgtattaact taataaaaga gtaaaacagt cttatctttc ataagtgaaa gaaatatctg 1560

tttnactccc tagccattat acttcatttc attatttgct tctgtgatac ggttgtttac 1620
tcgtttaagt aaatcatcga tttttttacg ctgcttagaa tctactaaga ttaaaacagt 1680
tctttcatcg tgttcattac gtttttttatt aaagtaattt tcttgagata aatttttaac 1740
agctttaaca acttgaggtt gtttataatt taagtgattg ataatatctt taagataata 1800
ttcctcttct ttattctcac taatataagt taatactgca aattcttcaa agctgattga 1860
gaattctttt ttaattattc cttttaatct gtcagcataa gtgaccatag ctaataattc 1920
aaagcagtca ttgatttttg aaatagccat taatgaaacc tccctattta tatcatatcc 1980
ataaatctta aaacccatct ttttaaattt aaagatagtt aattatatta ttgaattaag 2040
attacttgga tactataccc taatttatta atttatatct atttttctta tgaaaatacg 2100
aaagtgtccg tcataatata gtattaattt aaatttaaag aatatattta atgctatatt 2160
atttagttaa ttataactaa ataaaattaa gaagtaaaca aataagtgtt tataaaacaa 2220
attatctttt aaagtttata cttgaattag caatgtagca tttgctatat tcaaaaaaat 2280
aagattgttt ctaattttcc ttaatttaat aaaaattata ctaaaaagaa tactttttgg 2340
aaagaatttt actaacattt tttatatata aatgtttatt aatttagaag taggattttt 2400
aacaactttt tcatctatca ataagccttt agttatatta atatacccac tttttaaact 2460
ctttttgtat gttacttctc tttttgtaga attaaaacat agcgtttttg aacaatagct 2520
gacgtaggta actctatgtc atttgaggct aatttgattt taaagtgtgt tccaatttga 2580
tgattgggtt gtgtagaaag taaaatgtcg taatatgaga cgccattttt tatttttgat 2640
ggtatattcg aaatttcttt aattttacta gtaaattgag tgttgtcact agatgttaca 2700
gaaatatttt gatttatttt taataaattc aactcagatt ctgatatatt agcacgaata 2760
atacgttcgt tgctattaat ttgcactatc ttttcgtttg gttttgaagg gatagaatta 2820
atatatgaaa tacttccatt aattggtgaa aataaagtgg atttaattga ggatttagtt 2880
tgaatcattt gtaattttag ctgattaagg aatgaataat aatgtaaatc atttttagaa 2940
tttaaagttt tgttgttacg ttcattacta agtgtatttt ggagttcctc atataaatga 3000
tctttttcat aattgtaata ttctaacact ggagtgtttt tagatacttt gctatgattt 3060
tttactaaaa gtttttggag ttgtcctaaa gtgggagtgt agtagaaaat atagctgtta 3120
agaggggctt gtataccagt tgttgaaagg agtaatttgg gctttgcttt tatagttttt 3180
atatttttaa tatcttctgt tttagaagtt aatttagaga aagtaatgta actaaaacta 3240
caagttgtga gaatgaaaat gaatagtaat gaagaaataa cgatgcgttg cttggtcatg 3300
gatgttcacc tcataatatt attgtgaggt tattatacac tattatttta aatgaaatat 3360
attaatttta aataagcatt acttttggtt tgtatattgt tttatttcaa aaaataaagt 3420
aaatcaattt aataaattga aaaatagaag gctatcttta attttaaaat atatgattct 3480
acataaatgt tactataaga agaatcactc ataaaaactg ccaacaaaga caaaatcttt 3540
gttggcagtt cgaaatagac atttatttgt atgaggaatc tacattaata taagcggata 3600
atttttattc agaataagga atttaaaata atcgtaataa aataatacct atagctatac 3660
ataataatcc acctaactta cgtgatgtta ttttgttttt aggtgaaccc aacaaaccga 3720
aatgatcgat aataataccc ataatcattt ggcccatcat agcaattata gtagttaaag 3780

ctgctcctaa gaaaggcatt aaaataatat tagatgttac gaatgccatt cctagtatcc 3840
ctccaataaa ataaatagat ttaatcttac ctagtgtttt atgagtagat gatattttca 3900
gactacgatt aaatactaat gttaatataa ataacgctat tgtaccaacg ctaaatgata 3960
tgagtgaagc aaatatggat gagtgtgtgt gttgagccag tgtgctgttg attgttgttt 4020
ggattggcgg acgaaaccaa atacgaatcc aataagcaac cagaatacta ttggtgtatt 4080
cttatgtcta ttaacaggat gtctacgaac ataattcata aatataattc cagtaattaa 4140
aaatataatt ccaacacctt taaataatgt aaaagattgt tgatgggcgc ccaataatcc 4200
aaatgtatca atgattacac ccataataat ttgccctgta accgtaataa caacagtaag 4260
tgctgcgcct aatcttggta ataataataa gtttccagtt aaatagataa cacctaatag 4320
tcctcctagg acccaagtat agttaagtgt ttgcttagaa aagaattctg gtgttaatac 4380
ttgtggatga ataatgatat taagcacaag taagcatatt gttccgacag caaaagatat 4440
ggttgaagca taaaaagatg aacgggtaaa ttggcttagc cttgagttga ttgaagtttg 4500
aataggaagt aacatgccaa caaaaattcc taaaagatat agaaaaaaca atgataaaaa 4560
ccaactttct caatttaata tgattatcat accattcata atcatgtttc taaaatgatt 4620
gagccataag caaagtatag aaataagttg tgaatgttcc gaggtgtcat acagccgata 4680
ctattttgat gaatcattat aataaaatgc acattaaaca agttttagaa ttaaaaaaag 4740
cgagacatca ttttgaattt gatatctcac ttcatattaa taaaagaaca atgtaaatta 4800
agttcttttt tagacttgaa caattttaaa aaatttgttc ttcgataagt ctttttttatg 4860
atttagtac tttaaataaa gcgtcaaaaa taatgtttta tgaattaatt tttatcttca 4920
aatataacag ttgtcctttt atcaataagt tgtgcagcat aaattttgac aggctttccc 4980
aaactaaatc ttaaaatgtc taattctaaa atgtctaatt ctaaaagttg gttcatactt 5040
tctttaatta attgttctgt agtaatagcg ttaaaatcgg gtaatagtaa tttgacgggt 5100
ttattaagat ttgatttaaa tacgagttcc aaagtttttg acatactgat gtatcctcct 5160
taaattaaag attctgtttt aacgatctcg actttgtcat actcttcgcc actgaacgtt 5220
caatgatgga acgaaaagat ttgatttgat cattagaaac aagcggatta atgttagaaa 5280
aacgacgctt atgttcgact actttacctt cagaattatg tttgatttga gtaaagataa 5340
tcgtcacttg attgacttca ttcataataa aacctccttt cactatatat atcgaaatag 5400
attgaaaaaa aaggacacat tttttgaaaa atataggcaa atgcctttga tgtgatacaa 5460
acgtcattta tcattaatta tgaaacctgt tttagaaggt atatgaggta agtagaattg 5520
ttaagttgta aaagaaaaaa ttggaacctg atatttaaaa taaccaactt aaaagattga 5580
tcagtgtcta aaattactat ttatatatga attaaaatat taagatctcc caatatgaga 5640
atgaattagt ttaagtttat cgatgattga aaaattatag cctcatggat tctatcttat 5700
ataaaataaa gttctattcc cttttggata taaataagaa tagttacctt tttgtgatat 5760
gccaattcag aaaaaaagcg acagtgcttg aatctatgta tgctcaataa actcattcaa 5820
atcaactagc aatatcaaat cataaatcgt gttgcaccat aataaggatt aaaacctgtt 5880
agtttaacta atttaagaaa aacatttgat tatcttctct ttcaatcggg aatattaatt 5940
tctatcattc aacaatattt tggatatcag ataacttaag aaatattgag atttattgaa 6000

atacgatatg tttcaaatcg ccatacaatg attacactta ataaatgatt acacttaata 6060
taaatgtaaa aagaaaagga ggggttaaat gagtttagta tatcttatgg cgactaattt 6120
attagtcatg ctcatagttt tattcactct gagtcatcgt caactaagaa aggttgcggg 6180
ctatgttgca ttaatagctc ctattgtgac atctacatat tttattatga aaataccaga 6240
tgtgattcga aataagttta ttgctgttcg attaccatgg atgccttcaa ttgatattaa 6300
tttagattta agattagatg gtttaagttt aatgttcggc ttaattattt cgctaatagg 6360
tgtgggtgta tttttttatg ctacgcaata tttatcccac agtacggaca atcttcctag 6420
atttttcatc tatttactat tatttatgtt cagtatgatt ggcattgtaa tagctaataa 6480
taccatctta atgtatgtat tttgggaact cacaagtatt tcctcattct tgcttatatc 6540
ctattggtac aataatggtg aaagtcaatt aggcgccatt caatctttca tgattacagt 6600
gtttggtggg ctagcgttat taacaggatt tatcatttta tatatcatta caggaacaaa 6660
cacaattact gatatcttaa tcaacgcaat gcaatttcac gacatccttt atttatacca 6720
atgattttga tgctattatt aggtgctttt accaaatctg cacaatttcc gtttcatatt 6780
tggttaccaa aggccatggc agcacctaca ccagtaagtg cttatcttca ttcggcaaca 6840
atggtaaagg ctggaatctt tttactattt agatttacac ctttattggg acttagtaat 6900
gtttatattt atacagtgac atttgttggt ctaataacta tgttatttgg atctttaact 6960
gctttacgac aatacgactt aaaaggtata ctcgcttatt ctacaataag tcaattaggt 7020
atgattatga caatggtagg tctaggtggc ggttatgctc agcacacatc agatgaattg 7080
tctaagtttt atattttagt tttatttgct ggcttattcc atttaatgaa tcatgcggtt 7140
tttaaatgtg cattatttat gggcgttggt atcattgatc acgagtccgg aacacgtgat 7200
attcgtttgc taaatggtat gcgtaaagtc tcccctaaaa tgcatattgt catgttgctc 7260
gctgcattat ctatggcagg tgttcctttt ttaaatggct ttttaagtaa ggaaatgttt 7320
ttagattcgt taactaaagc aaacgaactt gatcaatatg gcttcgtatt aacgtttgtg 7380
attatttcaa taggtgtcat cgcgagtata ttgactttta cttatgcact ttacatgata 7440
aaagaaacat tctggggaaa ttacaatata gaaaaattta aacgtaaaca aatacatgaa 7500
ccatggctat ttagtttacc agctgtgatt ttaatgttac tcattccagt tatcttcttt 7560
gttccaaacg tttttggcaa ctttgttatt ttgcccgcaa ccagatctgt atctgggata 7620
gggcggaggt tgatgcattt gtgccacata tttctcagtg gcatggtgtg aatctccatt 7680
aattttaaga tagtgtatat attggactat tttagctcta gtgtgattgg aaagaggtta 7740
cgcatcaaat aatcaaaagt gctcgattac agtggctatc ggaaatttat agagaatttg 7800
aattatactc agcccgtggt atacgtgcat tgatgaataa taaattgaat tattacatca 7860
tgattacatt atttattttt gtagctattg tagttatgga tatttgactg tgggttttcc 7920
tcatgtactc agcttcatat tagttctttc ggaccgttgg aagttatctt atcagttgta 7980
acattgatta tcggcatttc attaatcttt attcgtcaac gactaacgat ggtggtattg 8040
aatggaatga ttggattcgc agttacatta tattttattg caatgaaagc tccagattta 8100
gctttaacac agttagttgt tgaaactatt acgacaatct tatttattgt tagtttttcg 8160
agactaccta acatccctcg agttaaggca aatttaaaaa aagagacctt caaaatcatt 8220

gtgtcacttg ttatggcatt gacggtggta tcacttattt ttgttgctca acaagcagat 8280
ggtatgcctt caattgctaa attttatgaa gatgcatatg aacttacagg tggaaaaaat 8340
attgtcaatg ctatactagg tgacttcaga gctttagata ctatgtttga aggactagtg 8400
ttaatcatag ctggattagg tatttatacg ttacttaatt acaaagatag gagggggcaa 8460
gatgaaagag aatgatgtag tacttaaatc agttacaaaa attgtagtgt ttattttgtt 8520
aacatttgga ttttatgtat tttttgctgg ccataataat ccaggtggtg gctttattgg 8580
tggcttgatt tttagctcgg catttatctt aatgtttctt gcctttgatg taaatgaagt 8640
gttgaaaaaa gctt 8654

<210> 5
<211> 2362
<212> DNA
<213> Staphylococcus epidermidis

<220>
<223> Designated as SE-3

<400> 5
aagcttcaca acttgaaaat atagcacaaa cattaaagga tttaggtaga aaacgagcaa 60
ttttaattca tggtgcaaat gggatggatg aggccacgct ttctggtgaa aatatcattt 120
atgaagttag cagcgaaaga gcattaaaaa aatatagttt aaaagcagaa gaagtcggtt 180
tagcttatgc aaataatgac acgttgatag gtggttcacc tcaaacaaat aaacaaattg 240
cattgaatat cctaagtggc acggatcact caagtaaacg agatgtagtt ttgttaaatg 300
ctggaattgc tttatatgtt gctgagcaag tggaaagtat caaacatggc gtagagagag 360
cgaaatatct cattgataca ggtatggcaa tgaaacaata tttaaaaatg ggaggttaag 420
taatgactat tttaaatgaa attattgagt ataaaaaaac tttgcttgag cgtaaatact 480
atgataaaaa acttgaaatt ttacaagata acggaaatgt taagaggaga aagctgattg 540
attcacttta actatgatag aacattatca gttattgctg aaataaaatc gaaaagccca 600
tctgtacctc aattaccgca acgtgatctt gttcaacaag ttaaagatta tcaaaaatat 660
ggtgctaatg ctatttcaat attaactgat gaaaaatact ttggcggtag ttttgaacga 720
ttaaatcagt tatcaaagat aacatcgtta ccagttttat gtaaagattt tattattgat 780
aaaattcaaa tagatgttgc aaaacgagct ggtgcatcta ttattttatt aatagtaaat 840
attttaagtg atgaccaatt aaaagaattg tattcatatg caacaaacca taatttagaa 900
gctctagtag aagttcatac aattagagaa cttgaacgtg cacaccaaat taaccctaaa 960
attattggtg ttaataatcg tgatttaaaa cgatttgaaa ccgatgttct acatacaaat 1020
aaattactta agtttaaaaa gtctaattgc tgctacattt cagagagtgg cattcataca 1080
aaagaagatg ttgagaaaat agtagattca agtattgacg gtttacttgt aggggaggca 1140

```
ttaatgaaaa caaatgactt aagtcagttt tttgcctagt ttaaagttaa agaagaatct   1200
ctatgatagt taaattttgt ggttttaaaa ccgaaagtga tattaagaaa attaaaaaat   1260
tagaagttga tgcagtaggg tttatacatt atcccgatag taagagacat gtctcactga   1320
aacaattaaa atatttggct aaaatagtgc cagatcatat agagaaagta gtgtcgtagt   1380
aaatcctcaa atgtccacca taaagagaat aattaatcaa actgatatta acacaatcca   1440
attacatgga aatgaaagca ttcaattaat tagaaatatt aagaaactta attcaaaaat   1500
aagaatcata aaagcaattc cagcaacaag aaatttaaat aataacattc aaaagtataa   1560
agatgagata gactatgttt attatagata caccatcaat cacatacgga gggacaggtc   1620
aaagttttga ctggaaatta ttaaaaaaaa taaaggcgtt gattttctca ttgcggtggt   1680
ttggattttg aaaagataaa acgattagaa atatattcat ttggacaatg tggttatgac   1740
atctcaactg gcattgagtc acataatgaa aaagatttta ataagatgac tcgaatatta   1800
aaatttttga aaggagacga atgattaatg aaaattcaaa cagaagtaga tgaattgggc   1860
tttttcggtg aatatggtgg ccaatatgta cctgaaacat tgatgccagc tattattgaa   1920
cttaaaaaag catatgagga cgcgaaatca gatactcact tcaagaaaga atttaattat   1980
tatttaagtg aatatgttgg tagagaaacg cctttaacat ttgctgaatc atacacaaaa   2040
ttgttaggtg gtgccaaaat atatcttaaa agagaagact taaatcacac tggtgctcat   2100
aaaattaata acgcgatagg acaggcacta ttagctaaaa ggatggggaa aactaaatta   2160
gtagccgaaa caggtgctgg tcaacatggt gtagcaagtg ccaccatcgc tgctttattc   2220
gatatggatc ttattgtttt catgggaagt gaagatatca aacgtcaaca acttaacgta   2280
tttagaatgg aattgctagg agctaaagta gtgtctgtgt cagatgggca aggaacacta   2340
tcagatgctg taaataaagc tt                                            2362
```

<210> 6

<211> 5024

<212> DNA

<213> Staphylococcus epidermidis

<220>

<223> Designated as SE-32

<400> 6

```
aagcttttg atttttaaag aaaaaattaa acaaggggc attgcttatg gtcaatagaa     60
gaaagatatc aattattggc gcgggacata caggtgggac tctagcattc attcttgcac    120
aaaaggaatt aggagatatt gtgttgattg aacgccagca atcagagggt atggctaaag    180
gaaaggcgtt agatatttta gaaagcggac ccatttgggg gtttgacaca tctgtacatg    240
gttcagtaaa tatagaagat attaaagatt cagacatagt ggtgatgact gcaggtatac    300
ctaggaaatc aggaatgaca aggagaagaa ttagttcaaa ctaatgaaca aatagtacga    360
```

gaaactgcat tacaaattgc aacgtatgca cctcattcaa taattattgt attgactaat 420
ccggttgatg ttatgacata tactgcattt aaagcatcag gttttcctaa agaacgtatt 480
attggtcaat ctggaatttt agacgctgca agatatcgaa cttttattgc tcaagaactt 540
aacgtgtctg tcaaagatgt aaatgggttt gttttaggtg gacatggtga tacgatgtta 600
cctttgatta ataacacaca cattaatggg attccagtta agcatcttat ttctgaagaa 660
aagattgatc aaattgttga acgtacacgt aagggtggtg cagaaattgt tgcattacta 720
ggtcaaggct cagcatatta tgcaccagca actgctatat atgaaactat agatgcaatt 780
tttaatgatc ggaaacggtt attaccaagt attgcttatc tagagggaga atacggttgt 840
tcagatattt gtttcggagt tcctactata ataggatatc aaggaataga aaagattata 900
gaggtagata tgaataatga tgagtatcaa caactacaac actctgcgca agatgtgagt 960
gaagtcaaaa actcactaaa attcaaataa ataattatga agttctacat cttaaattgt 1020
tagattttg tgaaaattgt gtaaagggta ttttttcgtt gatttataaa agcgctttct 1080
tgatataatg aacatatatt catagaataa ggagacgatt aaaatggcta aaggggacca 1140
atatcaagct catactgaaa aatatcatga gtaaaaagtc taaaaaaagt tataaacctg 1200
tgtggattat cattagtttt attattttaa ttacaatctt gttattaccc acaccagcag 1260
gattacctgt aatggctaaa gcagcactag ctattttagc tttcgctgta gttatgtggg 1320
ttacagaagc agttacttat ccagtttctg caacattaat tttaggatta atgatacttt 1380
tactaggttt aagtccagtt caagatttat ccgaaaaact tggaaaccta aaagtggcga 1440
cataatacta aaaggtagcg atattttagg aacgaataac gcgcttagtc acgcttttag 1500
tggtttttca acctcagccg tagcacttgt agctgcagca ttatttttag cagtagctat 1560
gcaggaaacc aatttacata aacgacttgc attatttgtg ctatcaattg ttggaaataa 1620
aactagaaat atagtcattg gtgctatttt agtatctatt gttctagcat tctttgtacc 1680
atcagctaca gcacgtgctg gtgcagttgt cccaatatta ctgggaatga ttgctgcatt 1740
taatgtgagt aaggatagta gacttgcttc attattaatt attactgctg tacaagcagt 1800
ttcgatatgg aatataggta ttaaaaacgg ctgcagcaca aaatattgta gccatcaatt 1860
ttattaacca aaatttagga catgatgtat catggggaga gtggttttta tatctgcgcc 1920
gtggtcaatc attatgtcta tagctcttta ttttataatg attaagttta tgccacctga 1980
acatgatgca attgaaggtg gaaaagagtt aattaaaaag gaacttaata aattaggacc 2040
agtcagtcat agagaatggc gactaattgt gatttcagtg cttttatatt ctctggtcga 2100
ctgagaaagt attgcatccg attgattcag cttcgattac actagttgct ctaggtatta 2160
tgctaatgcc aaagattggt gttattactt ggaaaggtgt tgaaaagaag attccttggg 2220
ggacgattat agtatttggt gtaggaatct cacttggtaa tgtattactt aaaacaggag 2280
ccgctcatgg ttagtgatca acatttgttt gatgggtctt aaacatttac cgatcatagc 2340
aactattgcg ttaattacct tatttaatat attaatacat ttaggttttg caagtgcaac 2400
gagcttagcc tctgcgttaa tacctgtgtt tatttctttg acttcaacgc taaatttagg 2460
tgatcatgct attggttttg tattaataca acaatttgtg attagttttg gtttcctact 2520
acctgtcagt gcaccacaaa atatgcttgc atatggtact gggactttta ccgtaaagga 2580

tttttaaag acaggtatac ctttaacgat agtaggttat attttagtta tcgtatttag 2640
tttaacgtat tggaaatggc ttggtttagt gtaagtaaaa gatttaggta ttaaaatgat 2700
aattataaat gtctcgtaaa gtttaatatt ttaactttac gacacatttt ttataaactc 2760
gtggcaagtt aatcttaata gttgaaatgt atcgtataaa aaatatatga atgtaaatag 2820
aatttagtat tagagaataa caaaaaattg atgttaggtg gtaaaatcta atggctatag 2880
gtgtcatatt aaatagagtt tttaggctaa ataataatcc attatttgat tatatatata 2940
gtaataaaga atctataaat cattgttatt ttattattcc aactgaagag tttgaagaag 3000
aagcaaaaaa gaaagcacaa tactattatg ggtccataca gaagtttatg tatgaactac 3060
aacgatatga tatagaaccc tttttgatgt cttatgataa attaatagac ttttgtaaaa 3120
aacaagctat agacaaagtt gttgttgcag gtgatattat gagttatcat cacgaagaat 3180
atgacatttt acatcaaagg aaacgattta aacaagctaa tattcaagta atatcattaa 3240
gagcaaatca ttattttaac ccccgcaaaa cacataataa acaaggggaa ccatataaag 3300
tatttaccag tttttataga aaatggcgtc cttacttaat gattagagat gaatatgact 3360
atcatttaga agatatttca aaggttgtag tgaaatctca acataaaatt aaagaagatt 3420
atcattcata tggtataagt gaacgtgatg ttcaaaatcg ttggtctgaa tttttatctc 3480
aagatatcga aaattataaa gaaaacaggg aatacttgcc tgaagtatta acaagccaac 3540
taagtattta cttagcttat ggaatgatag atattataca atgttttcaa cgatttactt 3600
caaaattatg ataaaaatga acaaaattac gaaacttttta tacgtgaatt gatttttaga 3660
gagttttatt atgtattaat gaccaattat cccgaaacag ctcatgttgc ttttaaagaa 3720
aaataccaac aattgaaatg gtcttataat gaagagaatt ttaaactgtg gaaagatggg 3780
aatactggtt ttccaattat tgatgcagca atggaggaac ttaaaacaac tggatttatg 3840
cataatcgca tgagaatggt agtttctcaa tttttaacta aagatttgtt tattgactgg 3900
atttggggtg agtcattttt caaacaaaaa ttaatagatt atgatgcagc ttcaaatgtt 3960
cacggatggc agtggtcagc ttctactgga acagatgctg taccatactt tagaatgttt 4020
aatcctataa gacaaagcga gcgttttgat aataatgcac gatatataaa aacttacatt 4080
ccaagattaa atcaggtaga tgctaagtat ttacacgata ctcataaatt cgagcaacaa 4140
ataaaggggc aaggtgttga aataggtaaa gactatccta aacaaatgat tgatcacaaa 4200
gaaagtagac aacgtgtaat gtcagaattc aaagctatag attaaataaa aaagatctga 4260
acaacatgat ataggtgttc agatctttat ctagttacat aaaaaagcaa acatgaatta 4320
aaatatattc taacaaagtt aaaatataca tatatttaag atttaattta gttttcaaag 4380
gtacttccca atttgtataa cggggctcat aataaaataa ttgcatcaaa tataatccta 4440
tccctaacgg taaacacatt aataaaatag ctttagtata actccatcct atttgatgcc 4500
ataaatgacc tatcataagt tgaataatga tgagacatac cattaaaatt acttcaatta 4560
tcattggtat aatctcaccc ctttaataaa caatatgact gttgcttgta tgagcaccat 4620
taaaacgaca aatagtaacg ctttaacatc tatgattaaa aaaacctctt tcacaatttt 4680
taaaggtgca tttaataaat agacagtatg taatcttaag aatcgaccga tgtaaatacc 4740
taatccattt aagaacatta atataactat caatagtcga tttaaccata cataagacgt 4800

aaaatgtgca atttctaaaa atataagaat tgtgaggtat attgctaaga gtacgccaag 4860
tattaaatag gtgaaataaa tccattctgt gatgtttaat ccagctaaaa agttaaattg 4920
aaattggttt aagtgtatga gatcggtaat catataaaat gtgtttggaa ctaataatag 4980
aaatatgagt ccgaaaacaa taaataaggg ccattcaaaa gctt 5024

<210> 7
<211> 9515
<212> DNA
<213> Pseudomonas aeruginosa

<220>
<223> Designated as P2-2

<400> 7
aagctttcct ccagaccctt caccgccgtg gagatcgacg gctgggcgat gtacagcttg 60
cgcgaggcct cggccacgct gccgcattcc acggtggtca cgaaatactt gagttgccgc 120
aaggtatagg acgccactgc aagacctcat cggcgcatca tcctccccgg gccgggcgtg 180
cgcgcctcga ttgttgtgtc cgccgcgctg caagcaagtt gcaggccgct gccgagcgtc 240
gcgcgctggc cgcggaacga ttgcccgcct gcacgataac ccagcacgac gcactttgcc 300
ggggcacgcc tggccagctt tttcttatgt cccgaggaca tttttaataa ttttccttcg 360
ccgcggcttg cgcgaccatc cttccccatc gaccccatgg acagcggttc gcctcccggc 420
ggtccgggcc atgcgtgcag aaccacgacc ggcgcagacc ggcgagataa caaggagaag 480
gtggggtgtt cgaactcagc gattggcaac ggcgcgccgc gacacagcgc ttcatcgacc 540
aggccctgat cggcggccgc cagcgtccag ccgccagcgg cgctaccttc gacgccatcg 600
atccggcgag caatcgcctg ctggcgcggg tcgcggcctg cgatgcggcc gacgtcgacg 660
cggcagtggc cgccgcccgc cgcgccttcg acgaaggccc ctgggcgcgt ctcgccccgg 720
tcgagcgcaa gcgcgtgctc tgcgcctggc cgagctgatg ctggcccatc gcgaagagct 780
ggcgctgctc gactcgctga acatgggcaa gccggtgatg gacgcctgga acatcgatgt 840
acccggcgcc gcccacgtct tcgcctggta tgcggaaagc ctcgacaagc tctacgacca 900
ggtcgcgccg gccgcccagc agaccctggc caccattacc cgcgtgccgc tgggggtgat 960
cggcgcggtg gtgccgtgga acttcccgct cgacatggcc gcctggaagc tcgccccggc 1020
cctggccgcc ggcaactcgg tggtgctcaa gccggccgag cagtcgccgt tctccgccct 1080
gcgcctggcc gagctggccc tggaggcggg ggtgccggaa ggcgtgctga acgtggtgcc 1140
gggcctcggc gagcaggccg gcaaggccct cggcttgcac ccggaggtgg acgcactggt 1200
gttcaccggc tccaccgagg tcggcaagta cttcatgcag tattccgcgc aatccaacct 1260
caagcaggtc tggctggagt gcggcggtaa gagtccgaac ctggtgttcg ccgattgccg 1320
cgatcttgac ctggcggcgg aaaaaggcgc cttcggcatt ttcttcaatc agggcgaggt 1380

ctgttcggcg aactcgcgct tgctggtgga gcgttcgatc cacgacgagt tcgtcgagcg 1440
cctgctggcc aaggcccgcg actggcagcc gggcgatccg ctggacccgg gccagccgcg 1500
ccggcgccat cgtcgaccgc cggcagaccg ccgggattct cgccgccatc gagcgggcgc 1560
aaggcgaggg cgcgaccctg ctcgcggtgg ccgccagttg acgatcaacg gttcggacaa 1620
cttcatcgaa ccgaccctgt tcggcgacgt acgcccggac atgcagctgg cccgcgagga 1680
aatcttcggc ccggtgctgg cgatcagcgc cttcgactcc gaggacgagg ccatacgcct 1740
ggccaaggac agccgctacg gcctcgccgc ctcgctgtgg agcgacgacc tgcaccgtgc 1800
gcaccgggtg gcgcggcgct tgaatgccgg aacgtgtcgg tgaataccgt ggacgcgctg 1860
gacgtcgcgg tgcctttcgg cggcggcaag cagtccggct tcggtcgcga cctgtcgctg 1920
cattccttcg acaagtacac ccagttgaag acgacctggt tccagttgcg ctgaagacgc 1980
gacggacgcg acacgactcg atgccgataa cgacaacaag aggacgatcg aatgaacgac 2040
acgccgaacg tgcgtgagcc ggccctgcgc cgcgtgctcg ggctgggacc gctgctggcg 2100
gtggccatcg gcctggtggt ttcccagggc gtgatggtac tgatgctgca aggcgccggg 2160
acggccggcc tgggcttcat cgtgccgctg ggagtggcct acctgctggc gctgactacg 2220
ccttttcctt ttccgagctg gccctgatga ttccccgcgc cggtagcctg agcagctaca 2280
ccgaggtggc catcgggcat ttcccggcga tcctggcgac cttttccggc tacgtggtgg 2340
tggcgatgtt cgccctctcg gcggaactgc tgctgctcga cctgatcatc ggcaaggtct 2400
accccggcgc gctgccgccg atgctggtgc tacggcgtgc tcggcctgtt caccctgctc 2460
aacctgctcg gcatcgacat cttcgcgcgc ctgcagagcg cgctggcgct gctgatgatg 2520
atcgtcctgc tggtgctcgg cctgggtgcg gtgagcagcg accacgcttc cgcgcagacc 2580
gccctggcga gcggctggaa cccgctgggg gtaagcgccc tggcgctcac cgcgatggcc 2640
gtgtggggct tcgtcggcgc cgagttcgtc tgcccgctgg tggaggagac gcggcgtccg 2700
gagcgcaaca tcccgcgttc gatgatcctc ggcctgagca tcatcttcct gaccatcgcc 2760
ctctactgct tcggtgcgct gctgtgcatc ccgcaggcgg aactggccgg cgacccgctg 2820
ccacacttcc tcttcgccaa ccgcgtgttc ggcgagtacg gccagctgtt cctggtgatc 2880
gccgcgatca ccgccacctg cagcaccctc aactcgtcgc tggcggcgat cccgcggatg 2940
ctctacggga tggcgcagaa cggccaggcc ttcccgcaat tcaagcagct cagccggcgg 3000
gcgcgcacgc cctgggtggc ggtgctgttc gtcgccgcga tcaccggcct gccgatcctg 3060
atcctcggcc aggacccgga ctcgatcaac ctgctgctgc tcgccgccgc gctggcctgg 3120
ctgctggcct acatcatcgc ccacgtcgac gtgctggccc tgcgccgtcg ctatccgcac 3180
atcgcccgtc cgtttcgcac gccgttctac ccgctgccgc aactgttcgg catcgccggg 3240
atgatctacg cggtggtcca cgtctcgccg accccggaaa tgaccggacg gatcttcgcc 3300
agcgccggcg tggtgctcgg cgtggtctcg ctggtggcgg tggtgtggat caagggcgtg 3360
atgcgcaagc ccctcttcgt acccgaaccg ctcgagacgg ccggtgagac tgcccagggc 3420
aagtccgtcg ccctcgatcc cctgcaatcc cttcggcctg acgcgccaag ggaacaagga 3480
gaacacagac gatgaccgct cagctcaacc cgcagcgcga cacccgcgac taccagcaac 3540
tggacgccgc gcaccacatc cacgccttcc tcgaccagaa ggcgctgaac cgcgaaaggc 3600

ccgcgggtga tggtccgcgg cgatggcctg cagctctggg acaacgacgg caagcgctac 3660
ctggacggca tgtccggcct ctggtgtacc aacctcggct acggccgcca ggacctcgcc 3720
gccgccgcca gccgccagct ggaacaactg ccgtactaca acatgttctt ccacaccacc 3780
cacccggcgg tggtggagct ttccgagatg ctcttcagcc tgctgccgga ccactacagc 3840
cacgcgatct acaccaactc cggctccgag gccaacgagg tgctgatccg taccgtgcgg 3900
cgctactggc agatcctcgg caagccgcag aagaagatca tgatcggccg ctggaacggc 3960
taccacggct cgaccctggg cagcaccgcg ctcggcggga tgaagttcat gcacgagatg 4020
ggcgcatgct gccggacttc gcccacatcg acgaacccta ctggtacgcc aacggcggcg 4080
agctgagccc ggccgaagtt cggtcgccgc gcggcgctgc aactggagga gaagatcctc 4140
gaactgggcg cggagaacgt cgccgccttc gtcgccgagc ccttccaggg cgccggtggc 4200
atgatcttcc cgccgcaaag ctattggccg gagatccagc gcatctgccg gcagtacgac 4260
gtgctgctgt gcgccgacga agtgatcggc ggcttcggcc gcaccggcga atggttcgcc 4320
cacgaacact ttcgcttcca gccggacacc ttgtccatcg ccaagggcct gacgtccggc 4380
tacatcccca tgggcggcct ggtactcggc aagcgcatcg ccgaggtgct ggtggagcag 4440
ggcggggtgt tcgcccacgg cctgacctat tccggccacc cggtggcggc ggcggtggcc 4500
atcgccaacc tcaaggctgc gcgacgaggg cgtggtcacg cgggtcaggg aggagaccgg 4560
cccctacctg caacgctgcc tgcgcgaggt cttcggcgac catccgctgg tcggcgaggt 4620
ccagggcgcc ggcttcgtcg ccgcgctgca gttcgccgag gacaaggtga cccgcaagcg 4680
cttcgccaac gagaacgatc tggcctggcg ctgccgcacc atcggcggct tcgaggaggg 4740
cgtgatcatc cgctccaccc tcggccgcat gatcatggcc ccggcgctgg tggccgggcg 4800
tgccgagatc gacgaactga tcgacaagac ccgtatcgcg gtggatcgca ccgcgcgcga 4860
gatcggcgtg ctctgacgcg ccccggcggc ccggcctcgg ccgggtcgcc tgcgacacgg 4920
agcgtccccc cataacgacg atgcggcgcc tggcgaccgc gcgcggaacc gtttcggcct 4980
ctggcggcaa ctgcctaagc aacatcacaa caatgccaat cggctgtggg agtgttccat 5040
gttcaagtcc ttgcaccagt acgcacacgt gttttcccgg ttgtccctgt tcgtcctggc 5100
gttcgccgcg gcggcccagg cgcagagcca gagcctgacg gtgatctcct tcggcggcgc 5160
gaccaaggcc gcccaggaac aggcctattt caaacccttc gagcgaagcg gcggcgggca 5220
ggtggtcgcc ggcgaataca acggcgaaat ggccaaggtg aaggccatgg tcgacgtcgg 5280
caaggtcagc tgggacgtgg tcgaggtgga gagccccgaa ctgctccgcg gctgcgacga 5340
ggggctgttc gaacgcctcg acccggcgcg tttcggcgac cccgcgcagt tcgtccccgg 5400
cactttcagc gagtgcgggg tggccaccta cgtctggtcg atggtgatgg cctacgactc 5460
gacgaagctg gccagggcgc cgcagtcctg ggcggatttc tggaacgtcc gcgagttccc 5520
ccggcaagcg tggcctgcgc aagggcgcca agtacaccct ggaagtggcg ttgctggccg 5580
acggggtgaa ggcggaggac ctctacaagg tactcgccac cccggagggg gtcagccgcg 5640
cctttcgcca agctcgacca gctcaagccg aacatccagt ggtgggaggc cggcgcccag 5700
ccgccgcaat ggctggcggc cggcgacgtg gtgatgagcg cggcctacaa cgggcgcatc 5760
gccgctgcgc agaaggaggg ggtgaaactg gccatcgtct ggcccggcag tctctacgat 5820

ccggagtact gggcggtggt gaagggcacc ccgaacaagg cgctggcgga gaaattcatc 5880
gccttcgcca gccagccgca gacgcagaag gtgttctccg agcagatccc ctacgggccg 5940
gtacacaagg gcaccctggc gttgctgccg aagacggtgc aggaggcgct gccgacccgc 6000
gccggccaac ctcgaaggcg cgcgggcggt ggatgccgag ttctgggtgg accacggcga 6060
ggagctggaa cagcgtttca atgcctgggc gcgcgctgag cgctgcgcgt cggcaaaaaa 6120
aatgacgggc cccaagtcgt ccgggcccgt cgggtcaaag cgctgacggg gtgatcagcg 6180
cagctcttcc aacaacccct gcagataccg acagccctcg gtatccagcg cctgcaccgg 6240
aaggcgcggc gcccccacct ccaggccgga gaggcccagg ccggccttga tggtggtcgg 6300
caggccccgg cggaggatga agtcgagcag cggcaactgc cggtagaaca gcgcgcgggc 6360
cttctccagg tcgccgtcga gcaccgcctg gtagagctgg ccgttgagcg tcgggatcag 6420
gttcggcgcg gcgctgcacc agcctttcgc gccggccacg aaggcctcca gcgccagcgc 6480
gttgcagccg ttgtagaagg gcacccggcc ttcgccgagc aggcgcagct tgtgcatgcg 6540
ctggatgtcg ccggtgctct ccttgaccat ggtcacgttg tccacttcgc ggacgatgcg 6600
caggatcagt tccaccgaca tgtcgatgcc gctggtgccc gggttgttgt agagcatcac 6660
cggcacgccg atggcttcgc caaccgcgcg gtagtgctgg aacacttccg cctcgttgag 6720
cttccagtag gagatcggca ggaccatcac cgcctcggcg ccgagggatt cggcgaactg 6780
cgcgcggcgc acggtcttgg cggtggtcag gtcggagacg ctgacgatgg tcggcacgcg 6840
atgggcgacg gtcttcaggg tgaagtcgac cacctcgtcc cattccgggt cgctcaggta 6900
ggcgccttcg ccggtgctgc cgagcggggc gatggcgtgc acgccgccgt cgatcaggcg 6960
ctcgatggag cggccgaggg ccggcaggtc gagaccgccg tcggcgccga aggggggtga 7020
tggtgtagcc gatgatgccg tggatggatg cggacattgg atgtacccgt gacattgagt 7080
gggaaatgcc aggacggacc tggtgggaaa ggtcgttcag ctcaggcagt cgctgttgcg 7140
cggcaggcag cgccgggcgt agtagttgaa tgcggcgccg tggcgcttcg gggtggagat 7200
ccagtcgtgg gcctcgcgcg ccagggccgg cgggatcggc ttgatctctc cggcggccat 7260
cgccagcaac tgcatcttcg ccgcgcgctc gagcagcacc gcgatcacgc aggcctcctc 7320
gatgctcgca ccggtggcca gcaggccgtg gtgggagagc aggatggcgc gcttgtcgcc 7380
gagggcggcg gagatgatct cgccttcctc gttgcctacc ggcacgcccg gccagtcctt 7440
gaggaaggcg cagtcgtcgt atagcgggca aaggtccatg tgcgagacct gcagcggtac 7500
ttccagggtc gacagcgcgg cgatgtgcag cgggtgggtg tggatgatgc agttgacgtc 7560
cgggcgggcg cgatagaccc agctgtggaa gcgattggcc ggattcgcca tgccgtgccc 7620
gtggaggacg ttgaggtctt cgtcgaccag cagcaggttg ccggcgctga tctcgtcgaa 7680
gcccaggccc agttgctggg tgtagtaggt ccccgcctcc gggccgcgcg aggtgatctg 7740
cccggcgagc ccggagtcgt ggccggcctc gaagagaatc cggcaggtca gggccagctt 7800
ttgccggtca gtccacgtat tatcgccgag gctgcttttc atctgcttca gcgcgtgctg 7860
gatcagttga tccttgggta attccagtgt cgtaaccatg cgaggttcct ttgacggagc 7920
gagtcggggg aaacgccagg cagttgcgcg ccacgcaacg acccggctgt aaatgacacg 7980
gatcaagtta tatgacacaa agtgtcattt agcaagagag aagtttcatc gccatcggga 8040

gaaggctgtc ctcaatgtcc atgcgcttga aattgctgag aaaaaaactc ggggtcacgc 8100
tggagaccct ggccgacaag accggcctga ccaagagcta cctgtccaag gtcgagcgcg 8160
ggctgaacac gccgtccatt gccgccgcgc tgaagctggc gaaggcgttg aacgtgcagg 8220
tggaggagct gttctccgag gaaagcgacg gtgtcgacgg ctacagcatc gttcgtcgcg 8280
accagcgcaa gtcgctgtcc agcggcgacg acggcccggc ctacgcctcc ctcgtcgcag 8340
cagatcggcg cccgcgcgct gttgccgttc atcgtccacc ccccgcgcga tttcagtcac 8400
tcgacgttca aggagcacct cggcgaagag ttcatcttcg tccatgaggg ccaggtcgag 8460
gtcgacttca tgaaccagcg gatcatcctc gagcgcggcg acgccctgca tttcaacgca 8520
cagaagccgc accgcatccg ctccctgggg gagacccagg cggaattgct ggtggtgatc 8580
cacagcgacg aatgaggcga cggcttcggt cgatcggatg cttgctaacg ttctgttcga 8640
ttatcgaact gttaatcgat tatcggattg tgagccctcg gaccccggcg taaggttctc 8700
gtcacgtgcc gtccaggcag cgcacaacaa gacgagaccc gaccgatggc tgaaatcctc 8760
tccctgcgcg aacggtgcga cgcttcgtcc acgatggcga cagcgtcgcc ctcgaaggct 8820
tcactcacct gatcccgacg nccgccggcc acgagctgat ccgccagggc aggaaagacc 8880
tgacgctgat ccgcatgact cccgacctgg tctacgacct gctgatcggt gcaggctgcg 8940
cgaagaagct ggtgttctcc tggggcggca accccggtgt cggttcgctg caccgcctgc 9000
gcgacgcggt ggagaagggc tcggccgcaa ccgctggaga tcgaggaaca cagccacgcc 9060
gacctcgcca acgcctattt tgccggcgcc tccgggctgc ccttcgcggt ntgcgcgcct 9120
acgccggctc cgacctgccg aaggtcaacc cgctgatccg cagcgtcacc tgcccgttca 9180
ccggcgaagt gctggcggcg gtgccctcgg tgcgtccgga cgtcagcgtg atccacgcgc 9240
agaaggccga ccgcaagggc aacgtgctgc tctggggcat cctcggcgtg cagaaggaag 9300
cggccctggc ggcgaagcgc tgcatcgtca ccgtcgagga gatcgtcgac gaactggacg 9360
ccccgatgaa cgcctgcgtc ctgccgagct ggggcgctca gcgccgtgtg cctggtgccc 9420
ggcggcgcgc atccgtccta tgcccacggc tactacgagc gcgacaaccg cttctaccag 9480
gactgggacc cgatcgcccg cgaccgcgaa agctt 9515

<210> 8

<211> 2291

<212> DNA

<213> Enterococcus faecalis

<220>

<223> Designated as EF-1

<400> 8

aagctttaga taatgataaa cgcgtgtatg tgaatgtcca gccgattcaa tcgcctactg 60
gagaaacagt gattggtgtc ctttatgtga aaagtaattt agaaaataaa taccaagaaa 120

ttactaacac agcaagtatc tttttcactg cttctattat tgccgcagca atctcgatta 180
ttgtgaccct actgattgca cgatcaatca cgaagccgat tggtgaaatg cgcgagcaag 240
ccattcgaat cgctcgtggt gattacgctg gaaaagtaga agtccatgga aaagatgaat 300
taggccaatt agcagaaaca tttaatcaat tatcagaacg gattgaagaa gcacaagaaa 360
caatggaagc agaagaatcg tttagatagt gtcttaacgc atatgacaga tggtgtcatt 420
gcgacggatc gccgcggaaa ggtgattacg attaatgaga tggccctttc attattaaat 480
gtaaaaaatg aaaatgtgat tgggacctcg ttattagagt tgttagatat tgaagaagat 540
tacacattgc ggaagctgtt agaagagcca gatgaactgc tgattgatcg ctcaacgtct 600
gatcgtgaag aagaccaaat gattatccgg gtagacttta cgatgattcg tcgggaatca 660
ggatttatta ctggcttagt ttgcgtactt catgacgtca cagaacagga aaaaaacgaa 720
cgggaaagac gggaatttgt ttccaatgtt tctcatgagt tgcgacgcct ttgacaagta 780
tgcgtagtta tatagaggct ttgagtgaag gagcttggga aaaccctgag attgcgccga 840
atttcttaaa agtcacgtta gaagaaaccg accggatgat tcgtatgatt aatgatttgt 900
taaatttatc tcggatggac tctgggaata cacatcttca attagagtat gtgaatttta 960
acgaattgat taattttgtc ttggatcgct ttgatatgat gattgaaaat gagcaaaaaa 1020
attacaaaat tcgccgtgaa tttactaaac gcgatttatg ggtagagtta gatacagaca 1080
aagtaattca ggtttttgac aacattttga acaatgcgat taagtattcg ccagatggcg 1140
gcgtcattac ctgccgacta gttgaaacac ataataatgt cgtctttagt atctcggacc 1200
aaggtttggg catccctaaa aaagatctcg ggaaagtctt cgagcgtttt tatcgtgtgg 1260
ataaagcacg tgcgcgagca caaggtggga ctggtttagg tttagcaatt tctaaagaag 1320
taattcgggc ccataacggg agtatttggg tggaaagtac agaaggtgaa ggatcaactt 1380
tctatatttc actaccatat gaaccttatg aagaggattg gtgggaatga tgaaaaaatc 1440
agaatggatt acaagaattg gcttgatttt gatggtcatt ttaagtatat atttttcagt 1500
caatatctgg ctgaattctg ccaaaaaaat accagaaatg aagtcgggaa gccaagtcac 1560
aacagctgtc aatgaaaaag ccattggcga tgtctattta cctttgcaat tgattcgaat 1620
agccgatgga aaagcgatgc aaagtaatcg tgaaacatta attagtaatg ttcaaaatga 1680
tattaaaatg gctacgtttg gtaaattgac acaagttgtg acaaaaaatg cagagcaact 1740
taagcgctac aaccaaatgg aacaaggcat tgaacttctt tatcaaggtc ccttttttaat 1800
ctcggactat gcttcgattt ataatctatc cattaatttt actaacttta atgagttgac 1860
ggaccagtat tttacgaaaa ttcaattgga ttttaacgaa aataagatac gtttttttaga 1920
ttatgatcaa tccaacgtct atgaagcgcc catgactgtt aataaggcgc gcttaatggg 1980
aattatcaat aaagagggat tgcaatatca agacgtttcc gaaaatacgc taaccaaaca 2040
aggacaatgt tatttaacca atgatatgaa gttgaaaaag tacagttata tcttanttcg 2100
caaccagtta ctcgttttag gaatgctttt ttcaatgaaa cggaagatat ccaaaccaat 2160
gaagacagtc aagacttaac ctatacgagt aaagaagaac gattgtttgc agaagaaaaa 2220
ctggggaaaa tcgattttaa agggaccttg ccagaagaga ataaacggga ctcaatctat 2280
aatcaaagct t 2291

<210> 9

<211> 2441

<212> DNA

<213> Enterococcus faecalis

<220>

<223> Designated as EF-27

<400> 9

aagcttctgc gctaggaacc agccctttaa ttacatctcc ccatactgga tttgacaatg 60

ccacttgata agcaaaaatc acaaaaataa caacaattaa agcaacaaca atagcttcaa 120

tttttctaaa accaattttt gtcaataaca acaaaagtaa aacatcaaat accgtaatga 180

agacagccag acctaaagga atatgaaata ataaatataa ggcaattgcg cccccgataa 240

cttcagcgat atctgtagcc ataattgcta actctgttaa aatccataat acaataccta 300

acgtcttact agttctagca cgaatcgctt gtgctaaatc catctgtgaa caatgcctaa 360

tttagcagcc atatattgga gcaacattgc aatcaaactg gaaattaaaa taatcgacat 420

caataaatat tgaaaatttt gtcccccagt aattgaagta gaccagtttc ctggatccat 480

ataccccact gctaccaatg ctcctggacc tgagtaagca aataacgttt tccaaaaact 540

catattttta ggcacgtcga tggtgccatt aatttcttca agcgaaggac catttgcata 600

ttcaatcaaa tgatgtcttt gctttggttc atgttcttct gaatttttca attcaattcc 660

ttctttcgtt ttgcaataat tttaaaaggc ccttccccgtt agaaggttaa cctctagtat 720

attttaggta cacctaaaat atactgctaa aaataacaaa atgcaagact tgaaagaaaa 780

ttttgacagt gtaaaaatag attgtcgtaa atgtgcgatc ttaaagtttg aagaaatcag 840

ggtagctggt agttgattat cttaagaagt agaaaataag ggacctaagt catttcggct 900

taggtccctt attttatttt tattcggtta ttctattaag aatggatgct acaatttctg 960

tcgtgtcagc tgaatgattt ctaaaatctc gtaaacttaa tctgacgaaa accttcaagt 1020

acttcgggca acttattttn cccccattca aaagttccat catttctttt caataatctt 1080

tgtaaaattt cttctttctc gaccgctaac aaaaaatgat aaacgtcaat gcctgctcgt 1140

ctcagatatc caatcagctc ttcttcatat tcatttttat aaagggtcat tgtaacaata 1200

atcggccgtc cagactcttt ggacattcgt tttaataaat gagcattcca gcaacgccat 1260

tcctgatact cctgaaaatc attttctttc atttcttcgg gaactagctc catcaatgca 1320

ctaccaataa tttctggatc ataaatgatt gcgttgggaa gtttttgttg taactcatgt 1380

gcaatggtcg tttttccgga tccaaacgca ccgtttaacc aaataattat cataatttcc 1440

ttttcttctg aacaaatttc tttgttgttt aatttaggtg ctagattact tttaattttt 1500

ttagccattc acttatagtt actacttaca tctttaacag taaacgagac aaactaaaaa 1560

tacaacatcc tacgctatta acctcgggtt atataacata ctcatctgat aatttctccc 1620

taaaaaaaca gaatgtgggc aatcttttta agaataattg aatagaataa caacaaacag 1680
taattcaggt ataaccagct agaaattgtt ttatttttag tcacgagtat gataagcatg 1740
taaatcaaat agaatcatat taggtgaggt tactctgaag aacacaggtt atcgctcgga 1800
aatgtcgaga gacagtaacg agtaaagcag ggattgtcga attaaggctt tcctaagata 1860
actagaattt ttttcttacg tctcagaaag ccaaagctca attattgtga ttaccctata 1920
atcttcttct tttattcggc gacctcttta atatgattaa ttggaggttt ttaaattgaa 1980
agctgtcact gcatcatcta agaaaaatac cctacttgct aaaagtatcg ggaatcttac 2040
cttgctcatc attttaggca ttttcatttt tatcatcgtc ttctcttggc taaaaatgaa 2100
tcgccctctc cacacccttc cctcagaaga attcctcgca acaccaagta aaacagatga 2160
tttcttatct ccatcaaatc ttttttactt ttcaattcga accatgtttc gaatgattgt 2220
ggggatggct tggtccttcc tgttttcctt tgtttttggt attttagccg taaaatataa 2280
aacggcacga agagtcattt taccattagt taatttcctt gaatctgttc cattgctagg 2340
ttttttgacc tttacaactg cttggttact tggtttattt ccaggaaatg tgatgggcgc 2400
agaagcggtt gctatttttg ccatcttcac aggtcaagct t 2441

<210> 10
<211> 3480
<212> DNA
<213> Enterococcus faecalis

<220>
<223> Designated as EF-7

<400> 10
aagcttctag cgtttcggat tggcgcctat gatgcaccag gagagcgacg aatcaatacc 60
aaaaatatgc ctacagcagg aggacttgca atctacattg cttttgctag ttcatgttta 120
ttgatttttc gttcgattat cccacaagat tatatttggc cgattatttt ggctggtgga 180
atggttgttt tgacaggcct cattgatgat attaaagaga ttactccaat gaaaaaaaca 240
atcggtattt tgttagcagc attagttatt ttattttgtt gctggaattc ggatagattt 300
tgtgacgttg ccagttgttg gaatgattga tttgcgctgg tttagtttac cactaacttt 360
attgtggatt ttagcgatta cgaatgcagt aaatttaatt gatggtttgg atggtttagc 420
atcaggcgta tccattattg gattaaccac gattggtatt acagggtatt ttttcctaca 480
tgctaaaacg gtctatatcc caattgttat ttttatttta gttgcgagca ttgcgggatt 540
tttcccatac aatttttatc cggctaaaat atttctagga gataccgggg cgttattcct 600
cgggtttatg attgcagtaa tgtcgttaca gggcttgaaa aatgctacgt ttattacggt 660
aattacgcca atggtgattt taggtgtgca attacggata cggtttatgc aattattcga 720
cggctattga acaagaagcc catttcctca gcagataaaa tgcatttaca tcaccgcttg 780

ttatctttag gttttaccca taaaggggcg gtcatgacta tttatgcatt agcgttagtt 840
ttttcctttg tctctttatt gttcagctat tcaagtacag tagcatcaat tttattaatt 900
gtcttttgtt taattggctt agaactattc attgaactaa tcggtctagt tggcgaaggg 960
catcaaccgt tgatgtattt gttacggatt ttagggaatc gtgaatatcg tcaggagcaa 1020
atgaaaaagc gacttggcaa gcattctaag agaaagtaaa gaaatcttta ggttgctttg 1080
cgagagctaa acctatgata taattccatt aaacttaaaa aagtatatgt gtgaaacata 1140
tgcttttttt ttaagacgat gtttcagtag taaggagaaa tgagcatgca agaaatggta 1200
acaatctcga ttgtcactta taatagtcgt tacattttta atgtactaga ccaattaaaa 1260
gccgaactag gtactgatag tatctatgat attcatatct atgacaatca ttctgaaaca 1320
gcgtatcttg aaaaattaac aacatatgaa ccatttatta ctatccatcg cgctgaagaa 1380
aatcaagggt ttggtcatgg tcataatcaa gtgttattca atgcttcgac aaagtatgca 1440
attattttta tcccgatgtg ttggttacta aagacgtgct tgatcgttat tagacgtatc 1500
aaatagataa gaacattgca gtcggtagcc ctaaagttgt taaatgaaga tggcacgacg 1560
caatatttag ttcgtcaaaa attagatgtc ttcgattata tgttacgttt tattcccttt 1620
caatttgtaa agaaaatttt tgataaacgt ttgagtattt atgaatgtcg cgatttgtcg 1680
gatacagaaa caacggatat taaaatgggc tcaggctgtt ttatgttgat tgatcgtgaa 1740
aaattcgttg aaattggtgg gttcgatgaa cgtttcttca tgtactttga agacaacgat 1800
ttatgtttac gctttggcaa agcaggctat cggattctct atacgccttt tgaaacggtt 1860
gttcacatgt atgaaaaggg cgcccataaa agtcgaaaat tgtttaaaat ctttatgcaa 1920
tcaatgggga aatttttaa caaatggggc tggaggttct tttaatgagt caaagattag 1980
cggtagtcat cgtcttatat caaatgaaaa tggctgatac gccgaattat ttgttattaa 2040
aagaagtggt agaccacccc caattgcact tatttattta tgacaacagt ccacttcctc 2100
aagaagatgc attattttta caaccaaatg ttacttatcg acataatcct gataatccag 2160
gactagcgac cgcttataat gaagcgattg cttttagtca agcgaatcaa tgtgaattat 2220
tgttgctcct tgaccaagac acagaagtgc cagcctctta ttttgatacg ttgatcatca 2280
tgccattaga tccgactgtg gcagtctatg ttccaattgt agaagcaaat ggacaacaaa 2340
tttcgccagt atatagtgat caatacgttg ggcttaaagg agcaaagcca acagcaggga 2400
tagccaacca accgttgatg gctatcaatt ctggtacagt tattacggca gaaacgctac 2460
gctggttgga aggattttcg gaagaatttc ctttggacta tttagaccat tggttctttt 2520
atcaattaaa tcaagccaat aaaaagattg aagtcttacc aatccaccta aaacaagaat 2580
tgtctgtttt agattatcgt acaatgagtc ctcaacgtta tcgctctatt attgaagcag 2640
aaacgttatt ttatcgtcga tatgatcaag aaaagttttc ccatcatcga cgccatttat 2700
ttttacgcag tagtaagcaa tttttaactg tcaaaaatcg ccaaatttgg cggcaaacat 2760
tggcagaatt tctcaagtta atgaaaggat aatctatgat ctcagtttgt attgcgacat 2820
ataatggaga aaaatatctc gcggaacaat tagatagtat tcttttacaa gtcagtgaag 2880
aagatgaact aattatttca gatgatggtt ctactgatca tacgttggaa attttgagga 2940
cgtatgcagc gaattatccc caaattcaat tgttacaagg tcccagggca aggagtgatt 3000

gctaattttg cattttgcct tacgcatacg aaaggcgaag taatattttt agcagatcaa 3060
gatgatgttt ggttgccaaa taaagtaacg acggtgacag aatattttga agcgcaccct 3120
gacatccaag tggttattag tgacttgaaa attgttgatg cggatttaca agttaccaat 3180
ccctcttatt taagtttcga aaagtcaaac cagggttttg gcgaaatgcg ataaaaagtg 3240
gctatattgg ggcaggtatg gcctttcgtc aagaaatgaa aaacgtcatt ttacccattc 3300
cgccagaagt tcctatgcat gatatgtgga ttggcttatt agctgcacgg aagaagcaaa 3360
cgggtctcat taaagaacca ttagtgcttt accgaagaca tggagcgaat gtcagcccca 3420
ttattaccaa aacaagtttc caacaaaaat taaattggcg tgtgaattta ttaaaagctt 3480

<210> 11

<211> 3615

<212> DNA

<213> Escherichia coli

<220>

<223> Designated as EC-24

<400> 11

aagcttttct tgcgtgttct tgtgaggctt ccttcgccat tatcatcacg atccacataa 60
ataaagccgt agcgcttaga catttgtgaa tgagatgcac tgactaaatc aattggcccc 120
caactggtgt accccataat atccacacca tcggcaatcg cttcatttac ctgtaccagg 180
tgatcgttta aataggcaat tcgataatcg tcctgtatcg aaccatccgc ttcaacgctg 240
tcttttgcgc ctaatccgtt ctcgacaata aataacggtt tttgataacg atcccaaagc 300
gtatttaaca gaacccgtaa tccaaccgga tcaatttgcc acccccactc tgaacttttc 360
agatgcggat tggggatcat attcagtatg ttgccctgcg catttttatt aatgctttcg 420
tcgtgggaac acaaccagtc atgtataact aaagagatga atcgacggta tgttttaaat 480
ctctgcgtca ctttcagtca tctcaatggt gatattgtgg tcgcggaaga aacgctgcat 540
atagccggga tactggccac gcgcctgaac atcaccaaag aacatccagc gccggttctc 600
ttccatggcc tgcaacatat cctgtggctg gcaggtgagg gggtaaacca gcccaccgag 660
aagcatattg ccgattttcg cttcggggag caggctatga caggctttaa ctgcccgcgc 720
actggcaacc agttgatggt ggatagcctg ataaacttcc gcctcgccac tctcttctgc 780
cagccccacg cccgtgaatg gcgcgtgtaa cgacatgttg atttcattaa acgtcagcca 840
taacgccact ttatgttggt agcgagtaaa gaccgtgcgg gcgtaatgtt cgaagtgatc 900
gatgaccgct cgattagcca accgccgtag tttttcacca gcccatatgg catttcgtaa 960
tgggataacg ttaccagcgg cttgatcccc gcctgcgcca tttcatcaaa cagccgatcg 1020
taaaacgcta accccgcttc attcggttcg acttcgtcgc cctgagggaa aattcgcgcc 1080
caggcaatgg aaatacgcag acaggtgaag cccatctcgg caaataacgc gatatcttcc 1140

gggtaacggt gataaaaatc gatggcgaca tctttgatat tctctttccc caggatgcgc 1200
ggttccattt ttcccattac gcatgaggct gtaaatctga ggtcgagatc cctttgccat 1260
cttcctgcca ggcaccttcc acctgattgg cagctgttgc ggcaccccaa agaaatgttt 1320
ctggaaatgc tttcataatt aactcctttt atcgttagcg aatgatggat aacagcggtt 1380
cacctgcgct tatctgcgcc gtgccgtggg gtaatacgtc cgtaaaatca tcgctattac 1440
tgattaatac cggcgtcgtc agatcaaatc cggcctcgcg aatagcaggg atatcaaaag 1500
aaatcagccg atcgcctgta ttgaccttgt cacccacgtt gacgtgagcg gaaaagaatt 1560
tgccgtccag ttttacggtg tcgataccga catgaatcag gatctccaca ccatcatctg 1620
actcaatgcc aatggcgtgt aatgtggcga acaacgaagc aattcgaccc gcaaccggag 1680
aacgcacttc accaaccgag ggcagaatgg caataccttt acccaacagg ccactggcaa 1740
acgtggtatc agcgacgtga atgagcgaca caatctctcc cgtcatcggt gaacagatac 1800
cgccctgctc aggtggtgta ataacctctg gtgtttctc ttcggggcac cctgcgctgg 1860
ctgacgttta gcggtgatga aatgaagcat caccgtaccg acaaatgcgc aaccgatggc 1920
aatgacaccg ccaataacgc tggcccagac ggtgaaatca attcccgttg acgggatggt 1980
ttgcatgaag gtgaaaatac ttggcaaacc aaaggagtag actttcgttt gcgcgtagcc 2040
aataatggtg gcccccaaag ccccactgat acaggcgata acaaaggggt acttacgcgg 2100
caggttgacg ccatataccg ctggttcggt gataccaaac agactcgtca acgccgctga 2160
tcccgccacc actttttct gcgcatcgcg ttcgcagagg aagacgccga gcgccgcccc 2220
gacctgcgcc ataatggcgg gcattaacag cgggatcatg gtgtcgtagc ccagcacggt 2280
gaagttattg atacacaccg gcaccaggcc ccagtgcagt ccgaacatga cgaagatttg 2340
ccagaagccg cccattaccg cgcccgcaaa tgcaggaacc gcctgataaa gccagagata 2400
accggcggca atcagttcgc ttatccaggt tgatagcggc cccaccagca gaaaggtgac 2460
gggtgtgata accatcagac atagcaatgg tgtgaagaaa tttttgattg ccgacggtaa 2520
ccacgcatta agtcggcgtt ccagaatgct gcacaaccag gcagaaaaaa taatgggaat 2580
aaccgatgac gagtaattca acaatgtgac cggaataccc aggaaatcca gccccagcgc 2640
atccgctttt gcgcgttctc gaaaagcagt acagaattaa tggatgcact aacgctccac 2700
caatcaccat ggcagtaaat ggattatcgc cgaagcgttt ccccgcggtg tatcccagga 2760
ttatcgggaa gaaccaaaac aaggcatcac tggcgctgaa taaaattaaa taagtaccac 2820
tttgttcggg cgtccactga aaagtgagcg ccagagccag catacctttc aagatccccg 2880
gttgcccgcc atcaaaccga tacagaggcg taaaaatacc tgaaataaca taaacaaagc 2940
ggtttagaca gattaccttt atcatacatt ttccggtgcc tgttgcgctt tttcgtcaag 3000
gcctgccaca ctgttaaccg ccaggaagac atcggccaca tggttaccta tgaccacctg 3060
aaactggcca ccgctttcca ccaccataat aataccgggg gtctttttca gtacctctgc 3120
ttgcgctttg ctttcatcct ttaatttaaa aacgtaaatc gcgttgcgca atgcatcaga 3180
ctcacaatgt tatctgcgcc cccgactcct gcgactattt ttctggctaa ctccgtcata 3240
acttgccctc tacgctttgc ggcaaaactc caaaaaaaaa cctgaaaaaa acggcctgac 3300
gtgaatcaag caattttttt caggttttgc ccgcttagtg cggtaacaat cctttactca 3360

gtaataatat ttcagtgttc tttgcgcacg cgctctatat ttatggctaa aaacataatc 3420
tctgcgggtg aaattttacg ttgatactgc aaaccaataa aaatggcgat ccgttccgca 3480
cattgccatg cttgcgggta attttgtttt actgcttgtt gtaatgattc atcactatcg 3540
ttaattgaag catgttcaag aatacgccag gataaaaact tcagatgtgt aaccagtcgc 3600
tgataactca agctt 3615

<210> 12
<211> 4954
<212> DNA
<213> Escherichia coli

<220>
<223> Designated as EC-34

<400> 12
aagcttaacc gctctcatct gttgaccgca cggcatagct atattctgcc ggtcctggga 60
cgtagcgaga ttgacatgca aaaaaacggt gcgcaggcgg taaccgttga ggattcaatg 120
tcgatgattc atgcctcgcg tggcgtgtta aaacccgccg gtgtaatgct gaaatcagag 180
tgtgcagtgg tcgcgggaat cgcgcaggca gcactacccc agagcgtggt agcctgggag 240
tatctggtgg aagattatga tcgcattcgc aatgacattg aagctgtgct gccagagttc 300
gccgactata accagcgcat ccgtcatccc ggtggttttc acctgataaa tgcagctgct 360
gaaaggcgct ggatgacgcc gtcaggtaag gctaatttca ttaccagcaa agggctgtta 420
gaagatccct cttcagcgtt taacagtaag ctggtcatgg cgacagtacg cagccacgat 480
cagtacaaca cgacgattta tggtatggat gatcgctatc gaggggtatt cggtcaacga 540
gatgtggtct ttatgagtgc taaacaagct aaaatttgcc gtgtaaaaaa cggcgaaaga 600
gttaatctta ttgcgcttac gccagacggt aagcgcagtc acgccgcatg gatagattaa 660
aagtggtcat ttaccctatg gctgaccgct cactggtgac ctattttcca gaatcgaatc 720
acatgctaac acttgataac cacgatccat taagtggcat tcctggctat aaaagtattc 780
cgcttgaatt agaaccatca aattaatgtc tcttctcatt tcttctgctg tcatccgcac 840
agcagaagaa ttcctcattg actattattt cgcaatttgc tcacatggat taaattaaac 900
tacatactat aagatataaa cttctgccta cagctgtaag aaactccgct cagtactgaa 960
gcaccagtcc tatttcctct tttctccagc ctgttatatt aagcatactg attaacgatt 1020
tttaacgtta tccgctaaat aaacatattt gaaatgcatg cgaccacagt gaaaaacaaa 1080
atcacgcaaa gagacaacta taaagaaatc atgtctgcaa ttgtgggtgt cttattactg 1140
acacttacgt gatagccatt ttttcggcaa ttgatcagct gagtatttca gaaatgggtc 1200
gcattgcaag agatcttaca catttcatta tcaatagttt gcaaggctgt aaacaaacag 1260
caaattataa atatgaaatg ttaaaaaagt atcgataaaa actttattgt tttaaggaga 1320

taaaatgtcg ctcgtttgtt ctgttatatt tattcatcat gccttcaacg ctaacatttt 1380
agataaagat tacgccttct ctgacggcga gatcctgatg gtagataacg ctgttcgtac 1440
gcattttgaa ccttatgagc ggcattttaa agagatcgga tttactgaaa ataccattaa 1500
aaaatatcta caatgcacta acatccagac agtgacggtg cctgttcctg cgaagttttt 1560
acgtgcttca aatgtaccga ctggattgct taatgaaatg attgcttatc tcaactcgga 1620
agaacgcaat catcataatt tttcagaact tttgcttttt tcttgcctgt ctatttttgc 1680
cgcatgcaaa ggtttcatta cactattaac taacggtgtg ctatccgttt ctgggaaagt 1740
gagaaatatt gtcaacatga agccggcgca cccatggaag ctgaaagata tttgtgactg 1800
cctgtacatc agtgaaagcc tgttgaagaa aaacttaagc aagagcaaac gacattctca 1860
cagattcttt tagatgcaag aatgcagcac gcaaaaaatt tgatacgcgt agaaggttca 1920
gtcaataaaa ttgccgaaca atgtggttat gccagtacat cttattttat ttatgcgttc 1980
cgcaaacatt tcggcaacag tccgaagaga gtttctaagg agtaccgttg tcaaagtcac 2040
acgggtatga atacgggcaa cacgatgaat gctttagcta tttgattatt tgctaacgag 2100
tagtcaacca cacacgctgc gtaagaatta aatggggcag ccattccctg ccccgcgttg 2160
tttttaggcg atatatttat tgaaataaat aagtgacatc catcacatat ttatgcactt 2220
gcataacctg ttgcatgatt atttatgatc tcaattctgc attttgtcag taaaatgcaa 2280
taatttatta aatatcaata aattagttgt ttatcggcga gaaattactt aatagaacag 2340
aaagtaatgt caacgcttta tggactgttt tttccctttt tttagctaaa tctgctatct 2400
ctttatgtga ctaacttcac ttacatccac ttatttctct tcgtaaaatt actttggaat 2460
taagtacaat aagaagagga acatttatga agtctgcatt aaagaaaagt gtcgtaagta 2520
cctcgatatc tttgatactg gcatctggta tggctgcatt tgctgctcat gcggcagatg 2580
atgtaaagct gaaagcaacc aaaacaaacg ttgctttctc agactttacg ccgacagaat 2640
acagtaccaa aggaaagcca aatattatcg tactgaccat ggatgatctt ggttatggac 2700
aacttccttt tgataaggga tcttttgacc caaaaacaat ggaaaatcgt gaagttgtcg 2760
atacctacaa aatagggata gataaagcca ttgaagctgc acaaaaatca acgccgacgc 2820
tcctttcatt aatggatgaa ggcgtacgtt ttactaacgg ctatgtggca cacggtgttt 2880
ccggcccctc ccgcgccgca ataatgaccg gtcgagctcc cgcccgcttt ggtgtctatt 2940
ccaataccga tgctcaggat ggtattccgc taacagaaac tttcttgcct gaattattcc 3000
agaatcatgg ttattacact gcagcagtag gtaaatggca cttgtcaaaa atcagtaatg 3060
tgccggtacc ggaagataaa caaacgcgtg actatcatga caccttcacc acattttctg 3120
cggaagaatg gcaacctcaa aaccgtggct ttgattactt tatgggattc cacgctgcag 3180
gaacggcata ttacaactcc ccttcactgt tcaaaaatcg tgaacgtgtc cccgcaaaag 3240
gttatatcag cgatcagtta accgatgagg caattggcgt tgttgatcgt gccaaaacac 3300
ttgaccagcc ttttatgctt tacctggctt ataatgctcc gcacctgcca aatgataatc 3360
ctgcaccgga tcaatatcag aagcaattta ataccggtag tcaaacagca gataactact 3420
acgcttccgt ttattctgtt gatcagggtg taaaacgcat tctcgaacaa ctgaagaaaa 3480
acggacagta tgacaataca attattctct ttacctccga taatggtgcg gttatcgatg 3540

gtcctctgcc gctgaacggg gcgcaaaaag gctataagag tcagacctat cctggcggta 3600
ctcacacccc aatgtttatg tggtggagaa ggaaaacttc aacccggtaa ttatgacaag 3660
ctgatttccg caatggattt ctacccgaca gctcttgatg cagccgatat cagcattcca 3720
aaagacctta agctggatgg cgtttccttg ctgccctggt tgcaagataa gaaacaaggc 3780
gagccacata aaaatctgac ctggataacc tcttattctc actggtttga cgaggaaaat 3840
attccattct gggataatta ccacaaattt gttcgccata cagtcagacg attacccgca 3900
taaccccaac actgaggact taagccaatt ctcttatacg gtgagaaata acgattattc 3960
gcttgtctat acagtagaaa acaatcagtt aggtctctac aaactgacgg atctacagca 4020
aaaagataac cttgccgccg ccaatccgca ggtcgttata gagatgcaag gcgtggtaag 4080
agagtttatc gacagcagcc agccaccgct tagcgaggta aatcaggaga agtttaacaa 4140
tatcaagaaa gcactaagcg aagcgaaata actaaacctt catgcggcgg atttttccgc 4200
cgccttattg agcgagatag cgatgcacgt tacagccaag ccctccagtt ttcaatgtaa 4260
tctcaaatgt gattactgtt tttaccttga aaaagagtcg cagtttactc atgaaaaatg 4320
gatggatgac agcactttga aagagttcat caaacaatat atcgcagcgt ctggcaatca 4380
ggtctatttt acctggcaag gcggtgaacc cactctggct ggcctggatt ttttccgtaa 4440
agttattcac tatcaacaac gctatgcagg ccaaaaacgt atttttaatg cattacaaac 4500
gaatggcatt ttattgaata atgaatggtg tgccttctca aagaacatga atttctggtg 4560
gtatctcgat cgatggcccc caggagttac atgaccgtta cagacgcagt aattcaggta 4620
acggtacttt tgcaaaagtg atagcagcca tcgagcgtct gaaatcatat caagtagagt 4680
ttaatacgtt aaccgtcatt aataacgtta atgtccatta ccctcttgag gtttatcatt 4740
ttttaaaatc tatcggcagt aaacatatgc aatttatcga attgctagaa accgggacgc 4800
cgaatattga tttcagtggt catagtgaga acacattccg tatcattgat ttttctgtgc 4860
ctcccacggc ttatggcaag tttatgtcaa ccatttttat gcaatgggtt aaaaacgatg 4920
tgggtgaaat tttcatccgt cagtttgaaa gctt 4954

<210> 13
<211> 3796
<212> DNA
<213> Escherichia coli

<220>
<223> Designated as EC-39

<400> 13
aagcttaatc gcgtgaatca ggagtaaaaa aatgacaacc cagactgtct ctggtcgccg 60
ttatttcacg aaagcgtggc tgatggagca gaaatcgctt atcgctctgc tggtgctgat 120
cgcgattgtc tcgacgttaa gcccgaactt tttcaccatc aataacttat tcaatattct 180

ccagcaaacc tcagtgaacg ccattatggc ggtcgggatg acgctggtga tcctgacgtc 240
gggcatcgac ttatcggtag gttctctgtt ggcgctgacc ggcgcagttg ctgcatctat 300
cgtcggcatt gaagtcaatg cgctggtggc tgtcgctgct gctctcgcgt taggtgcgca 360
attggtgcgg taaccggggt gattgtagcg aaaggtcgcg tccaggcgtt tatcgctacg 420
ctggttatga tgcttttact gcgcggcgtg accatggttt ataccaacgg tagcccagtg 480
aataccggct ttactgagaa cgccgatctg tttggctggt ttggtattgg tcgtccgctg 540
ggcgtaccga cgccagtctg gatcatgggg attgtcttcc tcgcggcctg gtacatgctg 600
catcacacgc gtctggggcg ttacatctac gcgctgggcg acaacgaagc gacaacgcgt 660
ctttctggta tcaacgtcaa taaaatcaaa atcatcgtct attctctttg tggtctgctg 720
gcatcgctgg cgggatcata gaagtggcgc gtctctcctc cgcacaacca cggcggggac 780
tggctatgag ctggatgcta ttgctgcggt ggttctgggc ggtacgagtc tggcgggcgg 840
aaaaggtcgc attgttggga cgttgatcgg cgcattaatt cttggcttcc ttaataatgg 900
attgaatttg ttaggtgttt cctcctatta ccagatgatc gtcaaagcgg tggtgatttt 960
gctggcggtg ctggtagaca acaaaaagca gtaataacga ctacaggcac atcttgaata 1020
tgaacatgaa aaaactggct accctggttt ccgctgttgc gctaagcgcc accgtcagtg 1080
cgaatgcgat ggcaaaagac accatcgcgc tggtggtctc cacgcttaac aacccgttct 1140
ttgtatcgct gaaagatggc gcgcagaaag aggcggataa acttggctat aacctggtgc 1200
tggactccca gaacaacccg gcgaaagagc tggcgaacgt gcaggactta accgttcgcg 1260
gcacaaaaat tctgctgatt aacccgaccg actccgacgc agtgggtaat gctgtgaaga 1320
tggctaacca ggcgaacatc ccggttatca ctcttgaccg ccaggcaacg aaaggtgaag 1380
tggtgagcca cattgcttct gataacgtac tgggcggcaa aatcgctggt gattacatcg 1440
cgaagaaagc gggtgaaggt gccaaagtta tcgagctgca aggcattgct ggtacatccg 1500
cagcccgtga acgtggcgaa ggcttccagc aggccgttgc tgctcacaag tttaatgttc 1560
ttgccagcca gccagcagat tttgatcgca ttaaaggttt gaacgtaatg cagaacctgt 1620
tgaccgctca tccggatgtt caggctgtat tcgcgcagaa tgatgaaatg gcgctgggcg 1680
cgctgcgcgc actgcaaact gccggtaaat cggatgtgat ggtcgtcgga tttgacggta 1740
caccggatgg cgaaaaagcg gtgaatgatg gcaaactagc agcgactatc gctcagctac 1800
ccgatcagat tggcgcgaaa ggcgtcgaaa ccgcagataa agtgctgaaa ggcgagaaag 1860
ttcaggctaa gtatccggtt gatctgaaac tggttgttaa gcagtagttt taatcaggtt 1920
gtatgacctg atggtgacat aaatacgtca tcgacagatg aacgtgtaat ataaagaaaa 1980
gcagggcacg cgccacccta acacggtggc gcattttatg gacatcccga atatgcaaaa 2040
cgcaggcagc ctcgttgttc ttggcagcat taatgctgac cacattctta atcttcaatc 2100
ttttcctact ccaggcgaaa cgtaaccggt aaccactatc aggttgcatt tggcggcaaa 2160
ggcgcgaatc aggctgtggc tgctgggcgt agcggtgcga atatcgcgtt tattgcctgt 2220
acgggtgatg acagcattgg tgagagcgtt cgccagcagc tcgccactga taacattgat 2280
attactccgg tcagcgtgat caaaggcgaa tcaacaggtg tggcgctgat ttttgttaat 2340
ggcgaaggtg agaatgtcat cggtattcat gccggcgcta atgctgccct ttccccggcg 2400

ctggtggaag cgcaacgtga gcgtattgcc aacgcgtcag cattattaat gcagctggaa 2460
tcaccactcg aaagtgtgat ggcagcggcg aaaatcgccc atcaaaataa aaactatcgt 2520
tcgcttaacc cgctccggct cgcgaacttc ctgacgaact ctgcgctgtg gacattatta 2580
cgccaaacga aacggaagca gaaaagctca ccggtattcg tgttgaaaat gatgaagatg 2640
cagcgaaggc ggcgcaggta cttcatgaaa aaggtatccg tactgtactg attactttag 2700
gaagtcgtgg tgtatgggct agcgtgaatg gtgaaggtca gcgcgttcct ggattccggg 2760
tgcaggctgt cgataccatt gctgccggag atacctttaa cggtgcgtta atcacggcat 2820
tgctggaaga aaaaccattg ccagaggcga ttcgttttgc ccatgctgcc gctgcgattg 2880
ccgtaacacg taaaggcgca caaccttccg taccgtggcg tgaagagatc gacgcatttt 2940
tagacaggca gaggtgacgc ttggctacaa tgaaagatgt tgcccgcctg gcgggcgttt 3000
ctacctcaac agtttctcac gttatcaata aagatcgctt cgtcagtgaa gcgattaccg 3060
caaagtgagc gcgattaaag actcaattac gcgccatcag ctctggcgcg tagcctcaaa 3120
ctcaatcaaa cacataccat tggcatgttg atcactgcca gtaccaatcc tttctattca 3180
gaactggtgc gtgtcgttga acgcagctgc ttcgaacgcg gttatagtct cgtcctttgc 3240
aataccgaag gcgatgaaca gcggatgaat cgcaatctgg aaacgctgat gcaaaaacgc 3300
gttgatggct tgctgttact gtgcaccgaa acgcatcaac cttcgcgtga aatcatgcaa 3360
cgttatccga cagtgcctac tgtgatgatg gactgggctc cgttcgatgg cgacagcgat 3420
cttattcagg ataactcgtt gctgggcgga gacttagcaa cgcaatatct gatcgataaa 3480
ggtcataccc gtatcgcctg tattaccggc ccgctggata aaactccggc gcgctgcggt 3540
tggaaggtta tcgggcggcg atgaaacgtg cgggtctcaa cattcctgat ggctatgaag 3600
tcactggtga ttttgaattt aacggcgggt ttgacgctat gcgccaactg ctatcacatc 3660
cgctgcgtcc tcaggccgtc tttaccggaa atgacgctat ggctgttggc gtttaccagg 3720
cgttatatca ggcagagtta caggttccgc aggatatcgc ggtgattggc tatgacgata 3780
tcgaactggc aagctt 3796

<210> 14
<211> 6914
<212> DNA
<213> Enterobacter cloacae

<220>
<223> Designated as ET-49

<400> 14
aagcttttcg agttcgccat ccggcaacag ctcactgagc ttttacgcgc ccagggtgcc 60
tttgaactca attcccagct cagtaaggcg gtcctgaata atctctttgc gagatttttc 120
actggtaccg gcatcaggtg ttgcaggttt cagctcgcca ccagcctcgc ccttcatcag 180

ccggacgtta gacttcagcg ccgggtgaag atctttcaac tccaccacgt cgccaacctt 240
tacgccgaac catgggcgca caacttcgta tttagccatg ctgtttcctt acgccaggtt 300
agcgccgtag acaacgccag acaggcctga tcgtctgcag taatttgcag gccttcagca 360
gacatgatct ggaagttgta gttaacgtta ggcagtgggc gcggcagtgg cacaacgcca 420
acagccatac ccaccagtgg ggagatcacg tcacgacgac gaacgtacgc gataaactcg 480
ttaccggtca gcgcgaagtc atgcggattt ctttcaccgg tgcgaatggc agaacagcct 540
gcaggagagt gccgctcacc acaccattaa ctacgtatgg ctgagccata tttgcccaga 600
tctcagggga aacccacatc acatcatact gagctacttt gttggtgcgt gcggtggtac 660
cgaatgctcc tttaccaaag aactcaaaat attgagtcgt ggttgcgctg gtcaggtcga 720
tgttcgcacc accagcacca gaaccgaggt taatcttctt ggtgttgcgg tggttcttga 780
tgccctgcgc cgggtaggac tgaacctgaa tttttgaatc gccgttcagg tagtagttga 840
cgcgcttctg gttgaacttg cgcatcttcg ccatctgcga atccagaacc agatcaatgc 900
ctacagagtt aaggccagca gcatgacgcc agttaacacc gtagccagca gtgaacaccg 960
gaatcgggtc gccatcgctc gcgtagtcag tgtggtcgaa ggagaatggc gcctgaccat 1020
cgatgcttac tgacacgtcg tcagcgatgt cgccgaccac gttatacagc ttggcggttt 1080
taccaaccgg cagcacggtc tgaacgccga tcaggtcgtt tacgatttcc atgccaactt 1140
cctgatcccg cagctgcagc acctggttgt caatctcagc ccagaagtca cgggagaaac 1200
cgccaacagc gttacaagcc agcatgtcag gcgtcatcat tgcgcggtta gctgcaatga 1260
tggaatcgtt ctgtaggttc cacatgttgc ggtttgccca cagctcactc cagtgcccgc 1320
cgaggcggga gttagtcgcc agcgtctctt tagagaagta catatgtgtt tgtccttttg 1380
ttacgcgcca gctgcggcga cagtgccaac gcgcatacgc acgcgaatga agtcagtggt 1440
gctggccgcg atggtgtatt catcctggct gtagccgatc actgaatcag tgtcggatgt 1500
ggcaagggta aactgaccgg cagttcccag cttgatcggg ctgtcttttt tatacgcacc 1560
aggcaggcag cgcagcgcca gctcacgacc ttcttcgacg tagttaccta ctgccgaatc 1620
cccggcaggg atttcttcgg tgattgtcag gccctggtga taaccgacat cgatgatgta 1680
caggcggccg gttagcgcgg tggcctgagc gaatttatcg gatgagttga tggttgcggc 1740
ggtgccagga agcaacccgg cggccgttgt gcgggtttcg gtcttgtaca gagactgacc 1800
gtcgatatta acgcgacgat aacgtggcat tattccggct ccttacttga agtgttcgtc 1860
tgcggctggt gcgccggttt ctttgtgctg ctgagcattg ttggtgccca gcgacttgaa 1920
catcgcgtcc agagcttcgc ctgacagagc gttcgcgagc gatatcgcca tggaccttcg 1980
caaccgcttc gcgctttgct ttctcttcgg cacgggagtt cgcggtaagg gtttccgcga 2040
gttgcttctg attggcctgc agcgcatcaa ccttttccgc gagaggctta atagccgctt 2100
cagtattggt cgcaacagcc tggccgatca tgctgccgat ttgttccagt tcttctttgg 2160
ttaaaggcat gtcgcctccg ttttgtggtt tggtgcaggc tgttcctgcg gtgtgaatag 2220
agctttgaat tgttagcgac gactgccacc cacgactcct ggcgcgctac tgcggttccg 2280
gtatcgtcga ttgtgatctt cccgccatca gcgaataccg taaacctgag catcaccgcc 2340
atttcgcacg atgaccacct gcgagtcagt gagtcagcaa cccaggcata ttcatccgtg 2400

cccggcgcaa acttggcttt ggctgcccga tcgagacgct gctcgcgctc ccggtaggat 2460
tcacccacca gcgcgccgga gttcgcttta agcggctgcg ccagatcggc gtttaccatc 2520
aggccaacgc cctgctcagg ggtggcggct ccgacttcgt gcagtaggat cgcgtcgtgg 2580
tccatgctgt gaatcttcgc cacccactcg gcacccgtag ctctctgttg ttcgttaggc 2640
tcaagctggt cgaggaaagc ggcgacactg gtatgaatcg gcggaacgtc atcgccgcgc 2700
tcgatggctg cgacgcgctc aagtagttct cggccacctt cagactcacc ggcgcgggca 2760
acatcaaccc acttttcgag gtagatacga ttaccggact tcttaacgtt gcggttccac 2820
gcgccgatat ggcctgcgtt aatcccctcc ggggagaaag cagacacgaa ctgaccatta 2880
acctgagggt ggcccagcgg cgccagggta ccttccagcc ccttatagtg ggcgtcgatt 2940
tgctcttgcg tgtacaagcc gccattcatg acgacgttag ctggaagtgt gtagctcggc 3000
agcaccaggt gctcacgccc gttgtatgtt tcgcgccgga tagactggct gttcaccttt 3060
gtggtgatgt tgacctgaat atgctcacca tgtttcggtg cctggattgg acgctgtgct 3120
tcgtggttta cctggaattt catgagttat ttctccgccc aggcgtaacc gctcgcctgc 3180
atcgatttat attcctgttt gagtttcgtg atggtgtccg ggtattccgg cttgccgtcc 3240
gcatccacca gcaccgactg ctggctgcat ttgcagttga tggagttgcc atctttgctg 3300
taccagtcac gcacctcttc gttggtgtag aggtgggcat gggcgcactg cgtgggtatg 3360
tcgcgttgtc ggcgacagag ctgagatgtg aaccagcagc gttttaaggc cgaacaggtc 3420
attcgcctct tggtcttcat cccacttggc ccggcgcagc gcggtagtca cttcagtgcg 3480
tgctatccgg ttagcccggc gtttctcgat gccggtctgg tctgtcaggt tgcgggcaat 3540
gtccagagga ttgagcccgc gcccaacacc atcagtaaga cacgcgccat gtcgcgctta 3600
acgtcagccg tcagcccctt catttcctca aatacacgcg catgcaccag cgccatgcgt 3660
ttctgatact ggtcgcttgc gaggatggag gccagcgact cacgcccggc tgcgtacacc 3720
ggggattgct gactgaggtt gtagaacgac tgcccggtcc ctttttccga agccagatcg 3780
atgtactcgt aaaaccacag gtcgtaatcg ccaccttcaa gcagtacctg atcaaccagg 3840
taactggcat cgttcaggat gatggagagt agcattgggt ttagctggta ttcgtatctg 3900
gcgtttactg cgagggagga aggtattttg ttgagtgctg atttgtacgc cttgccaatc 3960
ttattcatcc gcctggcgaa gtctttcatt gcccggcgtt ccagcgcatc ggctccggtc 4020
ggatcctgat agttacgcgg cagaatcggt ggcttcgtct tcttcgtcgc catcctcttc 4080
tcctaatgga aattcatcga cgttttcata accggcagca gtgcggaatt tcttcacgac 4140
taaaggctgg tttttctccg ctcccctgga acgtctggtt aatctctgcc atggttttgg 4200
catttgcgag tttctcagtt ccagtctgtt cgttgaggtc atcccagata accgtcttct 4260
cgctgactgc atcaataatt ttcaggtcga tgagcttgtc actgaagtct tcaatttcga 4320
atgacaggtc accgcgccgt gactggcagc gcgcgttgaa atatttctga tcctcggtgc 4380
ttgccctttc acccgtctgc atcccaacca gaaccttcac agggatatca acagatgcag 4440
cgaaggtttg caggttgacg ttataggtcg ctgacggatc cgctacagct gtgaccagtg 4500
gtgtgactgt agccccttgg gttgtcatca gaacatcgtt accacggttc atttccccgg 4560
caacttcgtt aaacttatcc tgcaactcgt ccatgtcacg ccataaagtg acgcgagatt 4620

gttgaaatcg atttccttct caaagttgac attaagctgc cgcgcggcgt tctttaggaa 4680
tgactcacca gaaccaccct cgaccttctc aaggctgacg caggcgttat agccaggctc 4740
aaggaagcca atagcatcat tagaatagtc accaaggata aagacgcgat cgggatgtac 4800
gaagcgctga ttagttccac cgcttggaag gctctcaaca tatttccact gctttggctg 4860
cccgtagcct gccgatttct ggtcagttac ccactcgctg actgttaatg acccagccca 4920
tgcgatcgta acctttttta gtgacttgcc acgaacaaca ggctgatccc atgttctgga 4980
atcattgata tgcagcagga tacccgcata acgtccgacc tgtcggcggc ggtctgcttc 5040
agcaaaagcc cgccaaaggc gctttgtgaa aacctttttg gtgttcttct cccaggcagt 5100
ttcatcctta ctctcgtcgg catcatcacc ctcgatgatt tccgggttgg tctgccagca 5160
cttgcccacc agcttctcta ctgcgccgtg ggctattcca ccgcgacgat acagtgcgta 5220
gaggttttcg taagtgacct gctcagggaa tccatactcg caccatgcgg aatggcgctt 5280
attgtccagc cccattgtag gcgccaacag ccccatacgg gcacgggcca tccgcgcatc 5340
gttcaacgca tggttgacgg cgagagttaa tttgtcagtc atggtttgtc cgttggtgga 5400
tttaaggcat aaaaaaaggc cgctttggcg accttgtggc tatttaaaaa gctaaactct 5460
gttgaacgaa ataaacataa tctgctcagg cttaacgcca taatcacttg ccaacttctg 5520
agtgcactca attaagacag ttgatgcaga tttcgaagag cttgcaccat aaatttcgaa 5580
gttttcaaat actccgccgt tggtgtggta aatcttatat gacataaacc aatcattcat 5640
aatatctact cccttacaga attgagtaga tattatcggc aagtgcatat gtttctttaa 5700
attatctcaa ccttttcggg atcatcatcc cggccatctg gcccttacgt ttaatgtgtc 5760
cgtcgaggct gtagcgaata ccgtcccagc agtgttcgta accgtctgcc agtttaggca 5820
atacctcgcc ggtgatgcgg tccgttttgt aggaccacat gcgggcctct ctcgccacat 5880
tcttgcagcg aggatggata atgatttcgt caaagccgcg aagatgcgcg ataccgtcct 5940
caacactccc ctgccatttc tcggcagccg agatgttgaa gccctggcgc ttgagatagc 6000
tgatagtctc gggtcgggcg gagtcggcct tgatgggcca gtcacgcgat ccggggattg 6060
tgtcgtatag ctctggcata tggtcgagct ctgtctgctg accgtatgcc tcgtattcga 6120
tgtacagccg gttgtgcagg atgaacgagc gcaccagcgt gttagggtct ttggcgaaac 6180
cgaagtcagc accgaagaaa aggcgatcgg cctctttcca tagctggtcc gagaactcag 6240
cgatccggta tttaccggcc agcacctgct tatcagagtt ttcgaggtaa gcaccttccc 6300
aaacccacgc gtatgttgcc gggtcaaggc ggcgctgatc gttctgtcgc tcaccttcca 6360
gcacgtcggg gaaccatgga ttatccgtgt agttcatctc aacgtgatac agtcgtcgcc 6420
agcctcttta cggaaacgct tatccgtgcg ctgccgtcgc gctccgggtt ccatgtcacc 6480
caaatctctg aaccttcctc acgaacggtc gggctcagct tctgccaggc tatttcgctg 6540
actgattcag cctcatcaac ccaacagagc aagatgcgcg ctttcgactt gatgctgtcg 6600
aggttatgcc gcagaccgca gaacacgtag ttaacgctct tgtcgatggt gcggatgtac 6660
ttctcgccga tatcaaagtt ggaagccagc cagggaacag acaggatagc ctgtttcacc 6720
tcctgcatac tcgactcttc cagtgagttc atgaattcac gcgcacagag caccacgccg 6780
ctttcaccgt tcatcatcga ctgatacgcc tttacggctg tcatcagcgc aaaagtgcgc 6840

gtcttggcac taccacgccc accatgcgag caccggtaac gcttattctc ggcgatgaac 6900
agtggcgcaa gctt 6914

<210> 15
<211> 5975
<212> DNA
<213> Klebsiella pneumoniae

<220>
<223> Designated as KI-50

<400> 15
aagcttattc cacgctggag gcgtccggga ttatcggcgt caacgctatc gccggcatcg 60
ccgggaccat catcgccggc atgctctccg accgcttttt caaacgcaac cgcagcgtga 120
tggccggatt catcagcctg ctgaacaccg ccggcttcgc cctgatgctc tggtcgccgc 180
acaattacta cactgatatt ctggcgatga ttatcttcgg ggccaccatt ggcgctctga 240
cctgcttcct tggcgggctg atcgccgtcg atatctcttc gcgcaaggcc gccggggccg 300
cgctcggcac catcggcatc gcagctacgc cggcgccggc ctgggcgagt ttctcaccgg 360
gttcattatt gataaaacgg ctatccttga aaacggcaaa acgctgtatg atttcagcac 420
gttggcgctg ttctgggtgg gtacggtctg ggttcngcgc tactctgttt taccactgcc 480
gccatcgtcg cccggcgcca tgccgtcgaa cggcagacct cgttctcctc ataaccgatt 540
aacgaataag gaagaagata tgatgcctgc aagacatcag ggctgttac gcctgtttat 600
cgcctgcgcg ctgccgctgc tggcgctgca atctgccgcc gccgcggact ggcagctgga 660
gaaagtggtc gagctcagcc gccacggtat tcgtccgccg acggccggca accgggaagc 720
catcgaggcc gccaccggcc gaccgtggac cgagtggacc acccatgacg gggagctcac 780
cggccatggc tatgccgccg tggtcaacaa agggcgtgcg gaaggccagc attaccgcca 840
gctcggcctg ctgcaggccg gatgcccgac ggcggagtcg atatacgtgc gcgccagccc 900
gctgcagcgg acgcgagcga ccgcccaggc gctggtggat ggcgccttcc ccggctgcgg 960
cgtcgctatc cattatgtca gcggggatgc cgatcccctg tttcagaccg acaagttcgc 1020
cgccacgcaa accgaccccg cccgccagct ggcgcggtga aagagaaggc cggggatctg 1080
gcgcaggtcg gcaggcgctg gcgccgacca tccagctatt gaaacaggcg gtttgtcagg 1140
ccgataagcc ctgcccgatc ttcgataccc cgtggcaggt cgagcagagc aaaagtggga 1200
agaccaccat tagcggactg agcgtgatgg ccaatatggt ggagacgctg cgtctcggct 1260
ggagtgaaaa cctgcctctc agccagctgg cgtggggcaa gatcacccag gccaggcaga 1320
tcaccgccct gctgccgctg ttaacggaaa actacgatct gagtaacgat gtgttgtata 1380
ccgcgcaaaa acgcgggtcg gtgctgctca acgctatgct cgacggcgtc aaaccggagc 1440
gaatcgaacg tacgctggct gctgctggtg gccatgacac caatatcgcc atggtgcgca 1500

cgctgatgaa ctttagctgg cagctgccgg gctacagccg gggaaatatc ccgccgggca 1560
gcagcctggt gctggagcgc tggcgcaacg cgaagagcgg agaacgctat ctgcgggtct 1620
atttccaggc ccagggcctc gacgacctgc gtcgtctgca gacgccggac gcgcagaccc 1680
cgatgctgcg tcaggagtgg catcagccgg gctgccgtca gaccgatgtc ggtacgctgt 1740
gtcccttcca ggcggctatt accgccctcg gtcagcgtat cgaccgatca tccgccccgg 1800
cggtagcatg gtcctgccgt agcggcgcgg tgtttgtccg ggcccgggaa aacctttttt 1860
tccaggccgg cacgacgtcc gttatccgtt gtccggcgca aacgccccgg cggcgacctg 1920
cgccggggtg acacccgctg tccagcaccc agccgcttat cagcccagca ggcgtgacgt 1980
cgaacgccgg attgtaaacg gtggcccccg tcggcgccca ctgtaccgcg ccgaagctgc 2040
ccgccactcc ggtcacttcc gccgccgcgc gctgctcaat ggggatcgcc gccccgttcg 2100
ggcaatggcg gtcgagggtg gtctgcgggg cagcgacgta aaacgggatc tggtgataat 2160
gggccaaaac cgccagagaa taggtgccga ttttattcgc cacgtcgccg ttggcggcga 2220
tacggtcggc gccgacccac accgcatcca cctgcccctg cgccatcagg ctggcggcca 2280
ttgaatcggc gatcagctga tagggcacgc ccagctcgcc cagctcccag gcggttaaac 2340
gaccgccctg cagcagcggc cgggtttcat caacccatac gttggtcact tttccctgcc 2400
ggtgcgccag cgcgataacg ccgagggcgg tccctacccc ggcggtcgcc aggccaccgg 2460
tgttgcagtg ggtcagcagt cgactgccgg gcttcaccag cgcactgccc gcctcagcga 2520
tgcggtcgca cagctgttta tcttcttcga ccagacgcaa ggcttccgct tccagcgcct 2580
gcgggtaatc tccgggccag cgctgcttca tgcgatcaga ttattcatca ggttgaccgc 2640
cgtcggccgc gccgcgcgca gtctccagcg cctgctggag tgcatcccgg ttcaggccgc 2700
gctgggccag cagggccagc agcaggctgg cggacaggcc aatcagcggc gcgccgcgca 2760
ccccgcaggt atgaatatgg tccaccagca gcgcaacgtt atccgccgcc agccagcgtt 2820
tttcctgcgg caaggcctgc tggtcgagaa taaaaagctg attttcactc acccgcaggc 2880
tggtggtctg taatgtctgc atgtcgttaa atccctgttg cgttgttgta tcacattgtg 2940
tcaggatgga atccagaagt atagacgtct gaacggctta atcagaattc gaggatcgag 3000
gcaatgtcgc aataccatac cttcaccgcc cacgatgccg tggcttacgc gcagagtttc 3060
gccggcatcg acanccatct gagctggtca gcgcgcagga agtgggcgat ggcaactcaa 3120
tctggtgttt aaagtgttcg atcgccaggg cgtcacgggc gatcgtcaaa caggctctgc 3180
cctacgtgcg ctgcgtcggc gaatcctggc cgctgaccct cgaccgcgcc cgtctcgaag 3240
cgcagaccct ggtcgcccac tatcagcaca gcccgcagca cacggtaaaa atccatcact 3300
ttgatcccga gctggcggtg atggtgatgg aagatctttc cgaccaccgc atcttgcgcg 3360
gagagcttat cgctaacgtc tactatcccc aggcggcccg ccagcttggc gactatctgg 3420
cgcaggtgct gtttcacacc agcgatttct acctccatcc ccacgagaaa aaggcgcagg 3480
tggcgcagtt tattaacccg gcgatgtgcg agatcaccga ggatctgttc tttaacgacc 3540
cgtatcagat ccacgagcgc aataactacc cggcggagct gggaggccga tgtcgccgcc 3600
ctgcgcgacg acgctcagct taagctggcg gtggcggcgc tgaagcaccg tttctttgcc 3660
catgcggaag cgctgctgca cggcgatatc cacagcgggt cgatcttcgt tgccgaaggc 3720

agcctgaagg ccatcgacgc cgagttcggc tacttcggcc ccattggctt cgatatcggc 3780
accgccatcg gcaacctgct gcttaactac tgcggcctgc cgggccagct cggcattcgc 3840
gatgccgccg ccgcgcgcga gcagcggctg aacgacatcc accagctgtg gaccaccttt 3900
gccgagcgct tccaggcgct ggcggcggag aaaacccgcg acgcggcgct ggcttacccc 3960
ggctatgcct ccgcctttct gaaaaaggtg tgggcggacg cggtcggctt ctgcggcagc 4020
gaactgatcc gccgcagcgt cggactgtcg cacgtcgcgg atatcgacac tatccaggac 4080
gacgccatgc gtcatgagtg cctgcgccac gccattaccc tgggcagagc gctgatcgtg 4140
ctggccgagc gtatcgacag cgtcgacgag ctgctggcgn gggtacgcca gtacagctga 4200
gtgcgcctgt ttccctcacc ccaaccctct cccacaggga gagggagcac cccctaaaaa 4260
agtgccattt tctgggattg cccggcgngn tgcgcttgcc gggcctacag atagccgcat 4320
aacggtttga tcttgcactc tttcgtaggc cgggtaaggc gaaagccgcc acccggcaga 4380
catgcgagta caattttgca tttaccttac cctcacccca gatactcaat caccgatagc 4440
ccgccgttgt aatcggtgct gtagataatg ccttgcgcat cgacaaacac gtcacaggac 4500
tggatcaccc gcgggcggcc gggacgggta tccatcattc tctcagcgca gccggcacca 4560
gcgccccggt ctccagcggg cgatacgggt tggaaatgtc gtaagcccgc acgccggcat 4620
tctgatacgt ggcaaaaatc agcgttgagc tgacaaagct ccccggccgg ttctcatgca 4680
ggttgtgcgg accgaaatgc gccccttcg ccacgtaatc cgcttcatcc ggcggcggga 4740
aggtggcgat gctcaccggg ttggttggct cgcggatatc aaacagccag atcagcttct 4800
cgccgtcctc ctggttatcg agcaccgctt catccagcac caccagcaga tcgcgatccg 4860
gcagcggcag cgcggtatgc gttccgccgc cgaacggcgg gctccagttg cgatggctaa 4920
tcagcctcgg ctgggtacgg tctttgacat ccagcagcgt caggccgccg tcgcgccagc 4980
tgcgtaggcg tatccccggc aataatggcg tgatgcagcg catagcgttt gccctgcggc 5040
cagtccggtg tttcaccgcc cgcctggtgc atccccggca gccaccagcg cccggctact 5100
tcgggcttac gcggatcggc cagatcgatg gtcaggaaga tgtagtcggt aaaaccgtcg 5160
atcagcgcag acacatacgc ccagcgcccg ccgacgtacc agatgcggtg aataccgatg 5220
ccgttaagcg acaggaaact gatttcccgc gctgcgcggg agtggaaata tcaaagatgc 5280
gcagcccggc gctccagccc ctgtcctgca catcgctgac cgtgtcaccc accgagcggg 5340
tgtagtacac cttctcatca gcaaaacggg cgtcagcaaa cagatcccgg gcgttgatca 5400
ccagcagcag atcgtcatgc gcctggagtg cacgttccag gtgcccggcg gcgcggcaat 5460
atagttgacg gtggtgggcc gggtcggatc gcgaacatcg accacggaaa aaccctgcga 5520
caccatatgg ccgatatagg cgaatccgcg gtgcaccatc agctgcacgc cgtccggacg 5580
accgccctga tcgctatggc caatcagccg catattgcgg ctgtattcgg gggaaggtaa 5640
tgctgacata ggggatccct ctcgcccggt ggcatggttt tcccccctct cctgcggaga 5700
gggccggggc gagggcacca ggccgccgcc caccgccacc cggcttgatt ttatttgttc 5760
ttcgcttcca gcgtcgcgaa ccacggcgcg ataaagtctt cggtctggcc ccagccaggg 5820
ataattttcc ccagcgacgc cacgtttacc gctcccggct gggccgccag cagcgcctgg 5880
ggaatcgctg ccgccttgaa gtcgtaggtg gctggcgtcg gctcgccggc gatcttgttg 5940

gcgatcagcc gcacgttggt cgcgccgata agctt 5975

<210> 16

<211> 899

<212> DNA

<213> Candida albicans

<220>

<223> Designated as CA-26

<400> 16

gaattcctag taagcgcaag tcatcagctt gcgttgatta cgtccctgcc ctttgtacac 60
accgcccgtc gctactaccg attgaatggc ttagtgaggc ctccggattg gtttaggaaa 120
gggggcaacc tcattctgga accgagaagc tggtcaaact tggtcattta gaggaagtaa 180
aagtcgtaac aaggtttccg tagtgaacct gcggaaggat cattactgat ttgcttaatt 240
gcaccacatg tgtttttctt tgaacaaact tgctttgcgg tgggcccagc ctgccgccag 300
aggtctaaac ttacaaccaa ttttttatca acttgtcaca ccagattatt acttaatagt 360
caaacttcaa caaacggatc tcttggttct cgcagcgaaa tgcgatacgt aatatgaatt 420
gcagatattc gtgaatcatc gaatctttga acgcacattg cgccctctgg tattccggag 480
ggcatgcctg tttgagcgtc gtttctccct caaaccgctg ggtttggtgt tgagcaatac 540
gacttgggtt tgcttgaaag acggtagtgg taaggcggga tcgtttgaca atggcttagg 600
tctaaccaaa aacattgctt gcggcggtaa cgtccaccac gtatatcttc aaactttgac 660
ctcaaatcag gtaggactac ccgctgaact taagcatatc aataagcgga ggaaaagaaa 720
ccaacaggga ttgcctcagt agcggcgagt gaagcggcaa aagctcaaat ttgaaatctg 780
gcgtctttgg cgtccgagtt gtaatttgaa gaaggtatct ttgggcccgg ctcttgtcta 840
tgttccttgg aacaggacgt cacagagggt gagaatcccg tgcgatgaga tgacccggg 899

<210> 17

<211> 189

<212> DNA

<213> Candida albicans

<220>

<223> Designated as CA-26-1

<400> 17

gcgcaagtca tcagcttgcg ttgattacgt ccctgccctt tgtacacacc gcccgtcgct 60

actaccgatt gaatggctta gtgaggcctc cggattggtt taggaaaggg ggcaacctca 120
ttctggaacc gagaagctgg tcaaacttgg tcatttagag gaagtaaaag tcgtaacaag 180
gtttccgta 189

<210> 18
<211> 224
<212> DNA
<213> Candida albicans

<220>
<223> Designated as CA-26-2

<400> 18
gctgggtttg gtgttgagca atacgacttg ggtttgcttg aaagacggta gtggtaaggc 60
gggatcgttt gacaatggct taggtctaac caaaaacatt gcttgcggcg gtaacgtcca 120
ccacgtatat cttcaaactt tgacctcaaa tcaggtagga ctacccgctg aacttaagca 180
tatcaataag cggaggaaaa gaaaccaaca gggattgcct cagt 224

<210> 19
<211> 369
<212> DNA
<213> Candida albicans

<220>
<223> Designated as CA-26-3

<400> 19
aaacggatct cttggttctc gcagcgaaat gcgatacgta atatgaattg cagatattcg 60
tgaatcatcg aatctttgaa cgcacattgc gccctctggt attccggagg gcatgcctgt 120
ttgagcgtcg tttctccctc aaaccgctgg gtttggtgtt gagcaatacg acttgggttt 180
gcttgaaaga cggtagtggt aaggcgggat cgtttgacaa tggcttaggt ctaaccaaaa 240
acattgcttg cggcggtaac gtccaccacg tatatcttca aactttgacc tcaaatcagg 300
taggactacc cgctgaactt aagcatatca ataagcggag gaaaagaaac caacagggat 360
tgcctcagt 369

## Description

[Technical Field]

The present invention relates to a process for evaluating a phagocytotic function, and more particularly, provides an experimental model of an infection which is useful in diagnosis of an infectious state by a foreign microorganism, such as for example, sepsis and the like by bacteria, fungi or the like, or in development of therapeutic drugs for an infectious disease. In addition, the present invention also relates to a process for detecting, identifying and evaluating phagocytotic ability and/or germicidal capacity by a phagocyte; a process for the determination of an effect of a modulator of a phagocytotic function; a process for screening a modulator of a phagocytotic function; and a kit for putting any of such processes into practice.

[Background Art]

Infectious diseases and sepsis are often caused due to the underlying disease which had been presented previously, through infection by an attenuated microorganism. Although such a state may frequently occur in a clinical scene, ideal animal models which cover all of the clinical symptoms have not yet established. Factors for such a current state involve complicated infectious conditions exhibited by a bacterial infection owing to the difference in the underlying disease, large gaps of sensitivity and the like of the animal spices toward the bacterial strain, and thus, systems of the infection model have been individually established depending on the purpose of the research. Examples of the system of the infection model which are well known at present include: (i) a process in which formation of intraabdominal abscess is allowed through the infection of any of various microorganisms into a peritoneal cavity of a mouse or a rat to chase the pathological state of sepsis (biphasic infection theory), (ii) particularly, in instances to study dynamics of the infection under a lowered immune state, a process in which infection of an attenuated microorganism such as Pseudomonas aeruginosa or the like is rendered through decreasing leukocytes by previously administering cyclophosphamide, and a process in which adhesion of *Pseudomonas aeruginosa* is allowed through making burn injury in a wide area of the skin with an electric trowel in order to facilitate the infection, (iii) in instances to examine dynamics of a living humoral factor such as cytokine released by a macrophage, neutrophil and the like, a process in which a pathological state of sepsis caused by the administration of an *Escherichia coli* related bacterium such as *Escherichia coli* and LPS of the same is observed, (vi) to determine the dynamics of tissue images of MOF caused by peritonitis observed in ICU and digestive surgery through allowing invasion of an enteric bacterium by cecal ligation and puncture (CLP) of a cecum of a rat (subacute superinfectious peritonitis model); and the like.

Requirements for preferred animal model include: (I) possible migration of bacteria from a primary focus of the infection into blood (direct administration of bacteria within blood causes bacterial shock in many cases, which can not be controlled as a pathological state), (II) capability of securing sufficient amount of the blood when comparison and examination is executed with time by both test processes, and of performing collection of blood without causing contamination and secondary infection at the site of blood collection, (III) capability of securing phagocytes in an identical amount to that in human because less phagocytes such as neutrophils may be present depending on the animal spices and age of weeks, (IV) no great influence on each individual and detection sensitivity by alteration of an immune system by the stress and shock upon blood drawing through frequent collection of blood, (v) possible securement of number of experiments to some extent such that individual difference is avoided, and the like.

Biphasic infection theory that is the most general process as a system to produce an animal model is a historically conventional system, which starts in 1931 by Meleney et al, and investigated and established by Hite (1949), Mergehagen (1958) and McDonald (1963) et al. This model has been established on the basis of a theoretical ground of an infection route in which a secondary infection focus is formed from the bacterium of a primary focus via blood irrespective of whether the bacterium is an anaerobe or aerobe. Thus, this model has been generally used as an infection model of sepsis. However, because this system was established as a system for use in analysis of pathologic states of bacterial infectious disease, no importance is attached to the amount of bacteria which migrate from the abdominal cavity into the blood. Therefore, because the amount of bacteria which was intraperitoneally administered is not reflected to the amount of bacteria in the blood due to the influence of the individual difference of each rat, it is difficult to consider the difference resulting from the administered amount of bacteria in an *in vivo* test.

In addition, Bacterial Translocation methods also involve problems. A factor for impossibility of easy comparison of the detection sensitivity of attenuated infectious bacteria such as *Escherichia coli, Enterobactor cloacae, Klebsiella pneumoniae, Enteroccocus faecalis, Staphylococcus epidermidis* and the like may involve that these bacterial strains are indigenous bacteria which are enteric and mucosal. In this *in vivo* test system, analysis of a pathogenic state of sepsis is intended rather than the dynamics of the administered bacteria, therefore, invasion of a bacterial strain other than the administered bacteria is not considered. Recently, also in clinical scene and animal experiments, it has been argued that enteric canal permeability is promoted by peritonitis, and that sepsis is caused through migration of enteric bacteria into the blood. Further, in clinical scene, there exist cases in which the primary focus can not be specified in MOF resulting from peritonitis, and thus attention has been drawn of the relationship with bacterial translocation.

Moreover, in an animal experiment, it was reported that in cases of intraperitoneal administration of *Enterococcus faecalis* in this *in vivo* test model, bacterial translocation from the enteric canal was caused due to the inflammation stress by peritonitis as an attraction, and thus, *Enterococcus faecalis* in the enteric canal was separated at the ratio of 33% (9/27). In addition, Steffen et al. also acknowledged that many enteric bacteria migrate into blood in this *in vivo* test model.

Further, although not by the bacterial translocation, in peritonitis caused by a cecum ligature puncturing method for use in rat sepsis models, it is also reported that *Escherichia coli, Enterobactor cloacae, Klebsiella pneumoniae, Enteroccocus faecalis* and *Staphylococcus epidermidis* were reparated at 12 hours later from the blood.

Because relationships between the causative microorganism of an infectious disease and the host is extremely complicated, it is further difficult to establish an ideal animal model which mimics various human infectious diseases, e.g., sepsis and bacteremia. Thus, experimental models of infectious diseases have been desired which enable the *in vitro* evaluation of phagocytotic ability and/or germicidal capacity of phagocytes, and which are stable and can be widely applied irrespective of species of the foreign microorganism, while retaining the morphology of the phagocyte, as an aid in diagnoses of infectious diseases including bacteremia and sepsis, and in determination of drug efficacy for developing therapeutic drugs for an infectious disease, however, current status is that those which satisfactorily meet the demand have not been provided.

[Disclosure of Invention]

An object of the present invention is to provide an experimental model which permits the evaluation of a phagocytotic function of phagocytes *in vitro*, taking into account of such a current status. Moreover, another object of the present invention is to provide a process which permits the evaluation of an immune function and efficiency of differentiation to phagocytes through the use of such an experimental model. Furthermore, still another object of the present invention is to provide a process for screening various kinds of drugs such as immune function stimulators, anticancer agents, leukocyte differentiation factors, antibiotics and the like; a process for clinical laboratory test in which dosage regimen of various agents are examined; and the like by using such an experimental model. Additionally, provided is a process in which performance tests such as sensitivity tests, specificity tests, reproducibility tests and the like are conducted, or in which the aforementioned experimental model is used as a positive control, by a kit through: obtaining phagocytes from a clinical specimen containing phagocytes derived from a living body; fixing thus resulting phagocytes; executing a treatment for promoting permeability of the cell membranes; executing a treatment for exposing the DNA of a foreign microorganism predicted as existing in the phagocytes; carrying out *in situ* hybridization using a DNA probe for detection capable of hybridizing with the DNA under a stringent condition; and detecting and/or detecting the foreign microorganism by the resulting signal.

The present invention was accomplished in light of the current status described hereinabove in detail, and aspects thereof are as described in the following Items 1 to 40.

1. A digested phagocyte prepared by contacting *in vitro* a phagocyte with a foreign microorganism and isolating the phagocyte so contacted.

2. The digested phagocyte according to Item 1 wherein a turbidity of bacterial liquid (O.D. = 600 nm) of the foreign microorganism used for *in vitro* contact between the phagocyte and the foreign microorganism is 0.01 to 0.03.

3. The digested phagocyte according to Item 1 or 2 wherein a density of the phagocyte digested with the foreign microorganism is $1 \times 10^4$ cells/μl to $5 \times 10^4$ cells/μl.

4. The digested phagocyte according to any one of Items 1-3 wherein said foreign microorganism is a gram negative bacterium.

5. The digested phagocyte according to any one of Items 1-3 wherein said foreign microorganism is one or more microorganism selected from the group consisting of *Staphylococcus aureus, Staphylococcus epidermidis, Enterococcus faecalls, Pseudomonas aeruginosa, Escherichia coli* and *Candida albicans,* and a mixture thereof.

6. A process for producing a phagocyte digested with a foreign microorganism comprising the steps of:

contacting *in vitro* a phagocyte with a foreign microorganism; and

isolating the phagocyte.

7. The process according to Item 6 wherein a turbidity of bacterial liquid (O.D. = 600 nm) of the foreign microorganism used for *in vitro* contact between the phagocyte and the foreign microorganism is 0.01 to 0.03.

8. The process according to Item 6 or 7 wherein a density of the phagocyte digested with the foreign microorganism is $1 \times 10^4$ cells/μl to $5 \times 10^4$ cells/μl.

9. The process according to any one of Items 6-8 wherein said foreign microorganism is a gram negative bacterium.

10. The process according to any one of Items 6-8 wherein said foreign microorganism is one or more microorganism selected from the group consisting of *Staphylococcus aureus, Staphylococcus epidermidis, Enterococcus faecalis, Pseudomonas aeruginosa, Escherichia coli* and *Candida albicans*, and a mixture thereof.

11. A process for detecting and/or identifying a digested foreign microorganism comprising the steps of:

fixing the phagocyte digested with a foreign microorganism according to any one of Items 1-5;
treating to promote permeability of the cell membrane of the phagocyte;
treating to expose DNA of the foreign microorganism existing in the phagocyte;
*in situ* hybridizing under a stringent condition between a DNA probe which can detect hybridization and the DNA; and
detecting and/or identifying the digested foreign microorganism by the resulting signal.

12. A process for evaluating a phagocytotic function against a foreign microorganism comprising the steps of:

fixing the phagocyte digested with a foreign microorganism according to any one of Items 1 to 5;
treating to promote permeability of the cell membrane of the phagocyte;
treating to expose DNA of the foreign microorganism existing in the phagocyte;
*in situ* hybridizing under a stringent condition between a DNA probe which can detect hybridization and the DNA; and
identifying by the resulting signal the phagocytosis and/or killing ability of the phagocyte against the foreign microorganism.

13. The process according to Item 11 or 12 wherein said process includes at least one aspect of:

(1) the density (X cells/ml) of the phagocytes to be fixed is $5\times10^6$ cells/ml < X cells/ml < $1\times10^8$ cells/ml;
(2) in said exposing step of the DNA, lysostafin having the titer of 1 unit/ml to 1,000 unit/ml is used;
(3) in said exposing step of the DNA, lysozyme having the titer of 1,000 unit/ml to 1,000,000 unit/ml is used;
(4) in said exposing step of the DNA, N-acetylmuramidase having the titer of 10 unit/ml to 10,000 unit/ml is used;
(5) in said exposing step of the DNA, zymolase having the titer of 50 unit/ml to 500 unit/ml is used;
(6) in said *in situ* hybridization step, a surfactant is used;
(7) said DNA probe for detection is one or more DNA probe having the chain length of 350 to 600 base length; and
(8) the concentration of said DNA probe for detection is 0.1 ng/μl to 2.2 ng/μl.

14. The process according to Item 13 wherein one or more enzyme selected from lysostafin, lysozyme, N-acetylmuramidase and zymolase is used in said exposing step of the DNA, with the titer of lysostafin being 10 unit/ml to 100 unit/ml; the titer of lysozyme being 10,000 unit/ml to 100,000 unit/ml; the titer of N-acetylmuramidase being 100 unit/ml to 1,000 unit/ml; and the titer of zymolase being 100 unit/ml to 500 unit/ml.

15. The process according to any one of Items 11 to 14 wherein an enzyme is used in said exposing step of the DNA, and wherein the temperature to allow the reaction of the enzyme is 26°C to 59°C, with the time period of the reaction of the enzyme being 15 minutes to 120 minutes.

16. The process according to any one of Items 11 to 15 wherein a substance for retaining the morphology of the phagocyte is additionally used in said exposing step of the DNA.

17. The process according to Item 16 wherein said substance is phenylmethylsulfonyl fluoride.

18. The process according to Item 17 wherein the concentration of said phenylmethylsulfonyl fluoride is 10 μmol/l to 10 mmol/l.

19. The process according to any one of Items 16 to 18 wherein said substance is a substance dissolved in dimethylsulfoxide.

20. The process according to Item 19 wherein the concentration of said dimethylsulfoxide is less than 5%.

21. The process according to any one of Items 11 to 20 wherein the DNA and the DNA probe is hybridized in the presence of a surfactant in said *in situ* hybridization step.

22. The process according to Item 21 wherein said surfactant is an anion surfactant.

23. The process according to Item 22 wherein said anion surfactant is sodium dodecylsulfate.

24. The process according to any one of Items 11 to 23 wherein the temperature to allow the hybridization reaction is 25°C to 50°C, with the time period of the hybridization reaction being 30 minutes to 900 minutes in said *in situ* hybridization step.

25. A process for evaluating a phagocytotic function against a foreign microorganism comprising the steps of:

fixing the digested phagocyte according to any one of Items 1 to 5;
staining the phagocyte with a dye; and
identifying the phagocytosis and/or killing ability of the phagocyte against the foreign microorganism by the detection through observation by microscopic examination on cell morphology which is characteristic in cells during or after phagocytosis.

26. A process for evaluating an immune function comprising the steps of:

isolating phagocytes from a subject;
evaluating a function of the phagocytes using the process for evaluating a phagocytotic function according to any one of Items 12 to 25; and
evaluating the immune function of the subject by comparing the evaluation result to that of the function of normal phagocytes.

27. The process according to Item 26 wherein said immune function is a phagocytotic ability of a microorganism by a leukocyte.

28. The process according to item 27 wherein said immune function is a phagocytotic ability against a microorganism by a leukocyte of a patient who received the radiation exposure or the administration of an anticancer agent.

29. A process for evaluating differentiation efficiency into a phagocyte comprising the steps of:

evaluating a phagocytotic function against a foreign microorganism according to any one of Items 12 to 25; and
evaluating the phagocytotic function in a time dependent manner to identify the alteration.

30. A process of the evaluation for determining an effect of a modulator of phagocytotic function comprising the steps of:

allowing phagocytosis by incubating a suspension of a foreign microorganism and phagocytes in the presence and absence of a phagocytotic function modulator; and
comparing the phagocytotic function in the presence and absence of said phagocytotic function modulator using the process for evaluating a phagocytotic function against a foreign microorganism according to any one of Items 12 to 25.

31. A process for screening a modulator of phagocytotic function comprising the steps of:

allowing phagocytosis by incubating a suspension of a foreign microorganism and phagocytes in the presence and absence of a candidate agent supposed to have a modulatory action toward the phagocytotic function; and comparing the phagocytotic function in the presence and absence of said agent using the process for evaluating a phagocytotic function against a foreign microorganism according to any one of Items 12 to 25.

32. A clinical testing process comprising the steps of:

obtaining phagocytes from a subject prior to and following the administration of an agent to the subject;
evaluating a function of the phagocyte using the process for evaluating a phagocytotic function according to any one of Items 12 to 25; and
examining a dosage regimen of the agent judging from the effect of the agent determined on the basis of the evaluation result.

33. A performance testing process of a kit for evaluating a phagocytotic function which comprises fixing phagocytes, treating to promote permeability of the cell membranes of the phagocytes, treating to expose the DNA of a foreign microorganism in the phagocytes, *in situ* hybridize under a stringent condition between the DNA and a DNA probe which can detect hybridization; and evaluating the phagocytotic function by the resulting signal, said kit has;

(1) the foreign microorganism,
(2) at least one or more enzyme(s) selected from the group consisting of lysostafin, lysozyme, N-acetylmuramidase and zymolase used in said exposing step of the DNA, and
(3) one or more DNA probe(s) for detection,

said process is characterized in that the digested phagocyte according to any one of Items 1 to 5 is used.

34. A performance testing process of a kit for detecting and/or identifying a foreing microorganism which comprises obtaining phagocytes from a clinical specimen containing phagocytes derived from a living body, fixing the phagocytes so obtained, treating to promote permeability of the cell membranes of the phagocytes, treating to expose the DNA of the foreign microorganism predicted as existing in the phagocytes, *in situ* hybridizing under a stringent condition between the DNA and a DNA probe which can detect hybridization, and detecting and/or identifying the foreign microorganism by the resulting signal,
the process is characterized in that the digested phagocyte according to any one of Items 1 to 5 is used.

35. The performance testing process according to Item 33 or 34 wherein said performance test is a sensitivity test, a specificity test or a reproducibility test.

36. The performance testing process according to Item 33 or 34 wherein the digested phagocyte according to any one of Items 1 to 5 is used as a positive control.

37. The process according to any one of Items 11 to 36 wherein the process further comprises a step prior to said fixing step to put the digested phagocyte onto a solid support which is a slide glass coated with 3-aminopropyltriethoxysilane.

38. The process according to any one of Items 11 to 37 wherein a dye for clarifying the contrast between the signal and the cell is used upon the detection of said signal.

39. The process according to any one of Items 11 to 38 wherein said phagocyte is from blood.

40. A kit for evaluating a phagocytotic function by fixing the digested phagocytes according to any one of Items 1 to 5, treating to promote permeability of the cell membranes of the phagocytes, treating to expose DNA of the foreign microorganism in the phagocytes, *in situ* hybridizing under a stringent condition between the DNA and a DNA probe which can detect hybridization; and evaluating the phagocytotic function by the resulting signal, wherein said kit has;

(1) the foreign microorganism,
(2) at least one or more enzyme(s) selected from the group consisting of lysostafin, lysozyme, N-acetylmuramidase and zymolase used in said exposing step of the DNA, and
(3) one or more DNA probe(s) for detection.

[Brief Description of Drawings]

Fig. 1 is a view illustrating results of *in situ* hybridization carried out (a) without the surfactant (SDS) and (b) with the surfactant (SDS).

Fig. 2 is a view illustrating the states obtained upon fixing with various leukocyte cell densities.

Fig. 3 is a view illustrating the activity of lytic enzyme on (a) *Staphylococcus aureus* and *Staphylococcus epidermidis,* (b) *Pseudomonas aeruginosa* and *Escherichia coli*, and (c) *Enterococcus faecalis* in a time dependent manner.

Fig. 4 is a view illustrating concentration dependent effects by the addition of DMSO on a lytic activity of (a) 300 unit/ml N-acetylmuramidase, (b) 10,000 unit/ml lysozyme, and (c) 50 unit/ml lysostafin.

Fig. 5 is a drawing illustrating effects of the addition of PMSF used for suppressing the action of protease which effects deterioration of morphology of leukocytes in respect of (a) 0.2 unit/ml protease alone, (b) addition of 1 μmol/ml PMSF, (c) addition of 10 μmol/ml PMSF, (d) addition of 0.1 mmol/ml PMSF, and (e) addition of 1 mmol/ml PMSF.

Fig. 6 is a view illustrating the occurrence of the alteration of morphology of phagocyte upon phagocytosis of bacteria, in the digested sample prepared according to the present invention.

Fig. 7 is a view illustrating the effects of the enzymatic treatment on digested samples, showing states of: (a) digested sample of S. aureus prior to the enzymatic treatment, (b) digested sample of E. faecalis prior to the enzymatic treatment, (c) sample (a) following the enzymatic treatment, and (d) sample (b) following the enzymatic treatment.

Fig. 8 is a diagrammatic view illustrating the slide glass for smear of digested samples used in the study of the optimal concentration of the probe upon *in situ* hybridization.

Fig. 9 is a diagrammatic view illustrating the slide glass for smear of digested samples used in the study of the optimal temperature upon *in situ* hybridization.

Fig. 10 is a view of Southern blotting (upper panel) and electrophoresis (lower panel) illustrating the chain length of the probe for detection obtained by digoxigenin labelling of (a) SA probe and (b) PA probe, and signal intensities by labelling.

Fig. 11 is a view illustrating results of signal detection observed when (a) EC-24, (b) EC-34, (c) EC-39, and (d) mixed probe of probes (a) to (c) as the probe for detection upon *in situ* hybridization for E. coli digested sample.

Fig. 12 is a diagrammatic view illustrating a slide glass for smear of digested samples.

Fig. 13 is a view illustrating results of signal detection observed when *in situ* hybridization was carried out using corresponding probe for the detection to each of digested samples of (a) SA, (b) SE, (c) PA, (d) EF and (e) EK.

Fig. 14 is a view illustrating states in which the probe for detecting SA specifically presents signals for the SA digested sample.

[Best Mode for Carrying Out Invention]

According to one embodiment of the present invention, an experimental model (hereinafter, referred to as digested sample ) is provided characterized in that: phagocytes are brought into contact *in vitro* with a foreign microorganism prepared such that turbidity of the bacterial liquid (O.D. = 600 nm) is preferably about 0.01 to about 0.03; phagocytes post phagocytosis of a foreign microorganism are prepared by isolating the cells; and adjusting thus resulting phagocytes post phagocytosis of a foreign microorganism to give about $1 \times 10^4$ cells/μl to about $5 \times 10^4$ cells/μl.
The term phagocyte post phagocytosis of a foreign microorganism used herein means a cell attached to a foreign microorganism or a cell including a foreign microorganism involving not alone cells after completing phagocytosis of a foreign microorganism already, but cells during phagocytosis and cells which are ready to the initiate phagocytosis after the adhesion of a foreign microorganism on the cell surface.

The phagocyte referred to herein is not particularly limited as long as it is a cell capable of incorporating a foreign substance as well as a foreign microorganism within the cell of its own, and examples thereof include macrophage, monocyte, neutrophil, eosinophil and the like. In addition, a phagocyte system such as U937 cells, HL60 cells or the like may be also used.

Because phagocytes derived from a living body are also included in body fluids such as e.g., blood, tissue fluids, lymph fluid, cerebrospinal fluid, pyo, mucus, snot, sputum, urine, ascites and the like, or dialysis drainage, or otherwise lavage obtained after washing nasal cavity, bronchus, skin, any of various organs, bone or the like, phagocytes can be also prepared from these sources. In addition, phagocytes can be prepared also from a tissue such as skin, lung, kidney, mucosa or the like. Macrophage which is one of phagocytes transforms into a variety of morphology such as monocyte, pulmonary alveolus macrophage, peritoneal cavity macrophage, fixed macrophage, free macrophage, Hansemann macrophage, inflammatory macrophage, hepatic Kupffer cell, cerebral microglia cell and the like, therefore, any tissue including these may be used also as a source of the phagocyte in addition to blood. For preparing phagocytes from a tissue, for example, cells are detached by using an enzyme such as trypsin after collecting the tissue to isolate phagocytes which are present in the tissue.

In order to obtain a phagocyte (leukocyte) fraction from a body fluid or the like, any known method can be used. For example, about 5 ml of heparinized venous blood (10 ml when number of leukocytes is small) is collected, followed by mixing of this blood with a reagent for separating blood (225 mg of sodium chloride, 1.5 g of dextran (MW: 200,000-300,000), adjusted to give the total volume of 25 ml with sterile purified water) at a ratio of 4:1 and leaving to stand still at about 10°C to about 40°C for about 15 minutes to about 120 minutes, preferably at 37°C for about 30 minutes. Accordingly, a leukocyte fraction (upper layer) can be obtained.

Leukocytes can be obtained by centrifugation of the resultant leukocyte fraction at 0°C to about 20°C for about 3 minutes to about 60 minutes at about 100 to about 500×g, preferably, at 4°C for 10 minutes at about 140 to about 180 ×g. When erythrocytes are contaminated upon this operation, it is preferred that a hemolysis operation is conducted. For example, 1 ml of sterile purified water may be added to a pellet of leukocytes and suspended, and immediately thereafter an excess amount of PBS (18.24 g of sodium chloride, 6.012 g of sodium monohydrogen phosphate 12 hydrate, 1.123 g of sodium dihydrogen phosphate dihydrate, adjusted to give the total volume of 120 ml with sterile purified water (PBS stock solution; hereinafter referred to simply as " PBSstock solution ) diluted to 20 fold with sterile purified water; hereinafter referred to simply as " PBS) may be added thereto to result in isotonization, followed by centrifugation once again at 4° C for 10 minutes at about 140 to about 180×g.

The foreign microorganism which may result in an infectious disease is not particularly limited as long as it is a microorganism which is subjected to phagocytosis by a phagocyte, and eaxmples thereof include bacteria, fungi, viruses, protozoan, parasites and the like. Examples of bacteria include e.g., staphylococci, *Pseudomonas aeruginosa,* enterococci, coli bacteria, Streptococci, pneumococci, tubercle bacilli, helicobacter pylori, listeria, yersinia, brucella and the like. Examples of fungi include e.g., *candida, aspergillus, actinomyces, coccidioldes, blastomyces* and the like. Examples of viruses include e.g., influenza virus, polio virus, herpes virus, hepatitis virus, AIDS virus and the like. Examples of protozoan include e.g., amebic dysentery, Trichomonas vaginalis, malaria, toxoplasma and the like. Examples of parasite include trypanosome and the like. In particular, examples of causative microorganism of sepsis or bacteremia include e.g., staphylococci (*Staphylococcus aureus, Staphylococcus epidermidis*), enterococci (*Enterococcus faecalis, Enterococcus faecium, Streptococcus pneumoniae, Streptococcus pyogenes, Streptociccus agalactiae*) which are Gram positive bacteria; coli bacteria (*Escherichia coli*), enterobacter (*Enterobacter cloacae*), *Escherichia coli* analogous enteric bacteria (*Klebsiella oxytoca, Serratia marcesens, Proteus vulgaris, Citrobacter freundii*) such as klebsiella (*Klebsiella pneumoniae*) which are Gram negative bacteria; pseudomonas *(Pseudomonas aeruginosa*) which are an aerobic bacilli; clostridium (*Clostridium perfringens*), bacteroides (*Bacteroides fragilis*) which are anaerobe and the like. On rare occasions, Acinetobacter calcoaceticus, Aeromonas hydrophilia, Flavobacterium meningosepticum, Bacillus cereus or the like may serve as the cause. Among these, in particular, Gram negative bacteria, or one or more microorganism selected from the group consisting of *Staphylococcus aureus, Staphylococcus epidermidis, Enterococcus faecalis, Pseudomonas aeruginosa, Escherichia coli* and *Candida albicans,* and mixtures thereof are suitably used.

For allowing phagocytosis of a foreign microorganism by phagocytes, the foreign microorganism is precultured to give a certain amount previously. After suspending in PBS thus collected microorganisms following proliferation, they are diluted in PBS to adjust the turbidity of the bacterial liquid (O.D.=600 nm) to about 0.001 to about 0.1, preferably about 0.01 to about 0.03 measured by an absorption meter. Thus produced bacterial liquid is transferred to separate flasks for culture, and left to stand still at room temperature for about 30 minutes. Heparinized healthy human blood is collected, and the aforementioned reagent for separating hemocyte is added thereto at a ratio of approximately 4:1, followed by leaving to stand still at about 20°C to about 40°C, preferably at 37°C for about 30 minutes to yield a leukocyte fraction. Thus obtained leukocyte fraction is suspended in PBS. The supernatant in the flask for culture which had been charged with the foreign microorganism is gently discarded, and the leukocyte fraction diluted in PBS is added to the flask followed by leaving to stand still at room temperature for about 10 minutes. The supernatant in the flask for culture is discarded, and leukocytes adhered on the bottom of the flask are recovered in a centrifuge tube using PBS containing 0.02% EDTA, and are collected by e.g., centrifugation at 4°C for 10 minutes at about 140 to about 180 × g. When contamination of erythrocytes is found in thus collected leukocytes, leukocytes may be collected by: allowing hemolysis through gently suspending the precipitates of leukocytes in sterile purified water, isotonization through adding PBS, followed by centrifugation once again at 4°C for 10 minutes at about 140 to about 180 g. The collected leukocytes are suspended in PBS, and cell number is counted with a counting chamber to adjust to give about $1\times10^4$ cells/pl to about $5\times10^4$ cells/μl.

Process for fixing leukocytes may involve for example, carrying out Carnoy fixation.

Specifically, leukocytes are supported on a carrier capable of supporting leukocytes (supporting carrier), immersed in Carnoy' s fixative(a mixed solution at a volume ratio of ethanol : chloroform : acetic acid = 6 : 3 : 1) for about 20 minutes, thereafter immersed in about 50% to about 90%, preferably about 75% ethanol solution for about 5 minutes, and then completely air dried.

The supporting carrier described above is preferably any of those made from an insoluble material, and for example, glass, metal, synthetic resins (polystyrene, polyethylene, polypropylene, polyvinyl chloride, polyester, polyacrylic ester, nylon, polyacetal, fluorine resin and the like), polysaccharides (cellulose, agarose and the like) are preferred.

The insoluble supporting carrier may be in any of various forms such as, for example, plate-like, tray-like, spherical, fibrous, cylindrical, discal, vessel-like, cell-like, tubular and the like. In particular, preferable supporting carrier for use in one embodiment of the present invention is a slide glass. Examples of the slide glass include e.g., a slide glass (item number: MS311BL) manufactured by JAPAN AR BROWN CO., LTD. This slide glass (item number: MS311BL) is provided with 14 circular wells having the diameter of 5 mm addition, upon practical use, it is preferred that an APS coated slide glass is used which is a slide glass with 3-aminopropyltriethoxysilane (APS, SIGMA) coated thereon for the purpose of improving adhesiveness of cells. Alternatively, a slide glass with poly-L-lysine or gelatin coated thereon may be also used.

For producing an APS coated slide glass, a slide glass (item number: MS311BL) is first fixed on a slide holder, and thereafter is washed by immersing in a diluted neutral detergent for 30 minutes, and the detergent is sufficiently removed with running water. Next, the slide glass is washed with purified water and dried sufficiently at high temperature (100°C or greater) followed by leaving to stand to cool at room temperature. Then, the slide glass is immersed in acetone containing 2% APS for 1 minute, and immediately thereafter washed briefly with acetone and sterile purified water sequentially followed by air drying. Further, after conducting the operation of immersing the slide glass in acetone containing about 1 to about 10% APS for 1 minute, followed by immediate and brief washes with acetone and sterile purified water in a sequential manner and air drying once again, the APS coated slide glass can be produced by drying at about 20°C to about 60°C, preferably at 42°C.

When the leukocytes are supported on the APS coated slide glass, it is preferred that leukocytes are smeared on each well such that they are spread over to give a single layer and air dried. It is preferred that phagocytes for use in fixing are prepared such that the density (x cells/ml) is about $5\times10^6$ cells/ml < x cells/ml < about $1\times10^8$ cells/ml; and preferably about $1\times10^7$ cells/ml x cells/ml about $5\times10^7$ cells/ml.

Moreover, corresponding to such alteration of density of the phagocytes per 1 ml, cell number of the leukocytes fixed on the APS coated slide glass per 1 well (y cells/well (diameter: 5 mm) is preferably adjusted to be about $2.5\times10^4$ cells/well < y cells/well < about $5\times10^5$ cells/well, and preferably about $5\times10^4$ cells/well y cells/well about $2.5\times10^5$ cells/well. Specifically, a small amount of PBS is added to a leukocyte pellet obtained by centrifugation of the leukocyte fraction at 4°C for about 10 minutes at about 140×g to about 180×g followed by suspension, and the cell number of the leukocytes is counted using a counting chamber. Preparation can be perfected by smearing 5 μl of the leukocyte suspension, which was prepared with PBS such that cell number becomes about $5\times10^4$ cells/well to about $2.5\times10^5$ cells/well, on each well of the APS coated slide glass to allow the leukocytes spread to form a single layer followed by complete air drying.

As a treatment for promoting permeability of the membranes of phagocytes, a process may be employed in which immersion is conducted in PBS for about 3 to about 30 minutes, followed by immersing in a solution of an enzyme pretreatment reagent (prepared by mixing 1.25 g of saponin, 1.25 ml of t-octylphenoxypolyethoxyethanol (specific gravity: 1.068 to 1.075 (20/4°C), pH (5 w/v%) 5.5-7.5) and 25 ml of a PBS stock solution, and adjusting to give the total volume of 50 ml with sterile purified water) diluted to about 2 to about 50 fold in sterile purified water, and allowing infiltration on a shaker for about 3 to about 30 minutes.

In a treatment for exposing the DNA of the causative microorganism of an infectious disease in the phagocytes, an enzyme reagent solution is prepared by adding 1 ml of an enzyme reagent dissolving solution (prepared by about 100 fold dilution of dimethylsulfoxide (DMSO) which contains phenylmethylsulfonylfluoride (PMSF) in PBS) to an enzyme reagent (N-acetylmyramidase, lysozyme and/or lysostafin) per 1 slide, and thereafter, 1 ml of this enzyme solution is dropped on a site of the leukocyte smear, and left to stand still for about 10 to about 60 minutes in a humid box at about 20°C to about 60°C, preferably at about 37° C to about 42°C. Then, it is immersed in PBS containing 0.2 mol/l hydrochloric acid (prepared by adding hydrochloric acid to the PBS stock solution, 20 fold dilution in sterile purified water, and adjusting to give the final concentration of hydrochloric acid of 0.2 mol/l) and thus the object is achieved by allowing infiltration on a shaker for 3 to 30 minutes as it is. Since DMSO has the potential of lowering the activity of lysozyme and lysostafin at the concentration of 5% or greater, it is preferably used at the concentration of less than 5%. Except for PMSF as a substance for retaining the morphology of the phagocytes, other known protease inhibitor, e.g., tosyl lysine chloromethyl ketone (TLCK) and a mixture thereof may be also used. In such a case, a solvent such as DMSO may be changed ad libitum.

In regard of preferable range of the titer of each enzyme used as an enzyme reagent, although lysostafin exerts a sufficient effect at a titer of 1 unit/ml upon lysis of *Staphylococcus aureus,* lysostafin having the titer of 10 unit/ml or greater was required for lysis of *Staphylococcus epidexmidis*. Therefore, optimal titer of lysostafin may be set to 1 unit/ml to about 1,000 unit/ml, and preferably about 10 unit/ml to about 100 unit/ml. Further, upon lysis of *Enterococcus faecalis*, lysis did not occur when the titer of N-acetylmuramidase is about 10 unit/ml or less, while the titer of lysozyme was fixed to be about 10,000 unit/ml. In respect of lysozyme, when the titer of N-acetylmuramidase was fixed to be 100 unit/ml, lysis did not occur with the titer of lysozyme of 1,000 unit/ml or less. Therefore, optimal titer of N-acetylmuramidase may be set to be about 10 unit/ml to about 10,000 unit/ml, and preferably about 100 unit/ml to about 1,000 unit/ml, whilst the optimal titer of lysozyme may be set to be about 1,000 unit/ml to about 1,000,000 unit/ml, and preferably about 10,000 unit/ml to about 100,000 unit/ml. Furthermore, in instances where the causative microorganism is a fungus such as *Candida albicans*, the range of titer may be about 50 unit/ml to about 500 unit/ml, preferably about 100 unit/ml to about 500 unit/ml of zymolase. Additionally, when zymolase is used in particular, it is preferred that PMSF or known protease inhibitor is used.

Moreover, depending on the difference of components in Gram positive bacteria and Gram negative bacteria, in other words, on the difference in peptidoglycan or lipopolysaccharide, the enzyme to be used may be optionally selected. Particularly, irrespective of whether Gram positive bacterium or Gram negative bacterium is, two or more enzymes may be used in combination for the purpose of achieving lysis more effectively. According to the present invention, it was revealed that by using a mixture of three kinds, lysozyme, lysostafin and N-acetylmuramidase, lytic activity was elevated in comparison with the case where a single enzyme was used.

Temperature of the enzymatic treatment may be preferably about 4°C to about 60°C for *Staphylococcus aureus*; higher than about 25° C, preferably about 37° C or higher for *Staphylococcus epidermidis*; and higher than about 25° C and less than about 60° C, preferably about 37° C to about 42° C for *Enterococcus faecalis*. Accordingly, it is most preferred that optimal temperature for the enzymatic treatment is set to be about 37° C to about 42° C. Additionally, critical temperature is expected to be about 26° C to about 59° C in the common range for those three kinds of the bacteria.

Further, time period of the enzymatic treatment may be 20 minutes or longer for any of digested samples of *Staphylococcus aureus, Staphylococcus epidermidis* and *Enterococcus faecalis* (inadequate in 0 minute and 10 minutes), and because no bacterial body was found within the leukocytes, the time period is preferably at least about 15 minutes or longer, preferably about 20 minutes or longer, and in addition, optimal time period of the enzymatic treatment shall be about 30 minutes to about 60 minutes. Moreover, the time period of the enzymatic treatment may be about 15 minutes to about 120 minutes.

N-acetylmuramidase is an enzyme which lowers the absorbance at 600 nm when thermally treated dry powder of *Enterococcus faecalis* by a heat treatment and N-acetylmuramidase are subjected to a reaction in a 5 mmol/l Tris-HCl buffer (pH 6.0) containing 2 mmol/l magnesium chloride at 37° C for 5 minutes. Additionally, when the enzymatic activity at 37°C, pH 7.0 in 1 minute to lyse 1 ug of cells of *Streptococcus salivarius* (IFO 3350), which was subjected to a heat treatment, is determined as 1 unit, it is preferred that one having the enzymatic activity of 2,000 unit/mg or greater is used.

Lysozyme is an enzyme which lowers the absorbance at 600 nm when *Micrococcus luteus* and lysozyme were subjected to a reaction in PBS at 37°C for 5 minutes. Furthermore, when the enzymatic activity to lower the absorbance at 540 nm of *Micrococcus luteus* by 0.001 at 35°C, pH 6.2 in 1 minute is determined as 1 unit, it is preferred that one having the enzymatic activity of 50,000 unit/mg or greater is used.

Lysostafin is an enzyme which lowers the absorbance at 600 nm when *Staphylococcus epidermidis* and lysostafin are subjected to a reaction in PBS at 37° C for 5 minutes. Furthermore, when the enzymatic activity to lower the absorbance at 620 nm of *Staphylococcus aureus* from 0.240 to 0.125 at 37°C, pH 7.5 in 10 minutes is determined as 1 unit, it is preferred that one having the enzymatic activity of 500 unit/mg or greater is used.

Zymolase (trade name: Zymolyase, Seikagaku Corporation) is an enzyme prepared from a culture liquid of *Arthrobacter lutesul,* having a potent lytic activity against cell walls of yeast living cells. Essential enzyme involving in lysis of cell walls included in zymolase is -1,3-glucan lanimaripentaohydrolase, which acts on a glucose polymer having -1,3-bonds to produce lanimaripentaose as a major product. Zymolyase-100T, which is purified by ammonium sulfate fractionation, and further purified by affinity chromatography (Kitamura, K. et al.; J. Ferment. Technol., 60, 257, 1982), has the activity of 100,000 unit/g. However, the activity of this enzyme is known to be altered depending on the type of yeast to be a substrate, culture condition and growing stage (Kitamura, K. et al.; J. Gen. Appl. Microbiol., 20, 323, 1974, Kitamura, K. et al.; Agric. Biol. Chem., 45, 1761, 1981, Kitamura, K. et al.; Agric. Biol. Chem., 46, 553, 1982). Zymolyase-100T includes about $1.0\times10^7$ unit/g of -1,3-glucanase, about 1.7 $\times10^4$ unit/g of protease, and about $6.0\times10^4$ unit/g mannase, however, DNase and RNase are not found therein (Kitamura, K. et al.; J. Gen. Appl. Microbiol., 18, 57, 1972). In addition, the optimal pH of Zymolyase is about 5.5 to about 8.5, and preferably about 6.5 to about 7.5, whilst the optimal temperature is about 25°C to about 55° C, and preferably about 35° C to about 45°C. Moreover, lysis spectrum (genus name) against yeast (cells in logarithmic growth phase) includes *Ashbya, Candida, Debaryomyces, Eremothecium, Endomyces, Hansenula, Hanseniaspora, Kloekera, Kluyveromyces, Lipomyces, Helschkowia, Pichia, Pullularia, Torulopsis, Saccharomyces, Saccharomycopsis, Saccharomycodes, Schwanniomyces* and the like.

In particular, examples of those in genus candida include *Candida albicans, Candida tropicalis, Candida parasilosis, Candida galacta, Candida guilliermondii, Candida krusei, Cryptococcus neoformans* and the like. As an activator of this enzyme, an SH compound, e.g., cysteine, 2-mercaptoethanol, dithiothreitol and the like can be used.

Fungi belonging to these genera may be also used in the present invention. According to this enzyme, an enzymatic activity required for decreasing about 30% of $A_{800}$ of a reaction liquid (enzyme: 1 ml of a 0.05 to 0.1 mg/ml solution, substrate: 3 ml of a beer yeast suspension (2 mg dry weight/ml), buffer: 5 ml of M/15 phosphate buffer (pH 7.5), adjusted to give the total volume of 10 ml with 1 ml of sterile purified water) using the beer yeast suspension as a substrate at about 25°C within two hours is determined as 1 unit. Zymolyase-100T has the activity of 100,000 unit/g.

It is preferred that the concentration of PMSF (added in order to protect the leukocytes from protease so that the morphology thereof is retained) which is used as a solvent for the enzyme reagent is in the range of 10 μmol/l to 10 mmol/l, and preferably 0.1 mmol/l to 1 mmol/l, because effects were observed at the concentration of 10 μmol/l or greater, while deterioration of morphology of leukocytes was completely suppressed at the concentration of 0.1 mmol/ l or greater. In addition, it is preferred that the concentration of DMSO is less than 5%, preferably 2% or less, and further approximately the concentration of 1%. As a consequence, the enzyme reagent dissolving solution is preferably prepared by 100 to 1,000 fold dilution of dimethylsulfoxide (DMSO) which contains 0.1 mol/l phenylmethylsulfonylfluoride (PMSF) in PBS.

Following the step of exposing the DNA of the causative microorganism of an infectious disease, the step of acetylation of cell membrane proteins may be inserted. Specifically, it can be carried out through immersing the slide glass in an acetylation reagent, which was prepared by adding acetic anhydride to an acetylating reagent (7.46 g of triethanolamine, an appropriate amount of hydrochloric acid, adjusted to give the total volume of 50 ml with an appropriate amount of sterile purified water) and diluting about 2 fold to about 50 fold, preferably about 10 fold in sterile purified water to give the final concentration of acetic anhydride of 0.1 to 3.0%, preferably 0.8%, followed by shaking for 5 to 30 minutes on a shaker. Thereafter, the slide glass is sequentially immersed in 75%, 85%, and 98% ethanol for 2 to 5 minutes respectively, and completely air dried.

Additionally, following the step of acetylation of cell membrane proteins, the step of forming a single stranded DNA by an alkali treatment of the DNA of the causative microorganism of an infectious disease can be also inserted. Specifically, it can be carried out through immersing the slide glass in PBS which contains about 10 mmol/l to about 300 mmol/l, preferably about 70 mmol/l sodium hydroxide (prepared by adding sodium hydroxide in the PBS stock solution, diluting to 20 fold with sterile purified water to give the final concentration of sodium hydroxide of 70 mmol/l) for about 2 to about 5 minutes. Thereafter, the slide glass is sequentially immersed in 75%, 85%, and 98% ethanol for 2 to 5 minutes respectively, and completely air dried.

Upon carrying out in situ hybridization using a DNA probe for detection capable of hybridizing with the exposed DNA of the causative microorganism of an infectious disease under a stringent condition, for example, a liquid containing the DNA probe for detection prepared in a probe dilution solution (probe solution) is coated on the smeared site, and is left to stand still in a humid box at about 25°C to about 50°C, preferably at about 37°C to about 42°C for about 1 to about 3 hours, preferably for about 2 hours.

Thereafter, a hybridization washing solution (prepared by mixing a hybridization stock solution (13.15 g of sodium chloride, 6.615 g of trisodium citrate dihydrate, adjusted to give the total volume of 75 ml with sterile purified water: hereinafter, referred to as merely hybridization stock solution ) in a ratio of the hybridization stock solution : sterile purified water : formamide = 5 : 45 : 50) is provided in three staining bottles, and sequentially, the sample is immersed at about 35 to about 45°C, preferably at about 42°C for 10 minutes, respectively. Then, the sample is immersed in PBS, and shaken as it is on a shaker for about 5 to about 30 minutes. In detail, the probe dilution solution includes 600 μl of salmon sperm DNA, 50 μl of 100 × Denhardt' s solution 500 pl of hybridization stock solution, 2,250 μl of formamide, 1,000 μl of 50% dextran sulfate. The probe solution preferably includes 15 ng of each DNA probe for detection, which may be adjusted to give the total volume of 50 μl with the probe dilution solution.

Concentration of the probe for SA, SE, PA, EF and EK may be about 0.6 ng/μl to about 1.8 ng/μl, preferably about 0.6 ng/μl to about 1.2 ng/μl. Further, the result of inadequate was brought at 0.06 ng/μl, and the result of adequate was brought at 0.6 ng/μl, therefore, it is preferred that the concentration is set to be at least 0.1 ng/μl or greater. Moreover, because the result of inadequate was brought at 2.4 ng/μl, and the result of adequate was brought at 1.8 ng/μl, it is preferred that the concentration is set to be 2.2 ng/μl or less. In addition, the optimal concentrations of positive control and negative control may be 0.4 to 2.0 ng/μl and 0.6 to 2.0 ng/μl respectively, and preferably 0.6 to 1.0 ng/μl in common.

Further, it is preferred that time period of the hybridization is at least 30 minutes or longer, preferably 60 minutes or longer, and more preferably 90 minutes or longer. More preferred optimal time period of the hybridization may be set to be about 120 minutes to about 900 minutes.

Moreover, to use a surfactant such as sodium dodecyl sulfate (SDS) in the step of *in situ* hybridization is preferred in light of the capability to improve the detection sensitivity. It is preferred that concentration of SDS is 1% or less, more preferably about 0.1% to about 0.5%, still more preferably about 0.25%. SDS may be added to a solution used upon the hybridization, or may be in the probe dilution solution or in the probe solution, which was mixed beforehand.

Additionally, it is preferred that one or more DNA probe having the chain length of about 350 to about 600 base length, preferably about 350 to about 550 base length is employed as the DNA probe for detection, because the probe is efficiently introduced into phagocytes, and firm contact with the gene of the incorporated foreign microorganism is permitted. It is not intended that base length (number of base pairs) of the subject probe must necessarily fall within the aforementioned range of the base length, but that it is allowable as long as the base length in the aforementioned range is included in the distribution of the base length of the probe. These probes may be used alone, or several kinds of probes (more than one) may be also used. More than one probes may be multiple kinds of probes which can hybridize to one bacterial strain. Alternatively, the kind of the probe may be multiple owing to the presence of multiple types of the bacterial strains although a single probe may be employed for a single bacterial strain. Thus, there is no particulate limitation as far as the kind of the probe is one or more.

These probes preferably comprise a DNA fragment having a sequence which does not any how hybridize with the phagocyte itself, and additionally, they should not cross hybridize with a gene derived from any other strain of microorganism. For example, when a subtraction method is used, a specific probe can be produced in a short period of time. These probes may be prepared and labelled according to a common nick translation process using a non-radioisotopic labelling substance such as fluorescein isothiocyanate (FITC), biotin, digoxigenin (digoxigenin (DIG)-11-dUTP) or the like. Chain length of the probe can be controlled such that most efficient labelling is enabled, by changing the ratio of amount of DNase I and DNA polymerase I added in the nick translation reaction. For example, for efficiently labelling 2 μg of the DNA probe (SA-24), and for regulating the chain length of a probe to enable efficient *in situ* hybridization with the DNA of a foreign microorganism (base length of about 350 to about 600), when 2 μl of 10 U/μl DNA polymerase I is included in the reaction liquid of total volume of 100 μl, 6 μl of DNase I may be included which was prepared such that about 10 to about 350 mU, preferably about 25 to about 200 mU, more preferably about 50 to about 150 mU is present in total volume of 100 pl. Volume of each enzyme and total volume of the reaction liquid and the like in this instance may optionally vary as long as the proportion according to the aforementioned essential condition for an optimal reaction is kept constant. Further, in other words, when 20U of DNA polymerase I is included in total volume of 100 μl, DNase I may be prepared in an amount of about 10 to about 350 mU, preferably about 25 to about 200 mU, and more preferably about 50 to about 150 mU. In additional other words, when 1 U of DNA polymerase is included, nick translation may be conducted using about 0.5/1,000 to about 17.5/1,000, preferably about 1.25/1,000 to about 10/1,000, and more preferably about 2.5/1,000 to about 7.5/1,000 unit of DNase I. In addition, with DNA in an amount of 1 μg, it is desirable to prepare such that DNA polymerase I is present in an amount of about 10 U, while DNase I is present in an amount of about 5 to about 175 mU, preferably about 12.5 to about 100 mU, and more preferably about 25 to about 75 mU. In respect of other probe, the amount of DNA as well as optimal conditions for the reaction of DNA polymerase I and DNase I can be determined with reference to the optimal conditions for the reaction as described above, and chain length of the probe (base length of about 350 to about 600) can be regulated to result in efficient labelling and efficient in situ hybridization with a foreign microorganism DNA.

The stringent condition for carrying out *in situ* hybridization may be for example, a condition which comprises incubating in the presence of about 30% to about 60%, preferably about 50% of formamide, at about 30 to about 50°C, preferably at about 38 to about 42°C followed by washing.

After carrying out *in situ* hybridization, an operation of blocking may be performed. Specifically, 1 ml of a blocking reagent (2 ml of normal rabbit serum, 0.5 ml of the PBS stock solution, adjusted to give the total amount of 10 ml with sterile purified water) is dropped on the smear site per one slide glass in a humid box, and left to stand still for 15 to 60 minutes. Thereafter, the blocking reagent is removed.

For detecting a signal which results from the hybridization with a gene derived from the microorganism (genomic DNA or RNA), color reaction may be conducted in which any conventional method for an antigen-antibody reaction is utilized. In other words, after enough washes of the sample following completing the hybridization, an operation for blocking is conducted. Thereafter, a treatment is executed using a conjugate of an anti-FITC antibody, anti-digoxigenin antibody or the like, e.g., an alkaline phosphatase conjugate, and then, color developmet of the signal is allowed by a color development system of the conjugate to determine the states of hybridization. For example, in instances where the probe labelled with digoxigenin-11-dUTP as described above is used as a probe, an anti-digoxigenin-alkaline phosphatase conjugate is used, and the detection may be conducted through utilizing a substrate which is generally used for alkaline phosphatase (nitroblue tetrazolium, 5-bromo-4-chloro-3-indolyl phosphate and the like). Next, the smear preparation washed after the color reaction is subjected to counter staining with naphthol black Fast Green (20 mg/50ml, manufactured by Wako Chemical Co. ) or the like to observe intracellular signals with a light microscope.

In detail, in order to obtain s signal by hybridization, for example, when a digoxigenin labelled DNA probe is used as a DNA probe for detection, a labelled antibody solution is prepared by diluting a labelled antibody (1.05 unit of alkaline phosphatase labelled anti-digoxigenin antibody solution, adjusted with 12.6 μl of buffer A (746 mg of triethanolamine, 17.5 mg of sodium chloride, 20.3 mg of magnesium chloride hexahydrate, 1.36 mg of zinc chloride, 1,000 mg of bovine serum albumine, an appropriate amount of hydrochloric acid, adjusted to give the total volume of 100 ml with sterile purified water) to give the total volume of 14 μl) in a labelled antibody diluent (8.48 mg of Tris-(hydroxymethyl)-aminomethane, 6.14 mg of sodium chloride, an appropriate amount of hydrochloric acid, adjusted to give the total volume of 0.7 ml with sterile purified water) to 10 to 200 fold, preferably 50 fold, and each 10 μl of this labelled antibody solution is dropped on the smear site, followed by leaving to stand still for 15 to 60 minutes. Thereafter, it is immersed in a solution of a labelled antibody washing solution (1 ml of polysorbate 20, 50 ml of the PBS stock solution, adjusted to give the total volume of 100 ml with sterile purified water) diluted to 2 to 50 fold, preferably 10 fold, and is allowed for infiltration on a shaker for about 5 to about 30 minutes as it is. After repeating this operation twice, it may be immersed in a coloring pretreatment liquid obtained by mixing a coloring pretreatment liquid 1 (6.06 g of Tris-(hydroxymethyl)-aminomethane, 2.92 g of sodium chloride, an appropriate amount of hydrochloric acid, adjusted to give the total volume of 50 ml with sterile purified water) and a coloring pretreatment liquid 2 (5.08 g of magnesium chloride hexahydrate, adjusted to give the total volume of 50 ml with sterile purified water) in an equivalent volume and diluting to approximately 5 fold in sterile purified water, and then shaken for 5 to 30 minutes on a shaker as it is. Thereafter, 1 ml of a coloring reagent (nitroblue tetrazolium (NBT)/ 5-bromo-4-chloro-3-indolylphosphate (BCIP)) per one slide glass is dropped on the smear site of the slide glass while filtration using a disposable syringe equipped with a 0.2 μm syringe top filter, and is left to stand still under light shielding in a humid box at about 10°C to about 45°C, preferably at about 37°C for about 15 to about 60 minutes. Thereafter, it is immersed in a solution of a coloring reagent washing solution (606 mg of Tris-(hydroxymethyl)-aminomethane, 186 mg of ethylenediamine tetraacetate disodium dihydrate, an appropriate amount of hydrochloric acid, adjusted to give the total volume of 50 ml with an appropriate amount of sterile purified water) diluted to about 2 to about 50 fold, preferably about 10 fold for about 2 to about 10 minutes, and is air dried. Then, it is immersed in a solution of a counter staining solution (50 mg of fast green FCF (edible dye, green color No. 3), adjusted to give the total volume of 50 ml with an appropriate amount of sterile purified water) diluted to 2 to 50 fold, preferably to 10 fold and then an acetic acid solution of about 0.1 to about 5%, preferably about 1%. Thereafter, the excess counter staining solution may be washed away by immersing again in a solution of the coloring reagent washing solution described above diluted to about 2 to about 50 fold, preferably about 10 fold, and may be completely air dried. Additionally, the coloring reagent described above may be one prepared separately.

The alkaline phosphatase labelled anti-digoxigenin antibody solution which may be preferably used is one which results in color development in a site of DNA blotting when 1 ng of a digoxigenin labelled DNA is blotted on a membrane for blotting, subjected to blotting, treated with the alkaline phosphatase labelled anti-digoxigenin antibody solution diluted to 10,000 fold, and allowed to react with a coloring substrate (NBT/BCIP), but one which does not result in color development even though similar operation is conducted with a DNA without digoxigenin labelling. Further, the anti-digoxigenin antibody is preferably derived from sheep. In detail, it may be purified from serum of an immunized sheep by an ion exchanging chromatography and an antibody column chromatography.

The coloring reagent (NBT/BCIP solution, pH 9.0 to 10.0) preferably contains 3.3 mg of nitroblue tetrazolium (NBT), 1.65 mg of 5-bromo-4-chloro-3-indolylphosphate (BCIP), 99 μg of N,N-dimethylformamide, 121 mg of Tris-(hydroxymethyl)-aminomethane, an appropriate amount of hydrochloric acid, 58.4 mg of sodium chloride, 101.6 mg of magnesium chloride hexahydrate, and is adjusted to give the total amount of 10 ml with an appropriate amount of sterile purified water. The coloring reagent which may be preferably used is one which exhibits a dark purple signal on the blotted site when a protein labeled with alkaline phosphatase is blotted on a membrane for blotting followed by a treatment of the membrane with the coloring reagent at room temperature under light shielding.

Upon counter staining as described above, an edible dye e.g., yellow No. 4 (tartrazine) can be used for the purpose of further clarification of the contrast between the signal and the cell. The grounds therefor may be difficulties in the counter staining on behalf of the similar color among the purple color developed by the substrate and the blue color developed by naphthol black. When this process was applied to the present invention, it was revealed that the process is beneficial upon the counter staining. The procedure involving in use of an edible dye has not been proposed heretofore.

The process which may be employed for labelling digoxigenin can be a nick translation method. In addition, a PCR method, a random primer labelling method, an *in vitro* transcription labelling method, a terminal transferase labelling method or the like can be employed.

Determination may be carried out by microscopic examination with a light microscope (× 1,000), and observation of at least one color development of bluish purple color may be determined as positive in cells within a single well stained with the counter staining solution as described above.

Moreover, in connection with the process of the production of the probe for detection, reference may be made to Japanese Patent Nos. 2558420, 2798499, 2965543, 2965544, 3026789 and so on.

For example, for the culture through picking a microorganism from a working cell bank, the working cell bank (SA-24) is smeared by streaking with a platinum loop, a disposable plastic loop or the like on an L-broth solid medium containing 50 μg/ml ampicillin prepared in a sterile petri dish (microorganism picking).

Following overnight culture, a single colony is collected, and inoculated in 5 ml of an L-broth medium containing 50 μg/ml ampicillin, and then shaking culture is conducted overnight at 37°C (preculture).

In a flask for culture including the medium described above in an amount of 400 ml is inoculated each 2. 5 ml of the preculture liquid followed by shaking culture at about 37°C overnight (regular culture).

Next, for extracting the SA-24 plasmid DNA, the culture liquid in the regular culture is centrifuged at 4° C for 10 minutes at 4,000 × g to collect the microorganism. The culture supernatant is removed, and thereto is added 20 ml of STE (10 mmol/l Tris-hydrochloric acid (pH 8.0), 1 mmol/l disodium ethylenediamine tetraacetate (EDTA), 0.1 mmol/l sodium chloride) to resuspend the cell bodies. Then, centrifugation is conducted at 4° C for 10 minutes at 4,000 × g to collect the microorganism. Thereto is added 5 ml of a solution-1 (50 mmol/l glucose, 25 mmol/l Tris-hydrochloric acid (pH 8.0), 10 mmol/l EDTA) containing 10 mg/ml lysozyme, and the cell are suspended therein followed by leaving to stand still at room temperature for 5 minutes. Thereto is added 10 ml of a solution-2 (0.2 mmol/l sodium hydroxide, 1% sodium dodecyl sulfate (SDS)) mixed by inversion and left to stand on ice for 10 minutes. Thereto is added 7.5 ml of an ice cold solution-3 (3 mol/l potassium acetate (pH 4.8)) mixed by inversion and left to stand on ice for 10 minutes.

After centrifugation by a high speed refrigerated centrifuge at 4°C for 30 minutes at 45,000×g, the supernatant is recovered, and left to stand to cool to room temperature. After leaving to stand, 0.6 volume of isopropanol (about 24 ml) is added thereto, mixed by inversion and left to stand at room temperature for 5 minutes or longer. After centrifugation by a high speed refrigerated centrifuge at 25°C, for 30 minutes at 28,000×g, the supernatant is discarded, and thus resulting pellet is washed with 70% ethanol and air dried. After air drying, 8 ml of TE (10 mmol/l Tris-hydrochloric acid (pH 8.0), 1 mmol/l EDTA) is added thereto to dissolve the pellet (extraction of plasmid DNA).

Next, for the purification of the plasmid DNA containing SA-24, 800μl of 10 mg/ml ethidium bromide and 8.6 g of cesium chloride are added to the resulting plasmid DNA followed by mixing by inversion to dissolve the plasmid. The solution is placed in a centrifuge tube, which is then capped or sealed. After centrifugation at 20°C for 5 hours at 500,000 × g with a vertical rotor, a band of the plasmid DNA is fractionated using a glass syringe or an injection needle under the irradiation of an ultraviolet ray light. To the fractionated plasmid DNA solution is added an equivalent amount of TE-saturated 1-butanol followed by mixing by inversion and centrifugation at 15,000 × g for 5 minutes by a high speed microcentrifuge to remove the supernatant. This operation is repeated to eliminate ethidium bromide in the plasmid DNA solution. Next, thereto is added TE to give the volume of 1.5 ml followed by desalting on a demineralization column (NAP-10). To the desalted plasmid DNA solution is added 30 μl of a 3 mol/l sodium acetate solution followed by mixing, and 3 fold amount of 99.5% ethanol is added thereto followed by mixing by inversion and leaving to stand at -20°C for 30 minutes or longer. After leaving to stand, centrifugation is conducted with a high speed refrigerated micro centrifuge at 4°C for 20 minutes at 15,000 × g to remove the supernatant. Thereafter, cold 70% ethanol is added thereto to suspend therein, and once again, centrifugation is conducted with a high speed refrigerated micro centrifuge at 4°C for 20 minutes at 15,000 × g to remove the supernatant. Thus resulting precipitate of the plasmid DNA is evaporated to dryness under a reduced pressure. TE in an amount of 100 μl is added to the plasmid DNA to dissolve completely, and the concentration is measured on the basis of the absorbance at 260 nm (Purification of plasmid DNA containing SA-24). Then, size check of the plasmid DNA containing SA-24 is carried out by a treatment with arestriction enzyme and agarose electrophoresis.

For conducting purification of SA-24 by the treatment of the plasmid DNA containing SA-24 using a restriction enzyme and agarose electrophoresis, 1 mg of the plasmid DNA containing SA-24 after finishing the check of the molecular weight is combined with a restriction enzyme HindIII alone or with other restriction enzyme, and is digested by the reaction at 37°C for 1.5 hours. Following the digestion of the plasmid DNA, a part of the reaction liquid is electrophoresed on a 0.8% agarose to ascertain that the digestion is completely terminated. After confirming the digestion, a band of SA-24 is recovered through the electrophoresis on a 0.8% preparative agarose gel. Thus recovered SA-24 is extracted from the agarose gel and purified, and the concentration is measured with an absorbance meter. A part of the purified SA-24 is electrophoresed on a 0.8% agarose gel to verify that a single band is found. For labelling SA-24, 2 μg of the purified SA-24 is used, and may be subjected to digoxigenin labelling in a reaction liquid having the composition described in Table 1 below.

Table 1:

| Composition of the reaction liquid for labeling | |
|---|---|
| | Amount included (μL) |
| DNA probe | X |
| 10 × L.B.(a) | 10 |
| 100 mmol/L dithiothreitol | 10 |
| dNTPs(b) (A, G, C: 0.5 mmol/L) | 4 |
| digoxigenin-dUTP(c) (0.4 mmol/L) | 5 |
| DNase I(d) | 6 |
| 10 U/μL DNA polymerase I | 2 |
| Sterile purified water | Y |
| Total | 100 |

[explanatory notes] (a) 10 x L.B.: 0.5 mol/L Tris-hydrochloric acid (pH 7.5), 50 mmol/L magnesium chloride, 0.5 mg/mL bovine serum albumines

(b) dNTPs: 0.5 mmol/L 2'-deoxyadenosine-5'-triphosphate, 0.5 mmol/L 2'-deoxyguanosine-5'-triphosphate, 0.5 mmol/L 2'-deoxycytidine-5'-triphosphate

(c) digoxigenin-dUTP: 0.4 mmol/L digoxigenin-11-2'-deoxyuridine-5'-triphosphate

(d) DNase I: deoxyribonuclease I is diluted in a solution of 25 mmol/L Tris-hydrochloric acid (pH 7.5) and 50% glycerin such that the amount of 50 to 150 mU per total volume of 100 μl is used to give the aforementioned amount included.

In Table 1, X represents the volume which may be added such that preferred concentration of the probe as described above is provided depending upon the concentration of the probe stock solution, and the amount Y of purified water is determined following this volume to adjust the final volume.

After the labelling, 100 μl of TE is added to the reaction liquid to terminate the reaction. The reaction terminated solution is poured into a spin column, and centrifuged at 4°C for 10 minutes at 380 x g to remove free nucleotides. Next, the concentration of the eluate is measured with an absorbance meter, and then adjusted to give 10 ng/μl with TE.

In order to verify the labelling, 0.5 μl of the labelled SA-24 is dropped onto a membrane, and air dried. The membrane is immersed in a blocking reagent, and blocked at room temperature for 30 minutes. In an alkaline phosphatase labelled anti-digoxigenin antibody solution diluted to 5,000 fold in 0.1 mol/l Tris-hydrochloric acid (pH 7.5) and 0.15 mol/l sodium chloride, is immersed the membrane at room temperature for 30 minutes. The membrane is immersed in 0.1 mol/l Tris-hydrochloric acid (pH 7.5), 0.15 mol/l sodium chloride, and washed twice by shaking at room temperature for 10 minutes. In 0.5 mol/l Tris-hydrochloric acid (pH 9.5), 0.15 mol/l sodium chloride and 50 mmol/l magnesium chloride is immersed the membrane at room temperature for 10 minutes. The membrane is immersed in the coloring reagent at room temperature under light shielding for 10 minutes. The membrane is immersed in TE to terminate the color development. Verification of the labelling is executed by the observation of bluish purple coloring at the potion under the spotting. For producing the spin column, a small amount of sterilized glass wool is packed in a 1 ml disposable syringe. Sephadex G-50 swelled with 1 mmol/l Tris-hydrochloric acid (pH 7.5), 1 mmol/1 EDTA and 0.1% SDS is filled in the syringe. The syringe is placed into a 15 ml disposable conical tube followed by centrifugation at 4°C for 10 minutes at 320×g to throw the excess buffer away. The syringe is drawn from the disposable conical tube, and after discarding the excreted buffer, the spin column is produced by placing the syringe on the bottom of a disposable conical tube which had been a 1.5ml Eppendorf tube placed therein.

To determine the specificity of the probe, dot blot hybridization may be carried out according to the following procedure.

First, for the denaturation of each spotted genomic DNA, each 100 ng of various types of bacterial genomic DNA as prepared is spotted to a nylon membrane (Pall Biodyne(R) type B, manufactured by Nihon Pall Ltd. ) on a filter paper (manufactured by Whatman, 3MM) saturated with a solution containing 0.5 mol/l sodium hydroxide and 1.5mol/l sodium chloride according to a conventional process, and the air dried membrane is left to stand for 10 minutes. Next, the membrane is allowed to stand still on the filter paper described above which is saturated with a solution containing 0.5 mol/l Tris-hydrochloric acid (pH 7.5) and 1.5 mol/l sodium chloride for 10 minutes to neutralize the denaturated DNA. Furthermore, it is left to stand still on the filter paper as described above which is saturated with a 2 × SSC (Standard Saline Citrate) solution for 5 minutes followed by rinsing. Thereafter, the membrane is air dried, immersed in a 2 × SSC solution and allowed for infiltration for 5 minutes. According to a conventional process, the membrane is immersed in a prehybridization solution within a plastic bag, and affinitized at 42°C for 60 minutes. The membrane is immersed in 15 ml of a hybridization solution containing 400 mg of the probe in the plastic bag, and the reaction is allowed at 42°C overnight. Next, the membrane is immersed in a solution containing 2×SSC and 0.1% SDS (sodium dodecyl sulfate), and washed for 5 minutes (repeated twice). Thereafter, the membrane is immersed in a solution containing 0.1×SSC and 0.1% SDS, and washed at 60°C, for 10 minutes (repeated three times). The membrane is then immersed in a 2×SSC solution, and washed for 5 minutes. The membrane is immersed in a solution containing 3% bovine serum albumines, 1% blocking buffer (manufactured by Boeringer), 0.1 mol/l Tris-hydrochloric acid (pH 7.5) and 0.15 mol/l sodium chloride, and is gently shaken for 30 minutes. Thereafter, the membrane is immersed in a solution of alkaline phosphatase labelled anti-digoxigenin antibody (manufactured by Boeringer) diluted to 5,000 fold in a solution containing 0.1 mol/l Tris-hydrochloric acid (pH 7.5) and 0.15 mol/l sodium chloride, and is gently shaken for 30 minutes. Next, the membrane is immersed in a solution containing 0.1 mol/l Tris-hydrochloric acid (pH 7.5) and 0.15 mol/l sodium chloride, and is shaken for 15 minutes (twice). The membrane is immersed in a solution containing 0.1 mol/l Tris-hydrochloric acid (pH 9.5), 0.1 mol/l sodium chloride and 5 mmol/l magnesium chloride, and is shaken for 5 minutes. The membrane is immersed in an NBT-BCIP solution (manufactured by GIBCO BRL), and the color development reaction is allowed under light shielding. In TE (10 mmol/l Tris-hydrochloric acid (pH 8.0), 1 mmol/l EDTA) is immersed the membrane to terminate the color development reaction, and is air dried. The prehybridization solution and the hybridization solution are as shown in Table 2 below.

Table 2

| | | [represented by ml] |
|---|---|---|
| | Prehybridization solution | Hybridization solution |
| Formamide | 7.5 | 6.75 |
| 20 x SSC solution | 3.75 | 3.75 |
| 100 x Denhardt's solution | 0.75 | 0.15 |
| 0.5 mol/L phosphate buffer | 0.75 | 0.6 |
| sterile purified water | 1.5 | 1.95 |
| 10 mg/mL salmon sperm DNA | 0.75 | 0.3 |
| 50% dextran sulfate | ---- | 1.5 |
| Total liquid volume | 15.0 | 15.0 |

The surfactant which may be used in the step of *in situ* hybridization is any of known surfactants. Surfactants are generally classified in anion surfactants, nonionic surfactants, cation surfactants and ampholytic surfactants.

Anion surfactants are also referred to as anionic surfactants, which yield an organic anion upon ionization in water. When a lipophilic group in the molecule of the surfactant is represented by R, examples of the anion surfactant include $RCOONa$, $RSO_3Na$, $RSO_4Na$ and the like. An aqueous solution of the surfactant containing a weakly acidic group such as $RCOONa$ is liable to be hydrolyzed and is weak alkaline. However, an aqueous solution of a surfactant having a strongly acidic group such as $RSO_3Na$, $RSO_4Na$ or the like is resistant to hydrolysis, which shall be neutral. Because it is anionic, it may lose surface activity in the presence of a large quantity of cationic substance, and may be inactivated in a strongly acidic circumstance.

Nonionic surfactants refer to those having a hydrophilic group which is nonionic. An ethylene oxide group ($-CH_2CH_2O-$) is often used as the hydrophilic group. As number of this group increases, hydrophilicity is increased. To the contrary, as number of the lipophilic group increases, lipophilicity is increased. Therefore, it is characterized in that surfactants with variously altered hydrophilicity and lipophilicity can be obtained. Because a nonionic surfactant does not ionize in water and is hardly affected by inorganic salts, less action is exerted also on a living body. In addition, the detergent action thereof is potent with comparatively less foaming, therefore, it is widely used not alone as a detergent, but in pharmaceuticals, cosmetics, foods and the like. Water soluble nonionic surfactant becomes insoluble in water at a certain temperature as the temperature rises, and then the aqueous solution starts to be turbid. Such turbidity results from the cleavage of hydrogen bonds between the hydrophilic groups and water.

Cation surfactants are also referred to as cationic surfactants, which yield an organic cation upon ionization in water. Although cation surfactants do not have potent detergent action in general, they strongly bind to anionic substances such as bacteria, leading to a great bactericidal action. Moreover, they also have an anti-static ability for fibers and plastics. Although dodecyltrimethyl chloride $[C_{12}H_{25}(CH_3)_3N]Cl$ as a typical exemplary cation surfactant is water soluble, didodecyldimethylammonium chloride $[(C_{12}H_{25})_2(CH_3)_2N]Cl$ is insoluble in water, which forms a vesicle in the form of a bimolecular film in water, and is soluble in benzene.

Ampholytic surfactants are surfactants having both an anionic group and a cationic group in the molecule. Ionization state thereof in water is similar to those of amino acids, and thus many of ampholytic surfactants are amino acid derivatives. Therefore, they have an isoelectric point similarly to amino acids, which act as an anion surfactant in an alkaline region from the isoelectric point, whilst as a cation surfactant in an acidic region. Water solubility becomes the lowest at the isoelectric point, and the surface tension is also reduced. Ampholytic surfactants are used for a bactericide, an antistatic agent or the like.

Furthermore; anion surfactants are classified into the carboxylic acid type, sulfonic acid type, sulfate ester type and phosphate ester type, whilst nonionic surfactants are classified into the ester type, ether type, ester ether type and alkanolamide type. Cation surfactants are classified into alkylamine salt type and quaternary ammonium salt type, whilst ampholytic surfactants are classified into carboxy betaine type, 2-alkylimidazoline derivative type and glycine type.

Moreover, the anion surfactants of carboxylic acid type are further classified into fatty acid monocarboxylate salts, N-acylsarcosine salts and N-acylglutamate salts. Representative examples thereof respectively include: sodium laurate and medicated soap as the fatty acid monocarboxylate salts; sodium N-lauroylsarcosine as the N-acylsarcosine salt; and disodium N-lauroylglutamate as the N-acylglutamate. Still more, the sulfonic acid type is further classified into dialkyl sulfosuccinate salts, alkane sulfonate salts, alpha-olefin sulfonate salts, straight chain alkyl benzenesulfonate salts, alkyl (branched chain) benzenesulfonate salts, alkyl naphthalenesulfonate salts, naphthalenesulfonate salts-formaldehyde condensates and N-methyl-N-acyltaurine salts. Representative examples include: sodium dioctyl sulfosuccinate as the dialkyl sulfosuccinate salt; sodium dodecane sulfonate as the alkane sulfonate; sodium straight chain dodecyl benzenesulfonate as the straight chain alkyl benzenesulfonate salt; sodium dodecyl benzenesulfonate as the alkyl (branched chain) benzenesulfonate salt; sodium butyl naphthalenesulfonate as the alkyl naphthalenesulfonate salt; and sodium N-methyl-N-stearoyltaurine as the N-methyl N-acyltaurine salt. In addition, the sulfate ester type is further classified into alkyl sulfate salts, polyoxyethylene alkyl ether sulfate salts and oil-and-fat sulfate ester salts. Representative examples include sodium dodecyl sulfate, sodium lauryl sulfate and sodium cetyl sulfate as the alkyl sulfate salt; and polyoxyethylene lauryl ether sulfate triethanolamine as the polyoxyethylene alkyl ether sulfate salt. Moreover, the phosphate ester type is further classified into alkyl phosphate salts, polyoxyethylene alkyl ether phosphate salts and polyoxyethylene alkylphenyl ether phosphate salts. Representative examples include disodium monolauryl phosphate as the alkyl phosphate salt; and sodium polyoxyethylene lauryl ether phosphate and polyoxyethylene oleyl ether phosphate (8MOL) as the polyoxyethylene alkyl ether phosphate salt.

Ester type of the nonionic surfactants is further classified into fatty acid glycerin, fatty acid sorbitan and fatty acid sucrose ester. Representative examples respectively include: glycerin monostearate as the fatty acid glycerin; sorbitan monostearate, sorbitan trioleate, sorbitan sesquioleate, sorbitan monolaurate, polysorbate 20 (polyoxyethylene sorbitan fatty acid ester), polysorbate 60 and polysorbate 80 as the fatty acid sorbitan; and stearic acid sucrose ester as the fatty acid sucrose ester. Additionally, the ether type is further classified into polyoxyethylene alkyl ether, polyoxyethylene alkyl phenyl ether and polyoxyethylene polyoxypropylene glycol. Representative examples include: polyoxyethylene lauryl ether, polyoxyethylene stearyl ether and polyoxyethylene cetyl ether as the polyoxyethylene alkyl ether; and polyoxyethylene nonyl phenyl ether and polyoxyethylene octyl phenyl ether as the polyoxyethylene alkyl phenyl ether. In addition, the ester ether type is further classified into fatty acid polyethylene glycol and fatty acid polyoxyethylene sorbitan. Representative examples thereof respectively include oleic acid polethylene glycol as the fatty acid polyethylene glycol; and polyoxyethylene sorbitan palmitate and polyoxyethylene sorbitan monolaurate as the fatty acid polyoxyethylene sorbitan. In addition, the alkanolamide type involves only fatty acid alkanolamide alone. Representative example is lauric diethanolamide.

The alkyl amine salt type of the cation surfactant includes monoalkyl amine salts, dialkyl amine salt and trialkyl amine salts. Representative examples thereof include monostearyl amine hydrochloride. Moreover, the quaternary ammonium salt type is further classified into alkyltrimethyl ammonium chloride (or bromide or iodide), dialkyldimethyl ammonium chloride (or bromide or iodide), and alkyl benzalkonium chloride. Representative examples respectively include: stearyltrimethyl ammonium chloride as the alkyltrimethyl ammonium chloride (or bromide or iodide); distearyldimethyl ammonium chloride as the dialkyldimethyl ammonium chloride (or bromide or iodide); and lauryl benzalkonium chloride as the alkyl benzalkonium chloride.

The carboxy betaine type of the ampholytic surfactant is only alkyl betaine alone. Representative example is lauryl betaine. Additionally, the 2-alkyl imidazoline derivative type is only 2-alkyl-N-carboxymethyl-N-hydroxyethyl imidazolinium betaine alone. Representative example includes 2-undecyl-N-carboxymethyl-N-hydroxyethyl imidazolinium betaine. In addition, the glycine type may be alkyl (or dialkyl) diethylene triaminoacetic acid, and the representative example includes dioctyl diethylene triaminoacetic acid.

Moreover, in addition to the representative examples as described above, Triton X-100, lauryl sarcosine, saponin, BRIJ35, alkyl allyl polyether alcohol, higher alcohol sulfate, N-cocoyl-L-arginine ethyl ester DL-pyrrolidone carboxylate salt, sodium N-cocoyl-N-methyl aminoethyl sulfonate, cholesterol, self emulsifying type monostearate glycerin, squalane, stearyl alcohol, stearic acid polyoxyl 40, cetyl alcohol, cetomacrogol 1000, sebacate diethyl, nonylphenoxy polyoxyethylene ethane sulfate ester ammonium, polyoxyethylene oleylamine, polyoxyethylene sorbit yellow bees wax, polyoxyl 35 castor oil, macrogol 400, N-coconut oil fatty acid acyl L-arginine ethyl·DL-pyrrolidone carboxylate salt, lauryldimethylamine oxide solution, lauromacrogol, methylcellulose, CMC (carboxymethylcellulose), polyoxyethylene hardened castor oil 20 and polyoxyethylene hardened castor oil 60, CHAPS, deoxycholic acid, digitonin, n-dodecyl maltoside, Nonidet P40, n-octyl glucoside, octyl thioglucoside, laurate sucrose, dodecyl poly(ethylene glycol ether)n, n-dodecyl-N,N-dimethyl-3-ammonio-1-propanesulfonate and the like are also included.

Various surfactants as listed above are essentially used in the step of *in situ* hybridization, but the process for use is not particularly limited. For example, the surfactant may be admixed in the probe solution or probe dilution solution, alternatively, a solution containing the surfactant which was separately prepared from the probe solution may be added prior to, concurrently with or later than coating of the probe solution on the smear site. Such a process may be altered ad libitum by the person skilled in this art.

In the present invention, when a positive control probe is required, it can be produced as follows. For example, in order to conduct the extraction and purification of the genomic DNA of U937 cell (ATCC CRL-1593.2), U937 cells are first cultured in a 5% carbon dioxide gas incubator at 37° C using an RPMI1640 medium (25 ml) in a cell culture flask (175 $cm^2$). The U937 culture solution is placed in a 50 ml centrifuge tube, and centrifuged at 4° C for 10 minutes at 220×g to recover the U937 cells. The cells are suspended and washed in 10 ml of PBS, and again centrifuged at 4° C for 10 minutes at 180×g to recover the cells. Thereafter, the supernatant is discarded, and the cells are suspended in 1 ml of a TE solution containing 200 μg/ml proteinase K and containing 1% SDS, followed by leaving to stand at 37°C for 30 minutes. Phenol extraction is repeated three to four times to execute deproteinization. Genome deposited through the ethanol precipitation is recovered, dissolved in 500 μl of sterile purified water containing 2.5 pg of ribonuclease, and left to stand at 42°C for 30 minutes.

The phenol extraction is repeated two to three times to execute deproteinization. Genome deposited through the ethanol precipitation is recovered, and dissolved in 500 μl of TE. Thereafter, a positive control probe can be produced by measuring the concentration with an absorbance meter, and subjecting to digoxigenin labelling. Moreover, the positive control probe which may preferably used is one which permits to ascertain the hybrid formation when the positive control probe is subjected to dot hybridization on a membrane with 100 ng of U937 genome spotted thereon. When a negative control probe is required, it can be produced by any known method.

Preparation of digested sample

Specific process for producing a phagocyte post phagocytosis of the present invention (hereinafter, referred to as digested sample ) is illustrated below.

Materials for use which are required include U937 cell (human monocyte established cell: ATCC CRL-1593.2), *Staphylococcus aureus* (ATCC 126000), *Staphylococcus epidermidis* (ATCC 14990), *Pseudomonas aeruginosa* (ATCC 10145), *Enterococcus faecalis* (ATCC 19433), *Escherichia coli* (ATCC 11775), heparinized healthy human blood, brain heart infusion (BHI) (manufactured by DIFCO), RPMI 1640 (RPMI medium 1640 (manufactured by GIBCO)) containing Fetal Bovine Serum (final concentration: 10%, manufactured by GIBCO) and Antibiotic-Antimycitic (final concentration: 1%, manufactured by GIBCO) and the like.

Instruments for use which are required include a carbon dioxide gas incubator (manufactured by Tabai Espec Corporation: BNA-121D type), a low speed refrigerated centrifuge (manufactured by Beckman: CS-6KR type), a counting chamber (manufactured by Elmer: bright line type), a shaking incubator (manufactured by TIETECH Co., Ltd. : BR-300L type), an absorbance meter (manufactured by Beckman: DU68 type), an incubator (manufactured by Yamato Scientific Co., Ltd.: IC-62 type), an incident-light inverted microscope (manufactured by Nikon: DIAPHOT type), a fluorescence microscope (manufactured by Nikon: OPTIPHOT type), a CCD camera (manufactured by Hamamatsu Photonics KK.: C5810-01 type) and the like.

First, for preparing U937 cells, U937 cells (human monocyte established cell: ATCC CRL-1593.2) are cultured in an RPMI 1640 medium within a cell culture flask (e.g., 175 $cm^2$) in a 5% carbon dioxide gas incubator at about 20 to about 40°C, preferably at about 37° C. The cell culture flask is preferably one including a material consisting of a component which is liable to adhere to cells. Next, the U937 cell culture liquid is placed in a centrifuge tube, and centrifuged at 0° C to about 10° C, preferably at about 4°C for about 10 minutes at about 150 to about 350×g, preferably about 220×g to recover the U937 cells. Then, thus recovered U937 cells are suspended in PBS, and the cell number is counted with a counting chamber. The cell number may be adjusted to about $1\times10^4$ cells/μl to $2\times10^4$ cells/μl.

For preparing a bacterial digested sample, *Staphylococcus aureus, Staphylococcus epidermidis, Pseudomonas aeruginosa, Enterococcus faecalis* and *Escherichia coli* are inoculated in a BHI culture liquid (supra), and cultured at about 20 to about 40° C, preferably at about 37° C for 6 hours or longer. The cultured bacterial liquid is centrifuged at 0 to about 10°C, preferably at 4° C, for example, at 2,000×g for about 10 minutes to collect the bacteria. After discarding the supernatant, it is preferred that pellet of the bacteria is suspended using PBS, and centrifuged once again at 4° C for 10 minutes at 2,000×g to collect the bacteria. After suspending thus collected bacteria in PBS, they may be diluted in PBS to produce a bacterial liquid prepared such that it has the turbidity (O.D. = 600 nm) of about 0.001 to about 0.1, preferably about 0.01 to about 0.03, and in particular, about 0.01 to about 0.03 for *Staphylococcus*

*aureus,* about 0.01 to about 0.03 for *Staphylococcus epidermidis*, about 0.02 to about 0.03 for *Pseudomonas aeruginosa,* about 0.01 to about 0.03 for *Enterococcus faecalis*, and about 0.02 to about 0.03 for *Escherichia coli,* respectively, when measured with an absorbance meter. Thus produced bacterial liquid is transferred to a discrete culture flask, and left to stand still for about 30 minutes at room temperature. Heparinized healthy human blood is collected, and thereto is added the aforementioned reagent for separating hemocyte at a ratio of approximately 4 : 1, and left to stand still at about 20 to about 40°C, preferably at about 37°C for 30 minutes to yield the leukocyte fraction. Thus obtained leukocyte fraction is suspended in PBS. The supernatant in the culture flask is gently discarded, and the leukocyte fraction diluted in PBS is added to the flask followed by leaving to stand still at room temperature for about 10 minutes. The supernatant in the culture flask is discarded, and the leukocytes attached to the bottom of the flask are recovered in a centrifuge tube with PBS containing 0.02% EDTA, and centrifuged e.g., at 4°C for 10 minutes at about 140 to about 180 × g to collect the leukocytes. When contamination of erythrocytes is found in the collected leukocytes, precipitates of the leukocytes are gently suspended in sterile purified water to allow hemolysis, subjected to isotonization through adding PBS, followed by centrifugation once again at 4° C for 10 minutes at about 140 to about 180×g to collect the leukocytes. The collected leukocytes are suspended in PBS, and cell number is counted with a counting chamber to adjust to give about $1 \times 10^4$ cells/μl to about $5 \times 10^4$ cells/μl. These digested samples are referred to as SA digested sample, SE digested sample, PA digested sample, EF digested sample and EK digested sample.

For conducting the smear fixation, the prepared U937 cells and each bacterial digested sample produced as described above is smeared on each well of an APS coated slide glass followed by air drying.

It is preferred that cell number of each bacterial digested sample smeared and fixed on the slide glass is about $5.0 \times 10^4$ to about $2.5 \times 10^5$ cells/well, while cell number of U937 cells is about $5.0 \times 10^4$ to about $1.0 \times 10^5$ cells/well. For the fixation, the sample is immersed in Carnoy' s fixative(supra) for 20 minutes and thereafter immersed in 75% ethanol for 5 minutes. After washing Carnoy's fixative and air drying, the sample may be stored at 4°C until use in the test.

Measurement of the phagocytosis rate is executed by staining the bacterial digested sample smeared and fixed on the slide glass with an acridine orange staining solution, and counting about 200 cells randomly with a fluorescence microscope (×1,000). Among the measured cells, cells including bacteria phagocytized within the cells are determined as positive cells, and the phagocytosis rate (%) is calculated according to the mathematical formula below.

$$\text{Phagocytosis rate (\%)} = [(\text{Positive cell number}/\text{Measured cell number}) \times 100]$$

Fig. 6 illustrates the state of the phagocytes prepared and observed by microscopic examination. Specific operation process involving Carnoy fixation, treatment for promoting permeability of the leukocyte cell membranes, lytic treatment, acetylation of the cell membrane protein, alkaline treatment of DNA of the bacterial body, *in situ* hybridization, blocking, reaction with labelled antibody, detection, and determination, which may be employed is as described herein.

Further, the present invention also includes a kit for evaluating a phagocytotic function which comprises fixing phagocytes post phagocytosis of a foreign microorganism, executing a treatment for promoting permeability of the cell membranes of the phagocytes, executing a treatment for exposing the DNA of the foreign microorganism existing in the phagocytes, carrying out in situ hybridization using a DNA probe for detection capable of hybridizing with the DNA under a stringent condition in the presence of a surfactant; and evaluating the phagocytotic function by the resulting signal, the kit having (1) the foreign microorganism, (2) at least one or more enzyme selected from the group consisting of lysostafin, lysozyme, N-acetylmuramidase and zymolase used in the exposing step of the DNA, and (3) one or more DNA probe for detection.

This kit includes, reagent for separating blood, enzyme pretreatment reagent, enzyme reagent, acetylation reagent, probe solution, blocking reagent, labelled antibody, labelled antibody diluent, coloring pretreatment liquid-1, coloring pretreatment liquid-2, coloring reagent, counter staining solution, PBS stock solution, hybridization stock solution, labelled antibody washing solution, coloring reagent washing solution, APS coated slide glass, probe dilution solution, buffer A and the like as demonstrated in the following Examples. Among these, it is preferred that at least the enzyme reagent and the probe solution are included. In addition, various reagents used in the present invention may be included for example, chloroform, ethanol, acetic anhydride, DMSO, PMSF, formamide, acetic acid, hydrochloric acid, sodium hydroxide and the like. Moreover, instrument and machine such as low speed centrifuge, incubator, counting chamber, shaker, humid box, incubator, light microscope, variable pipette, blood collection tube, tip, pipette, staining bottle, measuring cylinder, glass syringe, 0.2 μm syringe top filter may be included.

Furthermore, the present invention provides a process for monitoring the gene of a foreign microorganism phagocytized by a phagocyte included in a clinical specimen which contains a phagocyte derived from a living body.

Moreover, the present invention provides a process for identifying the gene of a microorganism which becomes a candidate of the causative microorganism which a causative microorganism of sepsis or a causative microorganism of bacteremia is specified on the basis of the results identified.

The clinical specimen which may be used herein is a clinical specimen which contains a phagocyte derived from a living body, and examples thereof include body fluids such as blood, tissue fluid, lymph fluid, cerebrospinal fluid, pyo, mucus, snot, sputum and the like. Additionally, in compliance with the disease states such as diabetes, renal disorder, hepatic disorder or the like, phagocytes derived from the living body may be included in urine, ascites, dialysis drainage and the like as well as in lavage obtained after washing nasal cavity, bronchial tube, skin, various organs, bone or the like, therefore, these may be used as the clinical specimen according to the present invention. In addition, tissues such as skin, lung, kidney, mucosa and the like may be used as the clinical specimen. Because a macrophage which is one of the phagocytes varies to several forms such as monocyte, pulmonary alveolus macrophage, peritoneal cavity macrophage, fixed macrophage, free macrophage, Hansemann macrophage, inflammatory macrophage, liver Kupffer cell, brain microglia cell, not only blood but also tissues including these cells can be used as the clinical specimen of the present invention. For example, a causative microorganism of nephritis can be detected and identified through obtaining the renal tissue from a patient suspected as suffering from nephritis by kidney biopsy, obtaining phagocytes which are present in the tissue by detaching the cells using an enzyme such as trypsin or the like, and using thus resulting phagocytes.

It was revealed that when this process was applied in practice to diagnoses for blood of a variety of patients suspected as suffering from sepsis, causative microorganism could be detected with about 4 times higher sensitivity compared to the blood culture process with no influence of the administered antimicrobial agent, and the identity of the detected microorganism strain was favorable. Furthermore, in comparison with the blood culture which requires 3 days or longer and approximately 14 days for the examination, an accurate result can be achieved by a simple operation within a short time period, i.e., about 8 hours, until the completion of the entire operation, according to the process of the present invention. Therefore, a useful marker can be provided in the monitoring and the like in prognosis or diagnosis of an infectious disease such as sepsis, bacteremia or the like in which a rapid and favorable care is required, in particular.

According to one embodiment of the present invention, a performance test is provided which is characterized in that a phagocyte post phagocytosis of a foreign microorganism is used, and examples of the test include sensitivity tests, specificity tests, reproducibility tests and the like of a kit for evaluating a phagocytotic function. In these tests, a phagocyte post phagocytosis of a foreign microorganism can be used as a positive control. When a digested sample is used in the performance test for *Staphylococcus aureus,* particularly in a sensitivity test, it may be defined that a signal can be detected when the test is performed according to the *in situ* hybridization process described herein using a digested sample of *Staphylococcus aureus.*

Additionally, upon performing a specificity test, it may be defined that a signal can be detected for *Staphylococcus aureus* alone, when the test is performed according to the *in situ* hybridization process described herein using various bacterial digested samples.

Further, upon performing a reproducibility test, it may be defined that the achieved results are identical when the specificity test is performed by concomitantly repeating three tests. Also in respect of other bacteria, e.g., *Staphylococcus epidermidis, Pseudomonas aeruginosa, Enterococcus faecalis, Escherichia coli, Enterobactor cloacae* and *Klebsiella pneumoniae*, definition may be made with reference to the performance tests as described above.

Moreover, when a digested sample is used as a positive control in the performance test such as sensitivity test, specificity test, reproducibility test or the like as described above, in connection with the standard of the digested sample and the process for testing, cell number smeared and fixed on the slide glass of each bacterial digested sample is preferably about $5.0\times10^4$ to about $2.5\times10^5$ cells/well, whilst cell number of U937 cells is preferably about $5.0\times10^4$ to about $1.0\times10^5$ cells/well.

Moreover, upon measurement of the phagocytosis ratio, specific morphology of a phagocyte can be observed as shown in Fig. 6, when a bacterial digested sample smeared and fixed on the slide glass is stained with an acridine orange staining solution, and about 200 cells are randomly counted with a fluorescence microscope ( $\times$ 1,000). Accordingly, cells including bacteria phagocytized within the cells are determined as positive cells among the measured cells, and the phagocytosis rate (%) is calculated.

$$\text{Phagocytosis rate (\%)} = [(\text{Positive cell number} / \text{Measured cell number}) \times 100]$$

In the process for evaluating phagocytotic function for a foreign microorganism, evaluation may be made by not only the signal obtained by carrying out *in situ* hybridization, but also for example, calculation through employing the phagocytosis rate as described above. Hence, the process for evaluating a phagocytotic function can be performed on the basis of the morphologic observation by the *in situ* hybridization process and staining. Such an evaluation process can be also utilized in a process for evaluating an immune function of a living body, a process for evaluating differentiation efficiency into a phagocyte, a process for evaluating a modulator against a phagocytotic function, a process for screening, a process for the clinical test to examine a dosage regimen of an agent.

Suitable immune function may be a phagocytotic ability for a living microorganism by a leukocyte, in particular, a phagocytotic ability for a living microorganism by a leukocyte of a patient after the radiation exposure or the administration of an anticancer agent. For example, when a certain agent is administered intending to promote or antagonize a function of a phagocyte such as potentiation of a declined immune system accompanied by the administration of a chemotherapeutic agent in a cancer therapy, suppression of a rejection symptom upon organ transplantation, and the like, this process can ascertain whether or not the agent effectively acts *in vivo* actually. Therefore, a useful guideline can be provided for the selection of a drug or a dosage.

Additionally, the process of the present invention has an effect to contribute to a basic study and a clinical study in regard to the interaction between a microorganism in the field of bacteremia and a phagocyte, and may be also employed in the determination of effectiveness of a modulator of a phagocytotic function or in the screening of a novel substance having a modulatory action against a phagocytotic function. Also in this process, the aforementioned process for evaluating a phagocytotic function is utilized, therefore, substantial effects by a modulator such as an agonist or an antagonist toward a subject can be assessed with higher reliability than any conventional process.

Further, because effects on a certain individual by a modulator which may cause great individual differences in terms of the effectiveness, side effects and the like can be identified, it may be helpful in the determination of a medical guideline in an order made fashion which is suited to each patient. In other words, a clinical testing process is provided which is characterized in: obtaining phagocytes from a subject prior to and following the administration of an agent to the subject; evaluating a function of the phagocyte by the process as described above; and examining a dosage regimen of the agent judging from the effect of the agent determined on the basis of the evaluation result.

The modulator is not limited as long as it is a substance which directly or indirectly participates in a phagocyte, for example, a substance which promotes or suppresses the differentiation of a phagocyte, a substance which promotes or suppresses a phagocytotic function, or the like. Examples thereof include G-CSG, anticancer agents, antibiotics, immune function activators, leukocyte differentiation factors and the like.

## EXAMPLES

Although the present invention is specifically explained by way of Examples below, as a matter of course, the disclosure of these Example should not be construed as limiting the present invention.

### Example 1

### Collection of Blood, Treatment of Blood Specimen

As clinical specimens, 12 specimens of blood collected from patients suspected as suffering from sepsis (specimens A to L) were used. Ten ml of heparinized venous blood was collected from each patient, and after admixing the blood with a reagent for separating blood (225mg of sodium chloride, 1.5 g of dextran (MW: 200,000-300,000)), adjusted to give the total volume of 25 ml with sterile purified water) at a ratio of 4:1, a leukocyte fraction (upper layer) was obtained by leaving to stand still at 37°C for 30 minutes. Leukocytes were obtained by centrifugation of the resultant leukocyte fraction at 4°C for 10 minutes at 160×g. Next, 1 ml of sterile purified water was added to thus resulting pellet of the leukocytes and suspended, and immediately thereafter an excess amount of PBS (18.24 g of sodium chloride, 6.012 g of sodium monohydrogen phosphate 12 hydrate, 1.123 g of sodium dihydrogen phosphate dihydrate, adjusted to give the total volume of 120 ml with sterile purified water (PBS stock solution) diluted to 20 fold with sterile purified water)was added thereto to result in isotonization, followed by centrifugation once again at 4° C for 10 minutes at 160×g.

### Example 2

### Fixation of Leukocytes

An APS coated slide glass was used which is a slide glass (manufactured by JAPAN AR BROWN CO., LTD., item number: MS311BL) with 3-aminopropyltriethoxysilane (APS, SIGMA) coated thereon. For producing the APS coated slide glass, a slide glass (item number: MS311BL) was first fixed on a slide holder, and thereafter was washed by immersing in a diluted neutral detergent for 30 minutes, and the detergent is sufficiently removed with running water. Next, the slide glass was washed with purified water and sufficiently dried at high temperature (100°C or greater) followed by leaving to stand to cool at room temperature. Then, this slide glass was immersed in acetone containing 2% APS for 1 minute, and immediately thereafter washed briefly with acetone and sterile purified water sequentially followed by air drying. In addition, after conducting the operation once again of immersing the slide glass in acetone containing about 2% APS for 1 minute, followed by immediate and brief washes with acetone and sterile purified water in a sequential manner and air drying, the APS coated slide glass was produced by drying at 42°C.

Leukocyte cell number of the leukocyte fraction is measured using a counting chamber after adding a small amount of PBS to the leukocytes pellet obtained by centrifugation at 4°C for 10 minutes at 160 × g followed by suspending therein. Leukocytes were supported on the APS coated slide glass by smearing 5 μl of the leukocyte suspension, which was prepared to yield the cell number of 1 x $10^5$ cells/well with PBS, on each well of the APS coated slide glass such that the leukocytes are spread over to give a single layer, and completely air drying. Thereafter, the slide glass was immersed in Carnoy' s fixative (a mixed solution at a volume ratio of ethanol : chloroform : acetic acid = 6 : 3 : 1) for 20 minutes, then immersed in 75% ethanol solution for 5 minutes, and completely air dried.

Example 3

The slide glass was immersed in PBS for 10 minutes, and thereafter, in a solution of an enzyme pretreatment reagent (prepared by mixing 1.25 g of saponin, 1.25 ml of t-octylphenoxypolyethoxyethanol (specific gravity: 1.068 to 1.075 (20/4°C), pH (5 w/v%) 5.5 - 7.5) and 25 ml of the PBS stock solution, and adjusting to give the total volume of 50 ml with sterile purified water) diluted to 10 fold in sterile purified water, and allowing infiltration on a shaker for 10 minutes.

Example 4

Enzymatic Lysis Treatment of Wall of Bacterial Body

In order to expose the DNA of a causative microorganism of an infectious disease, an enzyme reagent solution was prepared by adding 1 ml of an enzyme reagent dissolving solution (prepared by 100 fold dilution of dimethylsulfoxide (DMSO) which contains 0.1 mol/l phenylmethylsulfonylfluoride (PMSF) in PBS) to an enzyme reagent (N-acetylmyramidase 1,000 units/ml, lysozyme 100,000 units/ml and/or lysostafin 100 units/ml) per 1 slide glass, and thereafter, 1 ml of this enzyme reagent solution was dropped on a site of the leukocyte smear, and left to stand still for 30 minutes in a humid box at 37°C to 42°C. Then, it was immersed in PBS containing 0.2 mol/l hydrochloric acid (prepared by adding hydrochloric acid to the PBS stock solution, 20 fold dilution in sterile purified water, and adjusting to give the final concentration of hydrochloric acid of 0.2 mol/l) and allowed infiltration on a shaker for 10 minutes as it was.

Example 5

Acetylation of Cell Membrane protein

Acetylation was carried out through immersing the slide glass in an acetylation reagent, which was prepared by adding acetic anhydride to an acetylating reagent (7.46 g of triethanolamine, an appropriate amount of hydrochloric acid, adjusted to give the total volume of 50 ml with an appropriate amount of sterile purified water) and diluting 10 fold in sterile purified water to give the final concentration of acetic anhydride of 0.8%, followed by shaking for 10 minutes on a shaker. Thereafter, the slide glass was sequentially immersed in 75%, 85%, and 98% ethanol for 3 minutes respectively, and completely air dried.

Example 6

Alkaline Treatment of DNA of Bacterial Body (Denaturation from double strand to single strand)

An alkaline treatment was carried out through immersing the slide glass in PBS which contains 70 mmol/l sodium hydroxide (prepared by adding sodium hydroxide in the PBS stock solution, diluting to 20 fold with sterile purified water to give the final concentration of sodium hydroxide of 70 mmol/l) for 3 minutes. Thereafter, the slide glass was sequentially immersed in 75%, 85%, and 98% ethanol for 3 minutes respectively, and completely air dried.

Example 7

Hybridization

A solution containing 15 ng of a digoxigenin labelled DNA probe prepared with a probe dilution solution (including 0.25% SDS, 600 μl of salmon sperm DNA, 50 μl of 100×Denhardt s solution, 500 μl of a hybridization stock solution, 2250 μl of formamide, 1000 μl of 50% dextran sulfate) is coated on the smeared site, and the slide was left to stand still in a humid box at 37°C to 42° C for 2 hours. A probe solution without including SDS was determined as a control. The digoxigenin labelled DNA probe was produced by a nick translation method. Thereafter, a hybridization washing solution (prepared by mixing a hybridization stock solution (13.15 g of sodium chloride, 6.615 g of trisodium citrate dihydrate, adjusted to give the total volume of 75 ml with sterile purified water) in a ratio of the hybridization stock solution : sterile purified water : formamide = 5 : 45 : 50) was provided in three staining bottles, and sequentially the sample was immersed at 42° C for 10 minutes, respectively.

Then, the sample was immersed in PBS, and shaken as it is on a shaker for 10 minutes. Digoxigenin labelled DNA probe utilized was each probe of SA-24 (SEQ ID NO: 1), SA-36 (SEQ ID NO: 2) and SA-77 (SEQ ID NO: 3), and SE-22 (SEQ ID NO: 4), SE-3 (SEQ ID NO: 5) and SE-32 (SEQ ID NO: 6) (see, Japanese Patent No. 2798499), as a probe for *Staphylococcus aureus* and *Staphylococcus epidermidis.* Further, as a probe for *Pseudomonas aeruginosa*, the probe of P2-2 (SEQ ID NO: 7) (see, Japanese Patent No. 2965544) was utilized. In addition, as probes for *Enterococcus faecalis*, EF-1 (SEQ ID NO: 8), EF-27 (SEQ ID NO: 9) and EF-7 (SEQ ID NO: 10) (see, Japanese Patent No. 2965543) were utilized. Additionally, as probes for *Escherichia coli, Enterobacter cloacae* and *Klebsiella pneumoniae,* EC-24 (SEQ ID NO: 11), EC-34 (SEQ ID NO: 12) and EC-39 (SEQ ID NO: 13), and ET-49 (SEQ ID NO: 14) and KI-50 (SEQ ID NO: 15) (see, Japanese Patent No. 3026789) were utilized. In addition, as probes for *Candida albicans,* CA-26 (SEQ ID NO: 16), CA-26-1 (SEQ ID NO: 17), CA-26-2 (SEQ ID NO: 18) and CA-26-3 (SEQ ID NO: 19) (see, Japanese Patent No. 2558420) were utilized. Using each sequence of these probes, each probe was produced by a nick translation method.

## Example 8

### Blocking

After carrying out *in situ* hybridization, an operation of blocking was performed. One ml of a blocking solution (2 ml of normal rabbit serum, 0.5 ml of the PBS stock solution, adjusted to give the total volume of 10 ml with sterile purified water) was dropped on the smear site per one slide glass in a humid box, and left to stand still for 30 minutes. Thereafter, the blocking reagent was removed.

## Example 9

### Reaction with Labelled Antibody

A labelled antibody solution was prepared by diluting a labelled antibody (1.05 unit of alkaline phosphatase labelled anti-digoxigenin antibody solution, adjusted with 12.6 µl of buffer A (746 mg of triethanolamine, 17.5 mg of sodium chloride, 20.3mg of magnesium chloride hexahydrate, 1.36 mg of zinc chloride, 1000 mg of bovine serum albumine, an appropriate amount of hydrochloric acid, adjusted to give the total volume of 100 ml with sterile purified water) to give the total volume of 14 µl) in a labelled antibody diluent (8.48 mg of Tris-(hydroxymethyl)-aminomethane, 6.14 mg of sodium chloride, an appropriate amount of hydrochloric acid, adjusted to give the total volume of 0.7 ml with sterile purified water) to 50 fold, and each 10 µl of this labelled antibody solution was dropped on the smear site, followed by leaving to stand still for 30 minutes. Thereafter, it was immersed in a solution of a labelled antibody washing solution (1 ml of polysorbate 20, 50 ml of the PBS stock solution, adjusted to give the total volume of 100 ml with sterile purified water) diluted to 10 fold, and was allowed for infiltration on a shaker for 10 minutes as it was. After repeating this operation twice, it was immersed in a coloring pretreatment liquid obtained by mixing a coloring pretreatment liquid 1 (6.06 g of Tris-(hydroxymethyl)-aminomethane, 2.92 g of sodium chloride, an appropriate amount of hydrochloric acid, adjusted to give the total volume of 50 ml with sterile purified water) and a coloring pretreatment liquid 2 (5.08 g of magnesium chloride hexahydrate, adjusted to give the total volume of 50 ml with sterile purified water) in an equivalent volume and diluting to 5 fold with sterile purified water, and then shaken for 10 minutes on a shaker as it was.

## Example 10

### Detection

One ml of a coloring reagent (nitroblue tetrazolium (NBT)/-5-bromo-4-chloro-3-indolylphosphate (BCIP) solution, pH 9.0 to 10.0: 3.3 mg of NBT, 1.65 mg of BCIP, 99 pg of N,N-dimethylformamide, 121 mg of Tris-(hydroxymethyl)-aminomethane, an appropriate amount of hydrochloric acid, 58.4 mg of sodium chloride, 101. 6 mg of magnesium chloride hexahydrate, adjusted to give the total volume of 10 ml with an appropriate amount of sterile purified water) per one slide glass was dropped on the smear site of the slide glass while filtration using a disposable syringe equipped with a 0.2 µm syringe top filter, and was left to stand still under light shielding in a humid box at 37°C for 30 minutes.

Thereafter, it was immersed in a solution of a coloring reagent washing solution (606 mg of Tris-(hydroxymethyl)-aminomethane, 186 mg of ethylenediamine tetraacetate disodium dihydrate, an appropriate amount of hydrochloric acid, adjusted to give the total volume of 50 ml with an appropriate amount of sterile purified water) diluted to 10 fold for 5 minutes, and was air dried. Then, it was immersed in a solution of a counter staining solution (50 mg of fast green FCF (edible dye, green color No. 3) , adjusted to give the total volume of 50 ml with an appropriate amount of sterile purified water) diluted to 10 fold and then in 1% acetic acid solution. Thereafter, the excess counter staining solution was washed away by immersing again in a solution of the coloring reagent washing solution described above diluted to 10 fold followed by complete air drying.

Example 11

Determination

Determination was conducted by microscopic examination with a light microscope (×1,000), and observation of at least one color development of bluish purple color was determined as positive in cells within a single well stained with the counter staining solution. As a result, bacteria were detected in 5 specimens among 12 specimens by the process according to the present invention. Details of the 5 specimens were specimen A-SA (*Staphylococcus aureus*), specimens F and G-SE (*Staphylococcus epidermidis*), specimens J-SE and EF (*Enterococcus faecalis*), specimens L-SA and CA (*Candida albicans*). When blood culture was conducted using the same specimens according to a known method, SA was detected for the specimen A demonstrating the same result, however, any could not be detected for the specimens F, G, J and L. Therefore, it was revealed that the process of the present invention could achieve rapid detection with favorable sensitivity in comparison with blood culture.

In connection with the results by the specimen A-SA, Fig. 1 illustrates the effects of addition of SDS to the probe dilution solution. It is clear that detection sensitivity of the signal can be markedly elevated by adding 0.25% SDS, as shown in Fig. 1. Also with respect to other specimens, detection of a favorable signal was similarly enabled by adding SDS. The probe used in this Example is a probe produced by nick translation using the base sequences of SA-24 (SEQ ID NO: 1), SA-36 (SEQ ID NO: 2) and SA-77 (SEQ ID NO: 3) in combination.

Example 12

Examination on Optimal Cell Number of Leukocytes to be Smeared and Fixed

Optimal cell number of leukocytes to be smeared on the well of an APS coated slide glass (circular well having the diameter of 5 mm) was examined. Heparinized healthy human blood in an amount of 10 ml was collected, and leukocytes were obtained according to the procedure described in Example 1. Next, thus resulting leukocytes were suspended in an appropriate amount of PBS, and the cell number of the leukocytes per 1 ml was measured using a counting chamber. Starting from (a) $1\times10^8$ cells/ml, a serial dilution of (b) $5\times10^7$ cells/ml, (c) $1\times10^7$ cells/ml, (d) $5\times10^6$ cells/ml, (e) $1\times10^6$ cells/ml, (f) $5\times10^5$ cells/ml and (g) $1\times10^5$ cells/ml was produced, and each 5 μl was smeared on the slide glass. After air drying, Carnoy fixation (see, Example 2) was carried out, and immediately stained with the aforementioned counter staining solution to execute the determination using the process described in Example 11. Consequently, cell number of $1 \times 10^8$ cells/ml was excess, which was inadequate for the detection. Moreover, cell number of $5 \times10^6$ cells/ml or less results in small number of cells observed in the well, which was inadequate for the detection. Therefore, it is preferred that the density of the phagocytes to be immobilized (x cells/ml) is about $5\times10^6$ cells/ml < x cells/ml < about $1\times10^8$ cells/ml, and in particular, about $1\times10^7$ cells/ml x cells/ml about $5\times10^7$ cells/ml. In addition, corresponding thereto, it was revealed that cell number of the leukocytes fixed on the APS coated slide glass per 1 well (y cells/well (diameter of 5 mm)) may be prepared to be about $2.5\times10^4$ cells/well < y cells/well (diameter of 5 mm) < about $5\times10^5$ cells/well, and preferably, about $5 \times 10^4$ cells/well y cells/well (diameter of 5 mm) about $2.5\times10^5$ cells/well. Experimental results for the samples (a) to (f) are shown in Figs. 2 (a) to (f), respectively.

Example 13

Selection of Lytic Enzyme for Use

Conditions for the enzyme to lyse *Staphylococcus aureus* (ATCC 12600), *Staphylococcus epidermidis* (ATCC 14990), *Pseudomonas aeruginosa* (ATCC 10145), *Enterococcus faecalis* (ATCC 19433) and *Escherichia coli* (ATCC 11775) were studied. For *Staphylococcus aureus* and *Staphylococcus epidermidis*, lysostafin was used as the lytic enzyme (Bur. J. Biochem., 38, 293-300, 1973). For *Enterococcus faecalis,* N-acetylmuramidase (Archs. Oral Biol., 23, 543-549, 1978) and lysozyme (Seikagaku Corporation) were used. Further, for *Pseudomonas aeruginosa* and *Es-*

*cherichia coli*, PBS containing 70 mmol/l sodium hydroxide was used. Each type of these bacteria was inoculated in 5ml of BHI (Brain Heart Infusion) liquid medium (manufactured by DIFCO) , and cultured at 37° C for 8 hours or longer. Thus cultured bacterial liquid was collected by centrifugation at 4° C for 10 minutes at 2,000×g. The collected bacteria were suspended in PBS to give a sample.

Lysis was evaluated by decrease in turbidity of the bacterial liquid at the absorbance of 600 nm using a microplate reader. Consequently, *Staphylococcus aureus* and *Staphylococcus epidermidis* were lysed by lysostafin. In respect of *Pseudomonas aeruginosa* and *Escherichia coli*, no enzymatic treatment was required because lysis was conducted with PBS containing 70 mmol/l sodium hydroxide. Furthermore, in connection with *Enterococcus faecalis,* it was proven that more excellent lytic activity could be achieved when lysozyme was used in combination than use of N-acetylmuramidase alone. Moreover, when the bacterium incorporated upon phagocytotic action is for example, *Pseudomonas aeruginosa, Escherichia coli* or the like, bacterial cell wall is lysed during the alkaline treatment to result in the state in which the gene is exposed. Therefore, it is not necessary to conduct this enzymatic treatment. Each enzyme for the pretreatment which is used in lysis of the foreign microorganism according to the present invention is effective not only for the aforementioned bacterial strain, but also for other bacterial strain that includes other genus staphylococcus, genus streptococcus, genus bacillus, genus micrococcus and the like. Additionally, each of such an enzyme can be used alone, but is more effective when used as a mixture. The results are illustrated in Fig. 3, specifically, in regard to: (a) *Staphylococcus aureus* and *Staphylococcus epidermidis*, (b) *Pseudomonas aeruginosa* and *Escherichia coli*, and (c) *Enterococcus faecalis*.

Example 14

Examination on Enzymatic Lysis Solution (Examination on Optimal Concentration of DMSO)

Because protease included in the enzyme reagent deteriorates the morphology of leukocytes, influences of DMSO, which is a solubilizer of PMSF added for the purpose of retaining the morphology of the leukocytes, on enzymatic activity were examined. *Enterococcus faecalis* was inoculated in 50 ml of the aforementioned BHI liquid medium, and cultured at 37°C for 8 hours or longer. This culture liquid was centrifuged at 4° C for 10 minutes at 2,000×g to collect the bacteria, followed by subjecting to a heat treatment in an autoclave (120°C, for 10 minutes) after suspending in PBS. Next, the suspension was centrifuged at 4° C for 10 minutes at 2,000×g, and the supernatant was discarded. Precipitates were suspended in 1 ml of PBS, and thereafter, subjected to freeze-drying. This freeze-dried sample was suspended in 5 mmol/l Tris-hydrochloric acid (pH 6.0), 2 mmol/l magnesium chloride containing 0 to 10% DMSO to give the samples for N-acetylmuramidase. Further, Micrococcus luteus (JCM1464) was inoculated in 5 ml of BHI liquid medium (supra) , and cultured at 37° C for 8 hours or longer. The cultured bacterial liquid was centrifuged at 4° C for 10 minutes at 2,000 ×g to collect the bacteria. After discarding the supernatant, and suspending and washing the bacterial pellet with 5 ml of PBS, centrifugation was conducted once again at 4°C for 10 minutes at 2,000×g to collect the bacteria. Thus collected bacteria were suspended in PBS containing 0 to 10% DMSO to give the samples for lysozyme. On the other hand, *Staphylococcus epidermidis* was cultured and collected similarly to the instance of lysozyme, and suspended in PBS containing 0 to 10% DMSO to give the samples for lysostafin. Enzymatic activity was evaluated on the basis of the decrease in turbidity of the sample at the absorbance of 600 nm using a microplate reader. Each enzyme titer in this test was (a) N-acetylmuramidase: 300 unit/ml, (b) lysozyme: 10,000 unit/ml, (c) lysostafin: 50 unit/ml, and the influences of DMSO on the enzymatic activity were examined. As a result of evaluation of each enzymatic activity judging from the decrease per unit of time in turbidity (O.D. = 600 nm) of the bacteria, DMSO hardly influenced on the N-acetylmuramidase activity. However, in respect of both lysozyme and lysostafin, decrease in activity was found with DMSO at the concentration of 5% or more. Additionally, no decrease in enzymatic activity was found with DMSO at the concentration of 2% or less. Accordingly, the concentration of DMSO for dissolving PMSF may be at least less than 5%, preferably 2% or less, more preferably approximately 1%. The results are show in Fig. 4 (a) to (c) and Table 3 below.

Table 3:

| Influences of DMSO on Enzymatic Activity (Decrease in turbidity of bacteria) | | | |
|---|---|---|---|
| Amount of added DMSO (%) | N-acetylmuramidase O. D./5 minutes | lysozyme O.D./3 minutes | Lysostafin O.D./10 minutes |
| 0 (control) | 79.3±4.8 | 0.689±0.028 | 0.168±0.017 |
| 0.1 | 75.0±3.2 | 0.678±0.026 | 0.164±0.009 |
| 1 | 75.8±2.8 | 0.660±0.026 | 0.160±0.008 |

Table 3: (continued)

| Influences of DMSO on Enzymatic Activity (Decrease in turbidity of bacteria) | | | |
|---|---|---|---|
| Amount of added DMSO (%) | N-acetylmuramidase O. D./5 minutes | lysozyme O.D./3 minutes | Lysostafin O.D./10 minutes |
| 2 | 75.8±2.5 | 0.653±0.024 | 0.145±0.009 |
| 5 | 76.3±4.9 | 0.566±0.017 | 0.124±0.006 |
| 10 | 73.8±3.5 | 0.464±0.016 | 0.094±0.006 |

Example 15

Examination on Enzymatic Lysis Solution (Examination on Optimal Concentration of PMSF)

Because protease included in the enzyme reagent deteriorates the morphology of leukocytes, effects of PMSF (manufactured by PIERCE), which is added for the purpose of retaining the morphology of the leukocytes, on enzymatic activity were examined. PMSF was dissolved in 100 µl of DMSO (manufactured by Wako Pure Chemical Industries, Ltd), and diluted to 10 ml with PBS such that the final concentration of PMSF becomes none (0 mmol/l) to 1 mmol/l. To this solution was added proteinase K (manufactured by Boeringer Mannheim) such that titer of the protease becomes 0.2 unit/ml. Heparinized healthy human blood in an amount of 5 ml was collected, and leukocytes were obtained according to the process described in Example 1. Next, the leukocytes were suspended in an appropriate amount of PBS, and the cell number was measured using a counting chamber. Cell number was adjusted to about $5\times10^4$ cells/well to about $2.5\times10^5$ cells/well, and 5 µl therefrom was smeared on the well of the APS coated slide glass. After air drying, fixation was executed according to the method of Carnoy fixation described in Example 2. Using this sample, tests were performed according to the process described in Examples 3 to 11. As a consequence of performing the tests at the concentration of PMSF of 1 µmol/l to 1 mmol/l, effects were found at the concentration of 10 µmol/l or greater, while deterioration of morphology of the leukocytes was completely suppressed at the concentration of PMSF of 0.1 mmol/l or greater. The results are shown in Fig. 5, for (a): protease 0.2 unit/ml alone, (b): 1 µmol/ml PMSF added, (c) : 10 µmol/ml PMSF added, (d): 0.1 mmol/ml PMSF added, and (e): 1 mmol/ml PMSF added, respectively.

Example 16

Examination of Optimal Titer of Lytic Enzyme, Zymolase

Optimal titer of zymolase for exposing DNA was examined through lysis of Candida albicans. *Candida albicans* was inoculated in YPD medium, and cultured over day and night at 30°C. Then, two types of the solutions were prepared: a solution of *Candida albicans* as a substrate suspended in PBS (substrate 1); and a solution prepared by Carnoy s fixation, immersing in 70% ethanol, air drying and suspension in PBS (substrate 2). Upon the reaction, a mixture of zymolase/ PBS: 0.5ml, substrate: 1.5 ml, M/15 phosphate buffer: 2.5 ml and sterile purified water: 0.5 ml, adjusted to give the total volume of 5.0 ml was used.

Thereafter, the reaction was allowed at 37° C for 2 hours, and the $OD_{800}$ was measured. Furthermore, the concentration of zymolase (Zymolyase-100T) for use was 0 mg/ml, 0.01 mg/ml, 0. 025 mg/ml, 0.05 mg/ml, 0.1 mg/ml, 0.25 mg/ml, 0.5 mg/ml, 1 mg/ml, 2.5 mg/ml and 5 mg/ml. Consequently, each $OD_{800}$ value when the substrate 1 was used was 0.533, 0.521, 0.553, 0.554, 0.548, 0.417, 0.394, 0.288, 0.163 and 0.113, and each $OD_{800}$ value when the substrate 2 was used was 0.445, 0.411, 0.359, 0.282, 0.232, 0.146, 0.115, 0.096, 0.08 and 0.057. It was proven that effectiveness was brought when both of the substrate 1 and substrate 2 were in the range of 0.5 mg/ml to 5 mg/ml, and particularly 1 mg/ml to 5 mg/ml. That is, the amount of zymolase to be used is preferably 50 unit/ml to 500 unit/ml, particularly 100 unit/ml to 500 unit/ml.

Example 17

Examination of Optimal Condition (Titer) of Enzymatic Treatment

(1) Production of Digested sample

[1] Preparation of U937 cell

U937 cells (monocyte established cell line: ATCC CRL-1593.2) were cultured in an RPMI 1640 medium (25 ml) within a cell culture flask (175 $cm^2$) in a 5% carbon dioxide gas incubator at 37°C. Next, the U937 cell culture liquid was placed in a 50 ml centrifuge tube, and centrifuged at 4°C for 10 minutes at 220 × g to recover the U937 cells. Then, thus recovered U937 cells were suspended in 200 μl of PBS, and the cell number was counted with a counting chamber. The cell number was adjusted to $1\times10^4$ cells/μl to $2\times10^4$ cells/μl.

[2] Preparation of Bacterial Digested sample

*Staphylococcus aureus* (ATCC 12600), *Staphylococcus epidermidis* (ATCC 14990), *Pseudomonas aeruginosa* (ATCC 10145), *Enterococcus faecalis* (ATCC 19433) and *Escherichia coli* (ATCC 11775) were inoculated in each 5 ml of BHI culture medium, and cultured at 37°C for 8 hours or longer. The cultured bacterial liquid was centrifuged at 4° C for 10 minutes at 2,000×g to collect the bacteria. After discarding the supernatant, the bacterial pellet was suspended in 5 ml of PBS, and centrifugation was conducted once again at 4°C for 10 minutes at 2,000×g to collect the bacteria. Thus collected bacteria were suspended in 5 ml of PBS and thereafter, 15 ml of bacterial liquids was produced prepared by diluting in PBS to give the turbidity (O.D. = 600 nm) of the bacterial liquid, which was measured with a absorbance meter, of 0.01 to 0.03 for *Staphylococcus aureus,* 0.01 to 0.03 for *Staphylococcus epidermidis,* 0.02 to 0.03 for *Pseudomonas aeruginosa,* 0.01 to 0.03 for *Enterococcus faecalis,* 0.02 to 0.03 for *Escherichia coli*, respectively. Thus produced bacterial liquid was transferred into a separate 175 $cm^2$ flask for culture, and left to stand still at room temperature for 30 minutes. Fifty ml of heparinized healthy human blood was collected, and thereto was added the reagent for separating hemocyte at a ratio of 4 : 1, and left to stand still at 37°C for 30 minutes to yield the leukocyte fraction. Thus obtained leukocyte fraction was adjusted to 50 ml with PBS. The supernatant in the culture flask (*supra*) was gently discarded, and each 10 ml of the leukocyte fraction diluted in PBS was added to the flask followed by leaving to stand still at room temperature for 10 minutes. The supernatant in the flask for culture was discarded, and the leukocytes attached to the bottom of the flask were recovered in a 15 ml centrifuge tube with 10 ml of PBS containing 0.02% EDTA, and centrifuged at 4°C for 10 minutes at 140×g to 180×g to collect the leukocytes. Because contamination of erythrocytes was found in the collected leukocytes, precipitates of the leukocytes were gently suspended in 1 ml of sterile purified water to allow hemolysis, subjected to isotonization through adding 14 ml of PBS, followed by centrifugation once again at 4°C for 10 minutes at 140 × g to 180×g to collect the leukocytes. The collected leukocytes were suspended in PBS, and cell number was counted with a counting chamber to adjust to give $1\times10^4$ cells/μl to $5\times10^4$ cells/μl. These digested samples were referred to as SA digested sample, SE digested sample, PA digested sample, EF digested sample and EK digested sample.

[3] Smear and Fixation

Each 5 μl of U937 cells prepared in Example 17 (1) [1] and each 5 μl of each bacterial digested sample produced in Example 17 (1) [2] were smeared on each well of the APS coated slide glass, and air dried. Next, after immersing the slide glass in the Carnoy s fixative described in Example 2 for 20 minutes, it was immersed in 75% ethanol for 5 minutes. After washing Carnoy' s fixativeand air drying, the slide glass was stored at 4° C until use in the test (see, Example 2). Next, pretreatment of the fixed sample was carried out according to Example 3.

(2) Standard and Process for Testing Digested sample

[1] Cell Number

**[0135]** Cell number to be smeared and fixed on the slide glass of each bacterial digested sample was $5.0\times10^4$ to $2.5\times10^5$ cells/well, whilst cell number of U937 cells was $5.0\times10^4$ to $1.0\times10^5$ cells/well.

[2] Phagocytosis Rate

The bacterial digested sample smeared and fixed on the slide glass was stained with an acridine orange staining solution, and about 200 cells were randomly counted with a fluorescence microscope ( × 1,000). Among the measured cells, cells including bacteria phagocytized within the cells (cells with any change characteristicic in phagocytosis found in morphology, as shown by arrows in Fig. 6) were determined as positive cells, and the phagocytosis rate (%) was calculated according to the mathematical formula below.

$$\text{Phagocytosis rate (\%)} = [(\text{Positive cell number}/\text{Measured cell number}) \times 100]$$

Thus calculated phagocytosis rate (%) of each bacterial digested sample was 10% or greater.

[3] Test Process

The digested sample produced in Example 17 (2) [1] and [2] was employed as a specimen. The SA digested sample used had the phagocytosis rate of 23% with $1.98 \times 10^5$ cells/well. The SE digested sample had the phagocytosis rate of 27% with $1.74 \times 10^5$ cells/well. Moreover, the EF digested sample had the phagocytosis rate of 34% with $6.40 \times 10^4$ cells/well.

Using the slide glass having each digested sample smeared thereon, the enzymatic pretreatment was performed according to the process described in Example 3. Next, the slide glass after completing the enzymatic pretreatment was placed in a humid box, and the reaction was allowed by dropping 1 ml of each enzyme solution prepared to give each titer on the smeared site of the specimen. Thereafter, the slide glass was immersed in PBS containing 0.2 mol/l hydrochloric acid, and in 70% ethanol respectively, for 10 minutes, and air dried. After immersing this slide glass in PBS containing 70 mmol/l sodium hydroxide for 3 minutes, and in 70% ethanol for 10 minutes, it was air dried and stained with 1% acridine orange staining solution. Then, evaluation was made with a fluorescence microscope (× 1,000). For *Staphylococcus aureus* and *Staphylococcus epidermidis*, examination of the optimal titer was conducted with lysostafin. In order to examine the optimal titer when N-acetylmuramidase and lysozyme are used in combination for *Enterococcus faecalis,* examination on optimal titer of lysozyme was conducted in cases where N-acetylmuramidase was fixed at 100 unit/ml, and on optimal titer of N-acetylmuramidase in cases where lysozyme was fixed at 10,000 unit/ml. The determination was made as adequate when no bacterial body was identified in the leukocytes by the enzymatic treatment.

[4] Results

For the lysis of *Staphylococcus aureus,* as described in Table 4, sufficient effects were exerted at the titer of lysostafin of 1 unit/ml, however, upon lysis of *Staphylococcus epidermidis*, the titer of lysostafin of 10 unit/ml or greater was necessary. Therefore, the optimal titer of lysostafin was set to be 10 unit/ml to 100 unit/ml. In addition, for the lysis of *Enterococcus faecalis,* lysis did not occur with the titer of N-acetylmuramidase of 10 unit/ml or less when the titer of lysozyme was fixed at 10, 000 unit/ml. In respect of lysozyme, when the titer of N-acetylmuramidase was fixed at 100 unit/ml, lysis did not occur with the titer of lysozyme of 1,000 unit/ml or less, as described in Table 5. Therefore, the optimal titer of N-acetylmuramidase was set to be 100 unit/ml to 1,000 unit/ml, whilst the optimal titer of lysozyme was set to be 10,000 unit/ml to 100,000 unit/ml. The results are shown in Fig. 7. In the Figure, depicted are states of: (a) the digested sample of *Staphylococcus aureus* prior to the enzymatic treatment, (b) the digested sample of *Enterococcus faecalis* prior to the enzymatic treatment, (c) the sample (a) following the enzymatic treatment, and (d) the sample (b) following the enzymatic treatment.

Table 4: Optimal Titer for Enzymatic Treatment of Lysostafin on *S. Aureus*, *S. epidermidis*

| Lysostafin Titer (U/mL) / Digested Samples | | 0 | 0.1 | 1 | 10 | 100 | 1,000 |
|---|---|---|---|---|---|---|---|
| SA Digested Sample | once | inadequate | Inadequate | adequate | adequate | adequate | adequate |
| | twice | inadequate | inadequate | adequate | adequate | adequate | adequate |
| | thrice | inadequate | inadequate | adequate | adequate | adequate | adequate |
| SE Digested Sample | once | inadequate | inadequate | inadequate | adequate | adequate | adequate |
| | twice | inadequate | inadequate | inadequate | adequate | adequate | adequate |
| | thrice | inadequate | inadequate | inadequate | adequate | adequate | adequate |

Table 5: Optimal Titer of Enzymatic Treatment of N-acetylmuramidase and lysozyme on *E. faecalis*

| N-acetyl muramidase titer (U/mL) / Digested Sample | | 0 | 1 | 10 | 100 | 1,000 | 10,000 |
|---|---|---|---|---|---|---|---|
| EF Digested Sample | once | Inadequate | inadequate | inadequate | adequate | adequate | adequate |
| | twice | Inadequate | inadequate | inadequate | adequate | adequate | adequate |
| | thrice | Inadequate | inadequate | inadequate | adequate | adequate | adequate |
| Lysozyme titer (U/mL) / Digested Sample | | 0 | 10 | 100 | 1,000 | 10,000 | 100,000 |
| EF Digested Sample | once | Inadequate | inadequate | inadequate | inadequate | adequate | adequate |
| | twice | Inadequate | inadequate | inadequate | inadequate | adequate | adequate |
| | thrice | Inadequate | inadequate | inadequate | inadequate | adequate | adequate |

Applications of these results obtained using the digested samples to the present invention could result in similar results. Therefore, the optimal titer of each enzyme as described above in the identification of a causative microorganism of an infectious disease in the clinical specimen of the present invention was also set similarly.

Example 18

Examination on Optimal Condition of Enzymatic Treatment (Temperature)

Using a slide glass including each digested sample smeared thereon, examination was conducted according to the process described in example 17 (2) [3]. Time period of the enzymatic treatment in this test was 30 minutes, and the temperature for examination was 4°C, 25° C, 37° C, 42° C, and 60° C. Moreover, titer of each enzyme was N-acetylmuramidase (100 unit/ml, manufactured by Seikagaku Corporation), lysozyme (10,000 unit/ml, manufactured by Seikagaku Corporation), lysostafin (10 unit/ml, manufactured by SIGMA).

Determination was conducted according to the process described in example 17 (2) [3]. As a consequence, for *Staphylococcus aureus,* no bacterial body was found in the leukocytes in the range of temperature of 4°C to 60°C. For *Staphylococcus epidermidis,* although bacterial bodies remained in the leukocytes at the temperature of 4° C and 25° C, no bacterial body was found at 37°C or higher. Further, for *Enterococcus faecalis*, although bacterial bodies remained at the temperature of treatment of 4° C, 25 ° C and 60° C, no bacterial body was found at 37°C and 42°C. Hence, the optimal temperature for the enzymatic treatment was set to be 37° C to 42° C. The results are shown in Table 6.

Table 6: Optimal Temperature for Treatment of Enzyme Reagent

| Digested Samples \ Temperature for Treatment (℃) | | 4 | 25 | 37 | 42 | 60 |
|---|---|---|---|---|---|---|
| SA Digested Sample | Once | adequate | adequate | adequate | adequate | adequate |
| | twice | adequate | adequate | adequate | adequate | adequate |
| | thrice | adequate | adequate | adequate | adequate | adequate |
| SE Digested Sample | once | inadequate | inadequate | adequate | adequate | adequate |
| | twice | inadequate | inadequate | adequate | adequate | adequate |
| | thrice | inadequate | inadequate | adequate | adequate | adequate |
| EF Digested Sample | once | inadequate | inadequate | adequate | adequate | inadequate |
| | twice | inadequate | inadequate | adequate | adequate | inadequate |
| | thrice | inadequate | inadequate | adequate | adequate | inadequate |

Applications of these results obtained using the digested samples to the present invention could result in similar results. Therefore, the optimal temperature of the enzymatic treatment in the identification of a causative microorganism of an infectious disease in the clinical specimen of the present invention was also set similarly.

Example 19

Examination on Optimal Condition of Enzymatic Treatment (Time)

Digested samples produced according to the process described in Example 17 (1) [1] and [2] were used as specimens. Time period of the examination was 0 minute, 10 minutes, 20 minutes, 30 minutes, 60 minutes and 120 minutes. Phagocytosis rate of the used SA digested sample was 18% with $7.80 \times 10^4$ cells/well. Phagocytosis rate of the used SE digested sample was 34% with $1.10 \times 10^5$ cells/well. Further, phagocytosis rate of the EF digested sample was 28% with $1.30 \times 10^5$ cells/well.

Using the slide glass including each digested sample smeared thereon, examination was conducted according to the process described in example 17 (2) [3]. Temperature for the enzymatic treatment in this test was 37°C, and titer of each enzyme was 100 unit/ml for N-acetylmuramidase, 10,000 unit/ml for lysozyme, 10 unit/ml for lysostafin. Determination was conducted according to the process described in example 17 (2) [3]. As a consequence, for all of *Staphylococcus aureus, Staphylococcus epidermidis* and *Enterococcus faecalis* digested samples, no bacterial body was found in the leukocytes with the time period of the enzymatic treatment of 20 minutes or longer (inadequate at 0 minute and 10 minutes). Therefore, the optimal time period of the enzymatic treatment is at least 15 minutes or longer, preferably 20 minutes or longer, and still preferably 30 minutes to 60 minutes. The results are shown in Table 7.

Table 7: Optimal Time Period of Treatment of Enzyme Reagent

| Digested Samples \ Time of enzyme-treatment (minutes) | | 0 | 10 | 20 | 30 | 60 | 120 |
|---|---|---|---|---|---|---|---|
| SA Digested Sample | once | inadequate | inadequate | adequate | adequate | adequate | adequate |
| | twice | inadequate | inadequate | adequate | adequate | adequate | adequate |
| | thrice | inadequate | inadequate | adequate | adequate | adequate | adequate |
| SE Digested Sample | once | inadequate | inadequate | adequate | adequate | adequate | adequate |
| | twice | inadequate | inadequate | adequate | adequate | adequate | adequate |
| | thrice | inadequate | inadequate | adequate | adequate | adequate | adequate |
| EF Digested Sample | once | inadequate | inadequate | adequate | adequate | adequate | adequate |
| | twice | inadequate | inadequate | adequate | adequate | adequate | adequate |
| | thrice | inadequate | inadequate | adequate | adequate | adequate | adequate |

Applications of these results obtained using the digested samples to the present invention could result in similar results. Therefore, the optimal time period of the enzymatic treatment in the identification of a causative microorganism of an infectious disease in the clinical specimen of the present invention was also set similarly.

Example 20

Examination on Optimal Condition of Enzymatic Treatment (Time)

In *in situ* hybridization reaction according to the present invention, concentration of the probe is an important factor which affects the hybridizing velocity. When the probe concentration is too low, the reaction velocity may be lowered, leading to the possibility of unclear signal. Furthermore, use of an excess amount of probe may result in grounds for nonspecific reaction.

Thus, optimal concentration was examined in connection with various probe solutions. First, the digested samples produced according to the process described in Example 17(1) [1] and [2] were used as specimens. The phagocytosis rate of the used SA digested sample was 24% with $1.48\times10^5$ cells/well. The phagocytosis rate of the SE digested sample was 28% with $2.07 \times 10^5$ cells/well. The phagocytosis rate of the PA digested sample was 11% with $1.59\times10^5$ cells/well. In addition, the phagocytosis rate of the EF digested sample was 24% with $1.72 \times10^5$ cells/well. The phagocytosis rate of the EK digested sample was 12% with $1.63\times10^5$ cells/well. Using the slide glass including each digested sample smeared thereon, examination was conducted according to the process described in Example 17(2) [3]. The probes for use were labelled with digoxigenin, and the concentration of each probe for *Staphylococcus aureus, Staphylococcus epidermidis, Enterococcus faecalis, Pseudomonas aeruginosa* and *Escherichia coli* was adjusted to 0.06 ng/µl, 0.6 ng/µl, 1.2 ng/µl, 1.8 ng/µl, 2.4 ng/µl, 3 ng/µl, respectively. The probe solution prepared to each concentration described above was used on the slide glass including the digested sample smeared thereon (see, Fig. 8), and examined according to the process described in Examples 3-11.

Consequently, the signal became unclear at lower concentration (0.06 ng/µl), and on the other hand, increase in background was observed at higher concentration (2.4 ng/µl and 3 ng/µl). Therefore, the concentrations of probes of SA, SE, PA, EF and EK were determined to be 0.6 to 1.8 ng/µl, preferably 0.6 to 1.2 ng/µl. Moreover, since an inadequate result was yielded at 0.06 ng/µl, while an adequate result was yielded at 0. 6 ng/µl, it is preferably determined to be 0.1 ng/µl or greater.

Furthermore, since an inadequate result was yielded at 2.4 ng/µl, and an adequate result was yielded at 1.8 ng/µl, it is preferably determined to be 2.2 ng/µl or less. The results are shown in Tables 8-12 below.

Table 8:

| SA probe | | | | | | |
|---|---|---|---|---|---|---|
| Digested sample | Probe concentration (ng/µL) | | | | | |
| | 0.06 | 0.6 | 1.2 | 1.8 | 2.4 | 3 |
| SA digested sample | - | + | + | + | + | + |
| SE digested sample | - | - | - | - | + | + |
| PA digested sample | - | - | - | - | + | + |
| EF digested sample | - | - | - | - | + | + |
| EK digested sample | - | - | - | - | + | + |

Table 9:

| SE probe | | | | | | |
|---|---|---|---|---|---|---|
| | Probe concentration (ng/µL) | | | | | |
| Digested sample | 0.06 | 0.6 | 1.2 | 1.8 | 2.4 | 3 |
| SA digested sample | - | - | - | - | - | + |
| SE digested sample | - | + | + | + | + | + |
| PA digested sample | - | - | - | - | - | + |
| EF digested sample | - | - | - | - | - | + |
| EK digested sample | - | - | - | - | - | + |

Table 10:

| PA probe | | | | | | |
|---|---|---|---|---|---|---|
| Digested sample | Probe concentration (ng/µL) | | | | | |
| | 0.06 | 0.6 | 1.2 | 1.8 | 2.4 | 3 |
| SA digested sample | - | - | - | - | - | - |
| SE digested sample | - | - | - | - | + | + |
| PA digested sample | - | + | + | + | + | + |
| EF digested sample | - | - | - | - | - | + |
| EK digested sample | - | - | - | - | - | + |

Table 11:

| EF probe | | | | | | |
|---|---|---|---|---|---|---|
| Digested sample | Probe concentration (ng/μL) | | | | | |
| | 0.06 | 0.6 | 1.2 | 1.8 | 2.4 | 3 |
| SA digested sample | - | - | - | - | - | + |
| SE digested sample | - | - | - | - | + | + |
| PA digested sample | - | - | - | - | + | + |
| EF digested sample | - | + | + | + | + | + |
| EK digested sample | - | - | - | - | - | - |

Table 12:

| EK probe | | | | | | |
|---|---|---|---|---|---|---|
| Digested sample | Probe concentration (ng/μL) | | | | | |
| | 0.06 | 0.6 | 1.2 | 1.8 | 2.4 | 3 |
| SA digested sample | - | - | - | - | + | + |
| SE digested sample | - | - | - | - | + | + |
| PA digested sample | - | - | - | - | + | + |
| EF digested sample | - | - | - | - | + | + |
| EK digested sample | - | + | + | + | + | + |

Applications of these results obtained using the digested samples to the present invention could result in similar results. Therefore, the optimal concentration of each probe described above in the identification of a causative microorganism of an infectious disease in the clinical specimen of the present invention was also set similarly.

Example 21

Examination on Hybridization Temperature

Reaction temperature in the hybridization reaction is a parameter which affects the hybridizing velocity and stability of the hybrid. Because high temperature of the hybridization reaction is known to deteriorate the cell morphology, examination of the optimal temperature (4°C, 25°C, 37° C, 42° C, 50°C and 60°C) was performed.

First, the digested samples produced according to the process described in Example 17(1) [1] and [2] were used as specimens. The phagocytosis rate of the used SA digested sample was 31% with $1.38\times10^5$ cells/well. The phagocytosis rate of the SE digested sample was 42% with $1.95\times10^5$ cells/well. The phagocytosis rate of the PA digested sample was 14% with $1.27\times10^5$ cells/well. In addition, the phagocytosis rate of the EF digested sample was 48% with $1.05\times10^5$ cells/well. The phagocytosis rate of the EK digested sample was 17% with $1.85\times10^5$ cells/well.

Using the slide glass including the digested sample and U937 cells smeared and fixed thereon (see, Fig. 9), examination was conducted according to the process described in Examples 3-11. Consequently, no stable signal was observed for each type of probe at the hybridization temperature of 4°C or less owing to the lowered hybridization velocity. Further, at 60°C, changes in cell morphology were detected, and thus no stable signal was observed. In addition, at 25°C and 50°C, detection could be executed better compared to at the temperature of 37°C and 42°C, although the signal was unclear. Hence; optimal temperature of the hybridization may be 25°C to 50°C, more preferably 37 to 42°C. The results are shown in Tables 13-17 below.

Table 13:

| SA probe | | | | | | |
|---|---|---|---|---|---|---|
| Digested sample | Hybridization temperature (°C) | | | | | |
| | 4 | 25 | 37 | 42 | 50 | 60 |
| SA digested sample | - | + | + | + | + | + |
| SE digested sample | - | - | - | - | - | - |
| PA digested sample | - | - | - | - | - | - |
| EF digested sample | - | - | - | - | - | - |
| EK digested sample | - | - | - | - | - | - |

Table 14:

| SE probe | | | | | | |
|---|---|---|---|---|---|---|
| Digested sample | Hybridization temperature (°C) | | | | | |
| | 4 | 25 | 37 | 42 | 50 | 60 |
| SA digested sample | - | - | - | - | - | - |
| SE digested sample | + | + | + | + | + | - |
| PA digested sample | - | - | - | - | - | - |
| EF digested sample | - | - | - | - | - | - |
| EK digested sample | - | - | - | - | - | - |

Table 15:

| PA probe | | | | | | |
|---|---|---|---|---|---|---|
| Digested sample | Hybridization temperature (°C) | | | | | |
| | 4 | 25 | 37 | 42 | 50 | 60 |
| SA digested sample | - | - | - | - | - | - |
| SE digested sample | - | - | - | - | - | - |
| PA digested sample | - | + | + | + | + | - |
| EF digested sample | - | - | - | - | - | - |
| EK digested sample | - | - | - | - | - | - |

Table 16:

| EF probe | | | | | | |
|---|---|---|---|---|---|---|
| Digested sample | Hybridization temperature (°C) | | | | | |
| | 4 | 25 | 37 | 42 | 50 | 60 |
| SA digested sample | - | - | - | - | - | - |
| SE digested sample | - | - | - | - | - | - |
| PA digested sample | - | - | - | - | - | - |
| EF digested sample | + | + | + | + | + | - |
| EK digested sample | - | - | - | - | - | - |

Table 17:

| EK probe | | | | | | |
|---|---|---|---|---|---|---|
| Digested sample | Hybridization temperature (°C) | | | | | |
| | 4 | 25 | 37 | 42 | 50 | 60 |
| SA digested sample | - | - | - | - | - | - |
| SE digested sample | - | - | - | - | - | - |
| PA digested sample | - | - | - | - | - | - |
| EF digested sample | - | - | - | - | - | - |
| EK digested sample | - | + | + | + | + | - |

Applications of these results obtained using the digested samples to the present invention could result in similar results. Therefore, the optimal temperature of hybridization in the identification of a causative microorganism of an infectious disease in the clinical specimen of the present invention was also set similarly.

Example 22

Examination on Hybridization Time Period

The digested samples produced according to the process described in Example 17(1) [1] and [2] were used as specimens, and examination was conducted on the time period of hybridization of 10 minutes, 60 minutes, 90 minutes, 120 minutes, 180 minutes and 900 minutes. The phagocytosis rate of the used SA digested sample was 47% with $1.45\times10^5$ cells/well. The phagocytosis rate of the SE digested sample was 47% with $1.33\times10^5$ cells/well. The phagocytosis rate of the PA digested sample was 15% with $1.91\times10^5$ cells/well. In addition, the phagocytosis rate of the EF digested sample was 41% with $1.45\times10^5$ cells/well. The phagocytosis rate of the EK digested sample was 20% with $1.23\times10^5$ cells/well.

Using the slide glass including the digested sample and U937 cells smeared and fixed thereon (same as one shown in Fig. 9), examination was conducted according to the process described in Examples 3-11. Consequently, although no signal was observed with the time period of hybridization of 10 minutes, a signal was observed at 60 minutes or greater, and a stable signal was observed at 90 minutes or greater. Further, no alteration in detection of the signal was observed also with the time period of hybridization of 900 minutes. Therefore, it is preferred that the time period is at least 30 minutes or greater, preferably 60 minutes or greater, and more preferably 90 minutes or greater. More preferred optimal time period of hybridization may be set to be 120 minutes to 900 minutes. The results are shown in Tables 18-22 below.

Table 18: e

| SA prob | | | | | | |
|---|---|---|---|---|---|---|
| Digested sample | Hybridization time (minutes) | | | | | |
| | 10 | 60 | 90 | 120 | 180 | 900 |
| SA digested sample | - | + | + | + | + | + |
| SE digested sample | - | - | - | - | - | - |
| PA digested sample | - | - | - | - | - | - |
| EF digested sample | - | - | - | - | - | - |
| EK digested sample | - | - | - | - | - | - |

Table 19:

| SE probe | | | | | | |
|---|---|---|---|---|---|---|
| Digested sample | Hybridization time (minutes) | | | | | |
| | 10 | 60 | 90 | 120 | 180 | 900 |
| SA digested sample | - | - | - | - | - | - |
| SE digested sample | + | + | + | + | + | + |
| PA digested sample | - | - | - | - | - | - |
| EF digested sample | - | - | - | - | - | - |
| EK digested sample | - | - | - | - | - | - |

Table 20:

| SE probe | | | | | | |
|---|---|---|---|---|---|---|
| Digested sample | Hybridization time (minutes) | | | | | |
| | 10 | 60 | 90 | 120 | 180 | 900 |
| SA digested sample | - | - | - | - | - | - |
| SE digested sample | - | - | - | - | - | - |
| PA digested sample | - | + | + | + | + | + |
| EF digested sample | - | - | - | - | - | - |
| EK digested sample | - | - | - | - | - | - |

Table 21:

| EF probe | | | | | | |
|---|---|---|---|---|---|---|
| Digested sample | Hybridization time (minutes) | | | | | |
| | 10 | 60 | 90 | 120 | 180 | 900 |
| SA digested sample | - | - | - | - | - | - |
| SE digested sample | - | - | - | - | - | - |
| PA digested sample | - | - | - | - | - | - |
| EF digested sample | + | + | + | + | + | + |
| EK digested sample | - | - | - | - | - | - |

Table 22:

| EK probe | | | | | | |
|---|---|---|---|---|---|---|
| Digested sample | Hybridization time (minutes) | | | | | |
| | 10 | 60 | 90 | 120 | 180 | 900 |
| SA digested sample | - | - | - | - | - | - |
| SE digested sample | - | - | - | - | - | - |
| PA digested sample | - | - | - | - | - | - |
| EF digested sample | - | - | - | - | - | - |
| EK digested sample | - | + | + | + | + | + |

Applications of these results obtained using the digested samples to the present invention could result in similar results. Therefore, the optimal time period of hybridization in the identification of a causative microorganism of an infectious disease in the clinical specimen of the present invention was also set similarly.

Example 23

Influence of Surfactant Added to Hybridization Solution

The digested samples produced according to the process described in Example 17(1) [1] and [2] were used as specimens. When any of various surfactants (SDS, lauryl sarcosine, saponin, BRIJ35, Tween 20, Triton X-100) was added to the probe dilution solution followed by hybridization carried out according to Example 7, the detection sensitivity was dramatically enhanced by adding 0.25% SDS. In addition, the detection sensitivity could be improved by lauryl sarcosine, BRIJ 35 or Tween 20. The results are shown in Table 23 below.

Table 23

| Surfactant | Signal detection |
|---|---|
| None added | + |
| SDS | + + + |
| Lauryl sarcosine | + + |
| Saponin | + |
| BRIJ 35 | + + |
| Tween 20 | + + |
| Triton X-100 | + |

Furthermore, as a consequence of using SDS at various concentrations, it was revealed that preferable concentration was 1% or less, more preferably 0.1% to 0.5%, and still more preferably 0.25%.
Applications of these results obtained using the digested samples to the present invention could result in similar results. Therefore, also in the present invention, it is preferred that a surfactant, particularly SDS, is added at the step of *in situ* hybridization.

Example 24

Examination on Chain Length of Probe Used in Hybridization

*Staphylococcus aureus* probe (SA-24 (SEQ ID NO: 1)) and *Pseudomonas aeruginosa* probe (P2-2 (SEQ ID NO: 7)) were labelled with digoxigenin.
First, 1 μg of purified each type of the DNA probe was prepared to give the total volume of 50 μg with 5 μl of 10× L.B. (0.5 mol/l Tris-hydrochloric acid (pH 7.5), 50 mmol/l magnesium chloride, 5 μl of 0.5 mg bovine serum albumin), 5 μl of 100 mmol/l dithiothreitol, each 1 nmol of dNTPs (A, G, C), 0.5 nmol of digoxigenin-dUTP (Dig-dUTP), each 0.5 nmol of dTTP, 3 μl of DNase (amount corresponding to 25 mU, 75 mU and 200 mU) , 1 μl of 10 U/μl DNA polymerase and an appropriate amount of sterile purified water. Digoxigenin labelling was performed at 15°C for 2 hours. After the labelling, the mixture was boiled for 5 minutes to terminate the reaction. The reaction liquid after the termination was injected into a spin column (CENTRI-SEP COLUMUNS CS901, PRINCETON SEPARATIONS, INC.), and centrifuged at 25°C for 2 minutes (3,000×g) to remove free nucleotides. Then, concentration of the eluate was measured by an absorbance meter. Electrophoresis was performed on a 3% agarose gel to confirm the size.
Next, DNA in the agarose gel was transferred to a nitrocellulose membrane by Southern blotting method. Then, the membrane was immersed in 2% blocking reagent (manufactured by Roche) for 30 minutes, and thereafter, alkaline phosphatase labelled anti-digoxigenin antibody in an amount of 1/5,000 was added thereto and the immersion was allowed for 30 minutes. Next, the membrane was washed twice by shaking in 100 mmol/l Tris-hydrochloric acid (pH 7.5) and 150 mmol/l sodium chloride for 10 minutes. Thereafter, washing was executed by shaking in 100 mmol/l Tris-hydrochloric acid (pH9.5) and 150 mmol/l sodium chloride for 10 minutes. Then, color development was conducted by immersing in an NBT/BCIP solution.
Finally, the membrane was immersed in purified water to stop the color development, and dried. Consequently, as shown in Fig. 10 for (a) use of the SA probe and (b) use of the PA probe, respectively, it was indicates that in cases where cleavage was conducted using 25 mU of DNase (in Figure, lane 1) such that the chain length distributes the base length of predominantly about 350 to about 600, high labelling efficiency was achieved. When thus resulting probe for detection was used in the process for detecting a causative microorganism of an infectious disease according to the present invention in which a digested sample or a clinical specimen from a patient suffering from an infectious disease was used to carry out hybridization, a signal could be detected with excellent sensitivity. Therefore, it was reveled that chain length of the probe used in the hybridization may be the base length of about 350 to about 600, and preferably the base length of about 350 to about 550.

Example 25

Examination on Probe used in Hybridization

*Escherichia coli* digested samples produced according to the process described in Example 17(1) [1] and [2] were used as specimens to examine on the probes for detection.

Probes for detection were prepared through labelling with digoxigenin as described in Example 24 from EC-24 (SEQ ID NO: 11), EC-34 (SEQ ID NO: 12) and EC-39 (SEQ ID NO: 13) such that they have the base length of about 350 to about 600, and used alone or in combination of those three, respectively. From thus obtained results, it was evident that the signal could be detected more clearly resulting in elevated sensitivity for (d) the mixed probe MIX prepared by mixing the three ((a) EC-24, (b) EC-34 and (c) EC-39), than for each (a) EC-24, (b) EC-34 or (c) EC-39 used alone, as shown in Fig. 11.

Example 26

Comparison of Detection Capability for Various Amount of Bacteria Between Blood Culture Process and Process for Detecting Foreign Microorganisms Wherein Digested Sample of Present Invention is Used

*S. aureus, S. epidermidis* or *Enterococcus faecalis* described in Example 13 was admixed with healthy human blood at the concentration of $10^5$, $10^4$, $10^3$, $10^2$, $10^1$ or $10^0$ CFU/ml. After incubating the mixture, tests were performed with a kit for identifying a causative microorganism of an infectious disease of a clinical specimen (Hybrizep [trade name: FUSO PHARMACEUTICAL INDUSTRIES, LTD.]), and by a blood culture process according to any known process. Furthermore, after bringing each bacterium contact with piperacillin (PIPC) having a broad spectrum at a concentration of 10 MIC, each bacterium was admixed with healthy human blood at the concentration of $10^4$, $10^3$, $10^2$, $10^1$ or $10^0$ CFU/ml, and the tests were similarly performed. The results are shown in Table 24 to Table 26 below.

Table 24

*Staphylococcus aureus*

| | | Bacterial concentration (CFU/mL) | | | | | |
|---|---|---|---|---|---|---|---|
| | | $10^5$ | $10^4$ | $10^3$ | $10^2$ | $10^1$ | $10^0$ |
| PIPC untreated | Present process | + | + | + | + | + | − |
| | Blood culture | + | + | + | + | + | − |
| PIPC treated | Present process | d | + | + | + | − | − |
| | Blood culture | d | + | + | − | − | − |

d: not performed

Table 25

*Staphylococcus epidermidis*

| | | Bacterial concentration (CFU/mL) | | | | | |
|---|---|---|---|---|---|---|---|
| | | $10^5$ | $10^4$ | $10^3$ | $10^2$ | $10^1$ | $10^0$ |
| PIPC untreated | Present process | + | + | + | + | − | − |
| | Blood culture | + | + | + | + | + | − |
| PIPC treated | Present process | d | + | + | + | − | − |
| | Blood culture | d | − | − | − | − | − |

d: not performed

Table 26

*Enterococcus faecalis*

| | | Bacterial concentration (CFU/mL) | | | | | |
|---|---|---|---|---|---|---|---|
| | | $10^5$ | $10^4$ | $10^3$ | $10^2$ | $10^1$ | $10^0$ |
| PIPC untreated | Present process | + | + | + | + | − | − |
| | Blood culture | + | + | + | + | + | − |
| PIPC treated | Present process | d | + | + | + | − | − |
| | Blood culture | d | + | + | − | − | − |

d: not performed

Apparently from the results described above, in the tests performed using the bacteria subjected to a treatment with an antibiotic, the bacteria could not be detected in the blood culture process even in an amount of bacteria with which the detection was enabled in instances where the treatment with the antibiotic was not conducted, however the bacteria could be detected without being affected by the antibiotic through the use of Hybrizep.

Example 27

Determination of Sensitivity Test

Among the performance tests according to the present invention, availability of the digested sample in the sensitivity tests was examined. Each operation process herein was conducted according to the procedure described in Examples 2-11.

Digested samples used were SA digested sample, SE digested sample, PA digested sample, EF digested sample and EK digested sample. Each digested sample was produced by the process described in Example 17. SA digested sample had the phagocytosis rate of 29% and cell number of $1.05\times10^5$ cells/well, and was determined as adequate . The SE digested sample had the phagocytosis rate of 47% and cell number of $1.51\times10^5$ cells/well, and

was determined as adequate . PA digested sample had the phagocytosis rate of 19% and cell number of 1.99 $\times 10^5$ cells /well , and was determined as adequate . EF digested sample had the phagocytosis rate of 33% and cell number of 1.25 $\times 10^5$ cells/well, and was determined as adequate . EK digested sample had the phagocytosis rate of 19% and cell number of 1.13 $\times 10^5$ cells/well, and was determined as adequate .

As shown in Fig. 12, tests were performed according to the process described in Examples 2-3, using the slide glass including the digested sample smeared thereon.

Each of SA, SE, PA, EF and EK digested samples was subjected to tests of three times per single kit, and the tests were performed for 3 kits. Thus, as shown in Table 27 and Fig. 13 (a) to (e), the bacteria could be detected in all of the digested samples. Hence, it was proven that the digested samples were useful in the sensitivity test in the process demonstrated in Examples 1-11. Therefore, standard of the sensitivity test process of demonstrated in Examples 1-11 was defined as one which enables detection of a signal when the test was performed according the procedure described in Examples 2-11 using the digested sample of a known bacterium.

Table 27

| Digested Samples | Trial time(s) | Kit 1 | Kit 2 | Kit 3 |
|---|---|---|---|---|
| SA Digested Sample | 1 | SA detected | SA detected | SA detected |
| | 2 | SA detected | SA detected | SA detected |
| | 3 | SA detected | SA detected | SA detected |
| SE Digested Sample | 1 | SE detected | SE detected | SE detected |
| | 2 | SE detected | SE detected | SE detected |
| | 3 | SE detected | SE detected | SE detected |
| PA Digested Sample | 1 | PA detected | PA detected | PA detected |
| | 2 | PA detected | PA detected | PA detected |
| | 3 | PA detected | PA detected | PA detected |
| EF Digested Sample | 1 | EF detected | EF detected | EF detected |
| | 2 | EF detected | EF detected | EF detected |
| | 3 | EF detected | EF detected | EF detected |
| EK Digested Sample | 1 | EK detected | EK detected | EK detected |
| | 2 | EK detected | EK detected | EK detected |
| | 3 | EK detected | EK detected | EK detected |

Example 28

Determination of Specificity Test

Among the performance tests, availability of the digested sample in the specificity tests was examined. Each operation process herein was conducted according to the procedure described in Examples 2-11.

Digested samples used were SA digested sample, SE digested sample, PA digested sample, EF digested sample and EK digested sample. Each digested sample was produced by the process described in Example 17. SA digested sample had the phagocytosis rate of 29% and cell number of $1.05\times 10^5$ cells/well, and was determined as adequate . The SE digested sample had the phagocytosis rate of 47% and cell number of $1.51\times 10^5$ cells/well, and was determined as adequate . PA digested sample had the phagocytosis rate of 19% and cell number of 1.99 $\times 10^5$ cells/well, and was determined as adequate . EF digested sample had the phagocytosis rate of 33% and cell number of 1.25 $\times 10^5$ cells/well, and was determined as adequate . EK digested sample had the phagocytosis rate of 19% and cell number of 1.13 $\times 10^5$ cells/well, and was determined as adequate .

As shown in Fig. 12, tests were performed according to the procedure described in Examples 2-3, using the slide glass including the digested sample smeared thereon.

Each of SA, SE, PA, EF and EK digested samples was subjected to tests of three times per one probe included in single kit, and the tests were performed for 3 kits. Thus, as shown in Tables 28-31, an accurate signal could be detected for any of all the known bacterial digested samples. Fig. 14 (a) to (e) illustrates that SA digested sample can be specifically detected by only the probe (a) for detecting SA. Hence, it was proven that the digested samples were useful in the specificity test of the process demonstrated in Examples 1-11. Therefore, standard of the specificity test of the process demonstrated in Examples 1-11 was defined as one which enables detection of a signal for only the corresponding bacterial digested sample when the test was performed according the procedure described in Examples 2-11 using the digested sample of a known bacterium.

Table 28

| Kit 1 | Type of probe | | | | | | | | | | | | | | |
|---|---|---|---|---|---|---|---|---|---|---|---|---|---|---|---|
| Digested Samples | SA | | | SE | | | PA | | | EF | | | EK | | |
| | 1 | 2 | 3 | 1 | 2 | 3 | 1 | 2 | 3 | 1 | 2 | 3 | 1 | 2 | 3 |
| SA Digested Sample | + | + | + | - | - | - | - | - | - | - | - | - | - | - | - |
| SE Digested Sample | - | - | - | + | + | + | - | - | - | - | - | - | - | - | - |
| PA Digested Sample | - | - | - | - | - | - | + | + | + | - | - | - | - | - | - |
| EF Digested Sample | - | - | - | - | - | - | - | - | - | + | + | + | - | - | - |
| EK Digested Sample | - | - | - | - | - | - | - | - | - | - | - | - | + | + | + |

Table 29

| Kit 2 | Type of probe | | | | | | | | | | | | | | |
|---|---|---|---|---|---|---|---|---|---|---|---|---|---|---|---|
| Digested Samples | SA | | | SE | | | PA | | | EF | | | EK | | |
| | 1 | 2 | 3 | 1 | 2 | 3 | 1 | 2 | 3 | 1 | 2 | 3 | 1 | 2 | 3 |
| SA Digested Sample | + | + | + | - | - | - | - | - | - | - | - | - | - | - | - |
| SE Digested Sample | - | - | - | + | + | + | - | - | - | - | - | - | - | - | - |
| PA Digested Sample | - | - | - | - | - | - | + | + | + | - | - | - | - | - | - |
| EF Digested Sample | - | - | - | - | - | - | - | - | - | + | + | + | - | - | - |
| EK Digested Sample | - | - | - | - | - | - | - | - | - | - | - | - | + | + | + |

Table 30

| Kit 3 | Type of probe | | | | | | | | | | | | | | |
|---|---|---|---|---|---|---|---|---|---|---|---|---|---|---|---|
| Digested Samples | SA | | | SE | | | PA | | | EF | | | EK | | |
| | 1 | 2 | 3 | 1 | 2 | 3 | 1 | 2 | 3 | 1 | 2 | 3 | 1 | 2 | 3 |
| SA Digested Sample | + | + | + | - | - | - | - | - | - | - | - | - | - | - | - |
| SE Digested Sample | - | - | - | + | + | + | - | - | - | - | - | - | - | - | - |
| PA Digested Sample | - | - | - | - | - | - | + | + | + | - | - | - | - | - | - |
| EF Digested Sample | - | - | - | - | - | - | - | - | - | + | + | + | - | - | - |
| EK Digested Sample | - | - | - | - | - | - | - | - | - | - | - | - | + | + | + |

Table 31

| Kit | | Positive Control Probe | | | Negative Control Probe | | |
|---|---|---|---|---|---|---|---|
| 1 | Sample | 1 | 2 | 3 | 1 | 2 | 3 |
| | U937 cell | + | + | + | - | - | - |
| 2 | Sample | 1 | 2 | 3 | 1 | 2 | 3 |
| | U937 cell | + | + | + | - | - | - |
| 3 | Sample | 1 | 2 | 3 | 1 | 2 | 3 |
| | U937 cell | + | + | + | - | - | - |

Example 29

Determination of Reproducibility Test

Availability of the digested sample in the reproducibility tests was examined.

Each operation process herein was conducted according to the procedure described in Examples 2-11.

Digested samples used were SA digested sample, SE digested sample, PA digested sample, EF digested sample and EK digested sample. Each digested sample was produced by the process described in Example 17. SA digested sample had the phagocytosis rate of 29% and cell number of $1.05\times10^5$ cells/well, and was determined as adequate . SE digested sample had the phagocytosis rate of 47% and cell number of $1.51\times10^5$ cells/well, and was determined as adequate . PA digested sample had the phagocytosis rate of 19% and cell number of $1.99\times10^5$ cells/well, and was determined as adequate . EF digested sample had the phagocytosis rate of 33% and cell number of $1.25\times10^5$ cells/well, and was determined as adequate . EK digested sample had the phagocytosis rate of 19% and cell number of $1.13\times10^5$ cells/well, and was determined as adequate .

As shown in Fig. 12, tests were performed according to the procedure described in Examples 2-3, using the slide glass including the digested sample smeared thereon.

Each of SA, SE, PA, EF and EK digested samples was subjected to tests of three times per one probe included in single kit, and the tests were performed for 3 kits. Thus, as shown in Tables 32-35, an accurate signal could be detected for any of all the bacterial digested samples. Hence, it was proven that the digested samples were useful in applying the reproducibility test of the process demonstrated in Examples 1-11. Therefore, standard of the reproducibility test of the process demonstrated in Examples 1-11 was determined to comply with Examples 2-11 using a digested sample of a known bacterium, and was defined as one which leads the identical effects when the specificity tests are repeated three times at the same time.

Table 32

| Kit 1 | Type of probe | | | | | | | | | | | | | | |
|---|---|---|---|---|---|---|---|---|---|---|---|---|---|---|---|
| Digested Samples | SA | | | SE | | | PA | | | EF | | | EK | | |
| | 1 | 2 | 3 | 1 | 2 | 3 | 1 | 2 | 3 | 1 | 2 | 3 | 1 | 2 | 3 |
| SA Digested Sample | + | + | + | - | - | - | - | - | - | - | - | - | - | - | - |
| SE Digested Sample | - | - | - | + | + | + | - | - | - | - | - | - | - | - | - |
| PA Digested Sample | - | - | - | - | - | - | + | + | + | - | - | - | - | - | - |
| EF Digested Sample | - | - | - | - | - | - | - | - | - | + | + | + | - | - | - |
| EK Digested Sample | - | - | - | - | - | - | - | - | - | - | - | - | + | + | + |

Table 33

| Kit 2 | Type of probe | | | | | | | | | | | | | | |
|---|---|---|---|---|---|---|---|---|---|---|---|---|---|---|---|
| Digested Samples | SA | | | SE | | | PA | | | EF | | | EK | | |
| | 1 | 2 | 3 | 1 | 2 | 3 | 1 | 2 | 3 | 1 | 2 | 3 | 1 | 2 | 3 |
| SA Digested Sample | + | + | + | - | - | - | - | - | - | - | - | - | - | - | - |
| SE Digested Sample | - | - | - | + | + | + | - | - | - | - | - | - | - | - | - |
| PA Digested Sample | - | - | - | - | - | - | + | + | + | - | - | - | - | - | - |
| EF Digested Sample | - | - | - | - | - | - | - | - | - | + | + | + | - | - | - |
| EK Digested Sample | - | - | - | - | - | - | - | - | - | - | - | - | + | + | + |

Table 34

| Kit 2 | Type of probe | | | | | | | | | | | | | | |
|---|---|---|---|---|---|---|---|---|---|---|---|---|---|---|---|
| Digested Samples | SA | | | SE | | | PA | | | EF | | | EK | | |
| | 1 | 2 | 3 | 1 | 2 | 3 | 1 | 2 | 3 | 1 | 2 | 3 | 1 | 2 | 3 |
| SA Digested Sample | + | + | + | - | - | - | - | - | - | - | - | - | - | - | - |
| SE Digested Sample | - | - | - | + | + | + | - | - | - | - | - | - | - | - | - |
| PA Digested Sample | - | - | - | - | - | - | + | + | + | - | - | - | - | - | - |
| EF Digested Sample | - | - | - | - | - | - | - | - | - | + | + | + | - | - | - |
| EK Digested Sample | - | - | - | - | - | - | - | - | - | - | - | - | + | + | + |

Table 35

| Kit | | Positive Cntrol Probe | | | Negative Control Probe | | |
|---|---|---|---|---|---|---|---|
| 1 | Sample | 1 | 2 | 3 | 1 | 2 | 3 |
| | U937 cell | + | + | + | - | - | - |
| 2 | Sample | 1 | 2 | 3 | 1 | 2 | 3 |
| | U937 cell | + | + | + | - | - | - |
| 3 | Sample | 1 | 2 | 3 | 1 | 2 | 3 |
| | U937 cell | + | + | + | - | - | - |

[Industrial Applicability]

According to the present invention, a phagocytotic function of a phagocyte can be evaluated *in vitro*, and an experimental model is stably provided which can be utilized for a variety of objects such as evaluation of immune functions, evaluation of efficiency of differentiation of a phagocyte, screening of a modulator of phagocytotic functions, determination of effects, guidelines for dosage regimen in clinical tests, markers for the diagnosis of infectious diseases, performance tests of kit and the like.

## Claims

1. A digested phagocyte prepared by contacting *in vitro* a phagocyte with a foreign microorganism and isolating the phagocyte so contacted.

2. The digested phagocyte according to claim 1 wherein a turbidity of bacterial liquid (O.D. = 600 nm) of the foreign microorganism used for *in vitro* contact between the phagocyte and the foreign microorganism is 0.01 to 0.03.

3. The digested phagocyte according to Claim 1 or 2 wherein a density of the phagocyte digested with the foreign microorganism is $1\times10^4$ cells/μl to $5\times10^4$ cells/μl.

4. The digested phagocyte according to any one of Claims 1-3 wherein said foreign microorganism is a gram negative bacterium.

5. The digested phagocyte according to any one of claims 1-3 wherein said foreign microorganism is one or more microorganism selected from the group consisting of *Staphylococcus aureus, Staphylococcus epidermidis, Enterococcus faecalis, Pseudomonas aeruginosa, Escherichia coli* and *Candida albicans*, and a mixture thereof.

6. A process for producing a phagocyte digested with a foreign microorganism comprising the steps of:

   contacting *in vitro* a phagocyte with a foreign microorganism; and
   isolating the phagocyte.

7. The process according to claim 6 wherein a turbidity of bacterial liquid (O.D. = 600 nm) of the foreign microorganism used for *in vitro* contact between the phagocyte and the foreign microorganism is 0.01 to 0.03.

8. The process according to Claim 6 or 7 wherein a density of the phagocyte digested with the foreign microorganism is $1\times10^4$ cells/μl to $5\times10^4$ cells/μl.

9. The process according to any one of Claims 6-8 wherein said foreign microorganism is a gram negative bacterium.

10. The process according to any one of Claims 6-8 wherein said foreign microorganism is one or more microorganism selected from the group consisting of *Staphylococcus aureus, Staphylococcus epidermidis, Enterococcus faecalis, Pseudomonas aeruginosa, Escherichia coli* and *Candida albicans*, and a mixture thereof.

11. A process for detecting and/or identifying a digested foreign microorganism comprising the steps of:

    fixing the phagocyte digested with a foreign microorganism according to any one of Claims 1-5;
    treating to promote permeability of the cell membrane of the phagocyte;
    treating to expose DNA of the foreign microorganism existing in the phagocyte;
    *in situ* hybridizing under a stringent condition between a DNA probe which can detect hybridization and the DNA; and
    detecting and/or identifying the digested foreign microorganism by the resulting signal.

12. A process for evaluating a phagocytotic function against a foreign microorganism comprising the steps of:

    fixing the phagocyte digested with a foreign microorganism according to any one of Claims 1 to 5;
    treating to promote permeability of the cell membrane of the phagocyte;
    treating to expose DNA of the foreign microorganism existing in the phagocyte;
    *in situ* hybridizing under a stringent condition between a DNA probe which can detect hybridization and the DNA; and
    identifying by the resulting signal the phagocytosis and/or killing ability of the phagocyte against the foreign microorganism.

13. The process according to Claim 11 or 12 wherein said process includes at least one aspect of:

    (1) the density (X cells/ml) of the phagocytes to be fixed is $5\times10^6$ cells/ml $<$ X cells/ml $<$ $1\times10^8$ cells/ml;
    (2) in said exposing step of the DNA, lysostafin having the titer of 1 unit/ml to 1,000 unit/ml is used;

(3) in said exposing step of the DNA, lysozyme having the titer of 1,000 unit/ml to 1,000,000 unit/ml is used;
(4) in said exposing step of the DNA, N-acetylmuramidase having the titer of 10 unit/ml to 10,000 unit/ml is used;
(5) in said exposing step of the DNA, zymolase having the titer of 50 unit/ml to 500 unit/ml is used;
(6) in said *in situ* hybridization step, a surfactant is used;
(7) said DNA probe for detection is one or more DNA probe having the chain length of 350 to 600 base length; and
(8) the concentration of said DNA probe for detection is 0.1 ng/μl to 2.2 ng/μl.

**14.** The process according to Claim 13 wherein one or more enzyme selected from lysostafin, lysozyme, N-acetylmuramidase and zymolase is used in said exposing step of the DNA, with the titer of lysostafin being 10 unit/ml to 100 unit/ml; the titer of lysozyme being 10,000 unit/ml to 100,000 unit/ml; the titer of N-acetylmuramidase being 100 unit/ml to 1,000 unit/ml; and the titer of zymolase being 100 unit/ml to 500 unit/ml.

**15.** The process according to any one of Claims 11 to 14 wherein an enzyme is used in said exposing step of the DNA, and wherein the temperature to allow the reaction of the enzyme is 26°C to 59°C, with the time period of the reaction of the enzyme being 15 minutes to 120 minutes.

**16.** The process according to any one of Claims 11 to 15 wherein a substance for retaining the morphology of the phagocyte is additionally used in said exposing step of the DNA.

**17.** The process according to Claim 16 wherein said substance is phenylmethylsulfonyl fluoride.

**18.** The process according to Claim 17 wherein the concentration of said phenylmethylsulfonyl fluoride is 10 μmol/l to 10 mmol/l.

**19.** The process according to any one of Claims 16 to 18 wherein said substance is a substance dissolved in dimethylsulfoxide.

**20.** The process according to Claim 19 wherein the concentration of said dimethylsulfoxide is less than 5%.

**21.** The process according to any one of Claims 11 to 20 wherein the DNA and the DNA probe is hybridized in the presence of a surfactant in said *in situ* hybridization step.

**22.** The process according to Claim 21 wherein said surfactant is an anion surfactant.

**23.** The process according to Claim 22 wherein said anion surfactant is sodium dodecylsulfate.

**24.** The process according to any one of Claims 11 to 23 wherein the temperature to allow the hybridization reaction is 25°C to 50°C, with the time period of the hybridization reaction being 30 minutes to 900 minutes in said *in situ* hybridization step.

**25.** A process for evaluating a phagocytotic function against a foreign microorganism comprising the steps of:

fixing the digested phagocyte according to any one of Claims 1 to 5;
staining the phagocyte with a dye; and
identifying the phagocytosis and/or killing ability of the phagocyte against the foreign microorganism by the detection through observation by microscopic examination on cell morphology which is characteristic in cells during or after phagocytosis.

**26.** A process for evaluating an immune function comprising the steps of:

isolating phagocytes from a subject;
evaluating a function of the phagocytes using the process for evaluating a phagocytotic function according to any one of Claims 12 to 25; and
evaluating the immune function of the subject by comparing the evaluation result to that of the function of normal phagocytes.

**27.** The process according to Claim 26 wherein said immune function is a phagocytotic ability of a microorganism by a leukocyte.

**28.** The process according to claim 27 wherein said immune function is a phagocytotic ability against a microorganism by a leukocyte of a patient who received the radiation exposure or the administration of an anticancer agent.

**29.** A process for evaluating differentiation efficiency into a phagocyte comprising the steps of:

evaluating a phagocytotic function against a foreign microorganism according to any one of Claims 12 to 25; and
evaluating the phagocytotic function in a time dependent manner to identify the alteration.

**30.** A process of the evaluation for determining an effect of a modulator of phagocytotic function comprising the steps of:

allowing phagocytosis by incubating a suspension of a foreign microorganism and phagocytes in the presence and absence of a phagocytotic function modulator; and
comparing the phagocytotic function in the presence and absence of said phagocytotic function modulator using the process for evaluating a phagocytotic function against a foreign microorganism according to any one of Claims 12 to 25.

**31.** A process for screening a modulator of phagocytotic function comprising the steps of:

allowing phagocytosis by incubating a suspension of a foreign microorganism and phagocytes in the presence and absence of a candidate agent supposed to have a modulatory action toward the phagocytotic function; and
comparing the phagocytotic function in the presence and absence of said agent using the process for evaluating a phagocytotic function against a foreign microorganism according to any one of Claims 12 to 25.

**32.** A clinical testing process comprising the steps of:

obtaining phagocytes from a subject prior to and following the administration of an agent to the subject;
evaluating a function of the phagocyte using the process for evaluating a phagocytotic function according to any one of Claims 12 to 25; and
examining a dosage regimen of the agent judging from the effect of the agent determined on the basis of the evaluation result.

**33.** A performance testing process of a kit for evaluating a phagocytotic function which comprises fixing phagocytes, treating to promote permeability of the cell membranes of the phagocytes, treating to expose the DNA of a foreign microorganism in the phagocytes, *in situ* hybridize under a stringent condition between the DNA and a DNA probe which can detect hybridization; and evaluating the phagocytotic function by the resulting signal, said kit has;

(1) the foreign microorganism,
(2) at least one or more enzyme(s) selected from the group consisting of lysostafin, lysozyme, N-acetylmuramidase and zymolase used in said exposing step of the DNA, and
(3) one or more DNA probe(s) for detection,

said process is **characterized in that** the digested phagocyte according to any one of Claims 1 to 5 is used.

**34.** A performance testing process of a kit for detecting and/or identifying a foreing microorganism which comprises obtaining phagocytes from a clinical specimen containing phagocytes derived from a living body, fixing the phagocytes so obtained, treating to promote permeability of the cell membranes of the phagocytes, treating to expose the DNA of the foreign microorganism predicted as existing in the phagocytes, *in situ* hybridizing under a stringent condition between the DNA and a DNA probe which can detect hybridization, and detecting and/or identifying the foreign microorganism by the resulting signal,
the process is **characterized in that** the digested phagocyte according to any one of Claims 1 to 5 is used.

**35.** The performance testing process according to Claim 33 or 34 wherein said performance test is a sensitivity test, a specificity test or a reproducibility test.

**36.** The performance testing process according to Claim 33 or 34 wherein the digested phagocyte according to any one of Claims 1 to 5 is used as a positive control.

**37.** The process according to any one of Claims 11 to 36 wherein the process further comprises a step prior to said fixing step to put the digested phagocyte onto a solid support which is a slide glass coated with 3-aminopropyltriethoxysilane.

**38.** The process according to any one of Claims 11 to 37 wherein a dye for clarifying the contrast between the signal and the cell is used upon the detection of said signal.

**39.** The process according to any one of Claims 11 to 38 wherein said phagocyte is from blood.

**40.** A kit for evaluating a phagocytotic function by fixing the digested phagocytes according to any one of Claims 1 to 5, treating to promote permeability of the cell membranes of the phagocytes, treating to expose DNA of the foreign microorganism in the phagocytes, in situ hybridizing under a stringent condition between the DNA and a DNA probe which can detect hybridization; and evaluating the phagocytotic function by the resulting signal, wherein said kit has;

(1) the foreign microorganism,
(2) at least one or more enzyme(s) selected from the group consisting of lysostafin, lysozyme, N-acetylmuramidase and zymolase used in said exposing step of the DNA, and
(3) one or more DNA probe(s) for detection.

FIGURE 1

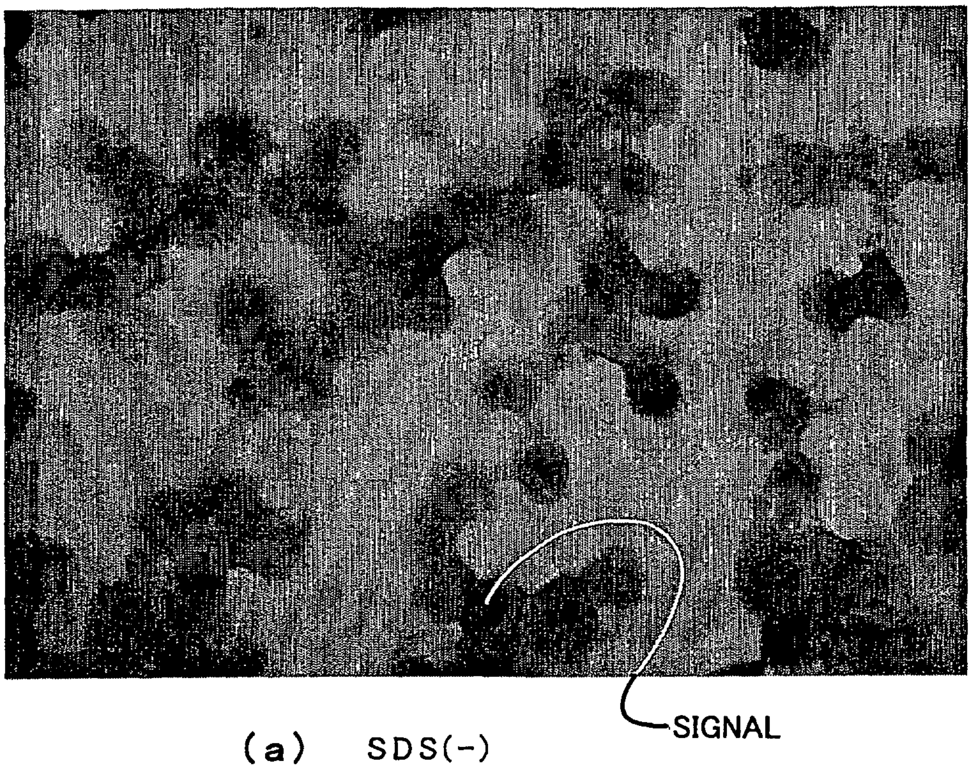


(a) SDS(-)

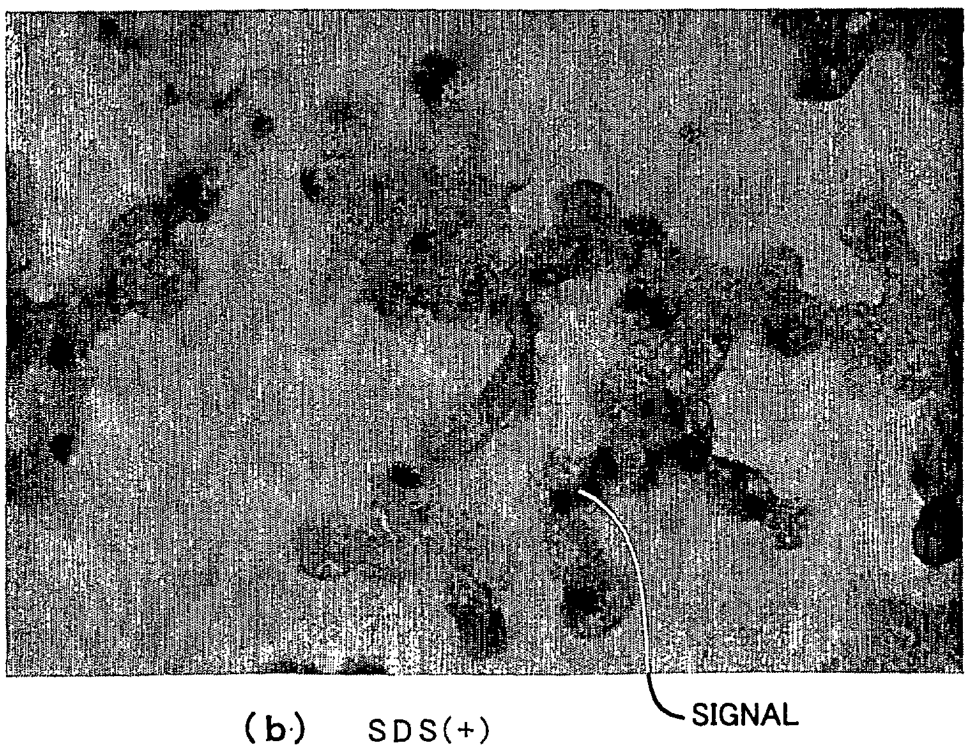


(b) SDS(+)

FIGURE 2

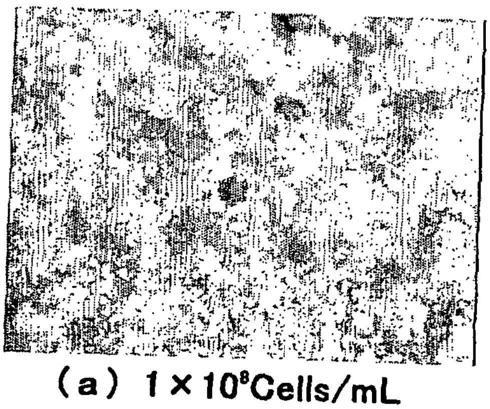

(a) $1\times10^{8}$ Cells/mL

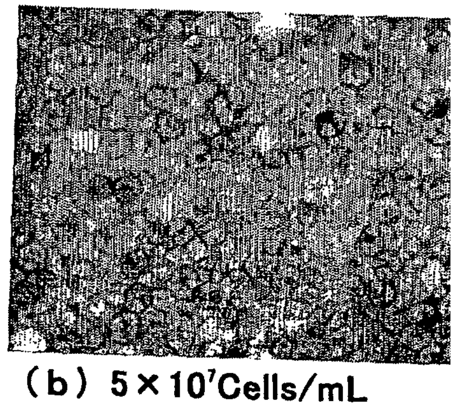

(b) $5\times10^{7}$ Cells/mL

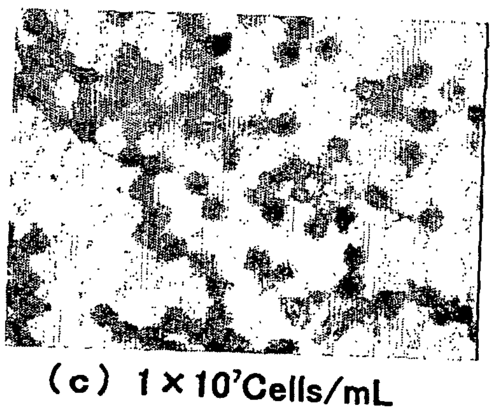

(c) $1\times10^{7}$ Cells/mL

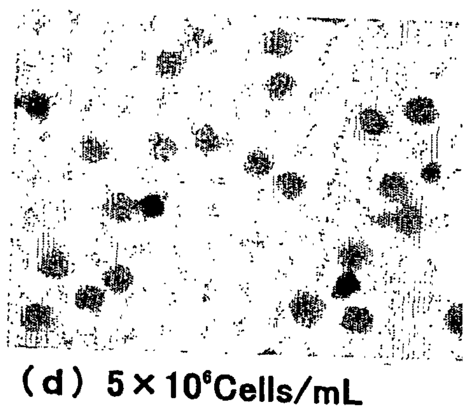

(d) $5\times10^{6}$ Cells/mL

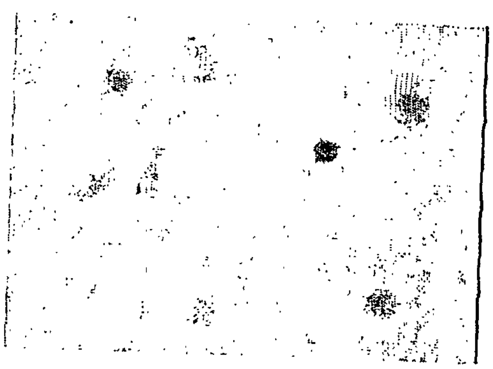

(e) $1\times10^{6}$ Cells/mL

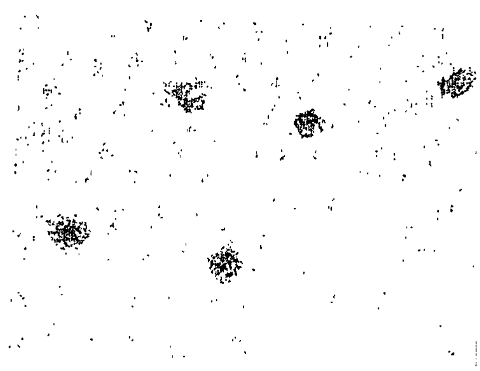

(f) $5\times10^{5}$ Cells/ml

# FIGURE 3

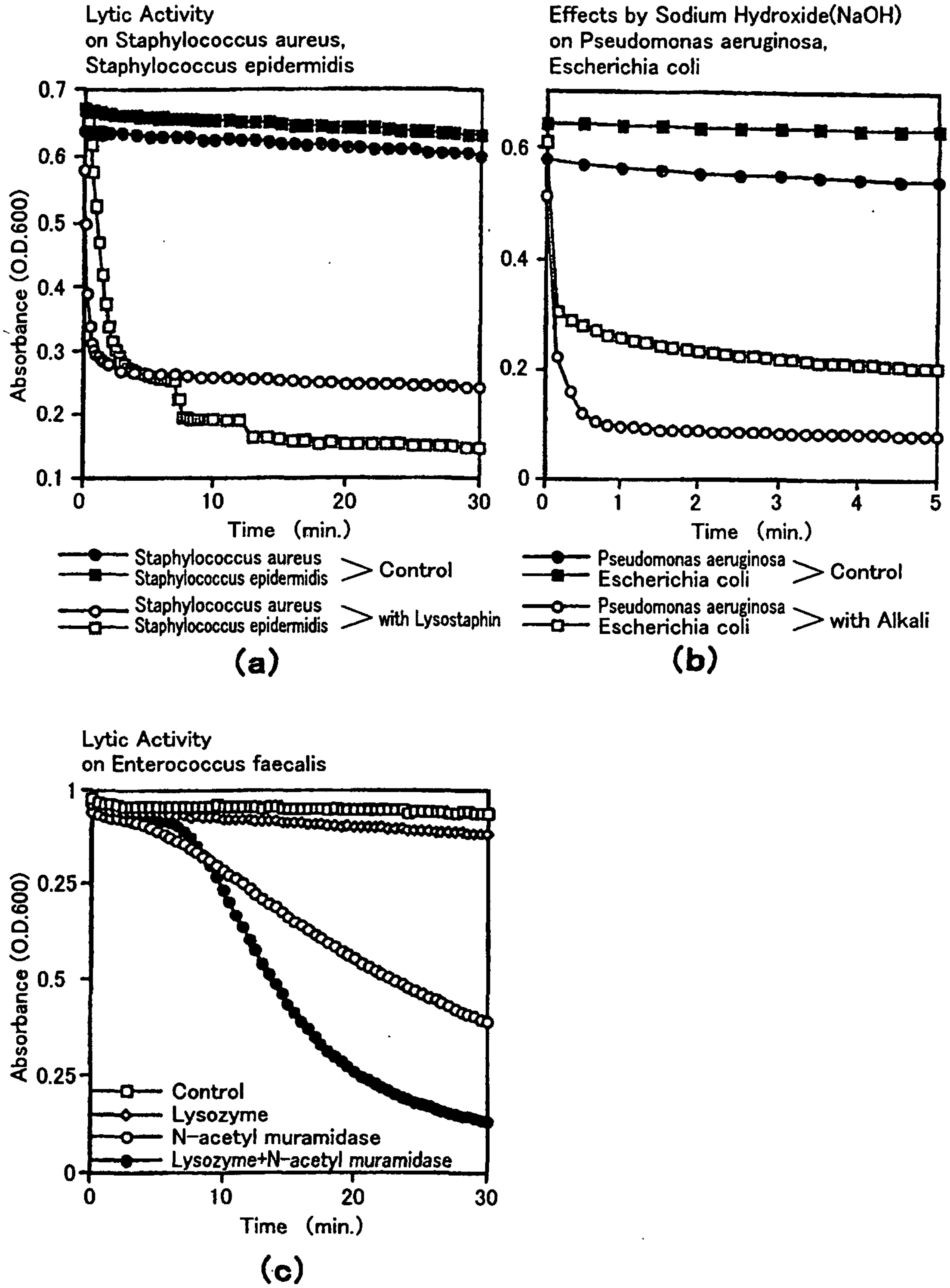
Lytic Activity
on Staphylococcus aureus,
Staphylococcus epidermidis
Absorbance (O.D.600)
0.7
0.6
0.5
0.4
0.3
0.2
0.1
0
10
20
30
Time (min.)
Staphylococcus aureus
Staphylococcus epidermidis
Control
Staphylococcus aureus
Staphylococcus epidermidis
with Lysostaphin
(a)
Effects by Sodium Hydroxide(NaOH)
on Pseudomonas aeruginosa,
Escherichia coli
0.6
0.4
0.2
0
1
2
3
4
5
Time (min.)
Pseudomonas aeruginosa
Escherichia coli
Control
Pseudomonas aeruginosa
Escherichia coli
with Alkali
(b)
Lytic Activity
on Enterococcus faecalis
Absorbance (O.D.600)
1
0.25
0.5
0.25
0
10
20
30
Time (min.)
Control
Lysozyme
N-acetyl muramidase
Lysozyme+N-acetyl muramidase
(c)

FIGURE 4

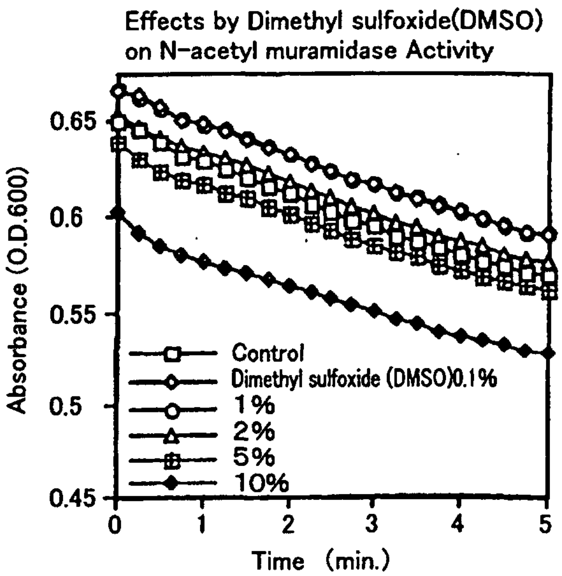
Effects by Dimethyl sulfoxide(DMSO)
on N-acetyl muramidase Activity
Absorbance (O.D.600)
0.65
0.6
0.55
0.5
0.45
Control
Dimethyl sulfoxide (DMSO)0.1%
1%
2%
5%
10%
0
1
2
3
4
5
Time (min.)

(a)

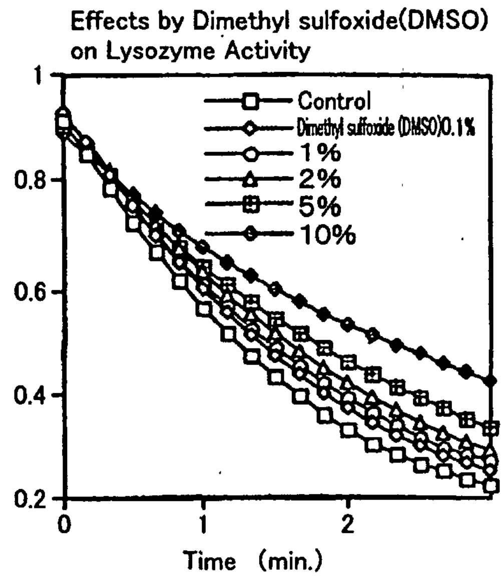
Effects by Dimethyl sulfoxide(DMSO)
on Lysozyme Activity
1
0.8
0.6
0.4
0.2
Control
Dimethyl sulfoxide (DMSO)0.1%
1%
2%
5%
10%
0
1
2
Time (min.)

(b)

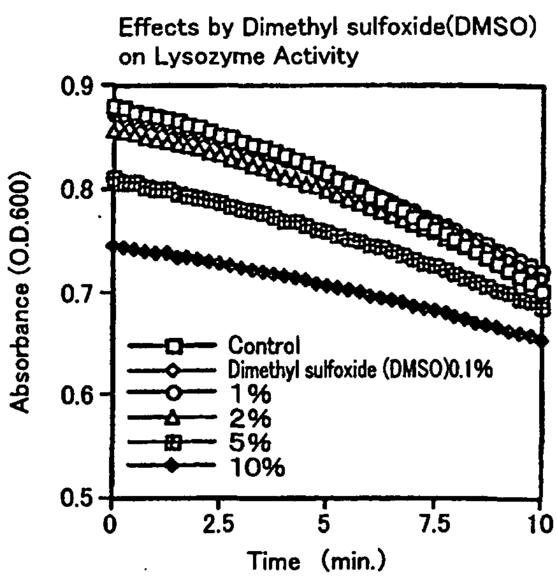
Effects by Dimethyl sulfoxide(DMSO)
on Lysozyme Activity
Absorbance (O.D.600)
0.9
0.8
0.7
0.6
0.5
Control
Dimethyl sulfoxide (DMSO)0.1%
1%
2%
5%
10%
0
2.5
5
7.5
10
Time (min.)

(c)

FIGURE 5

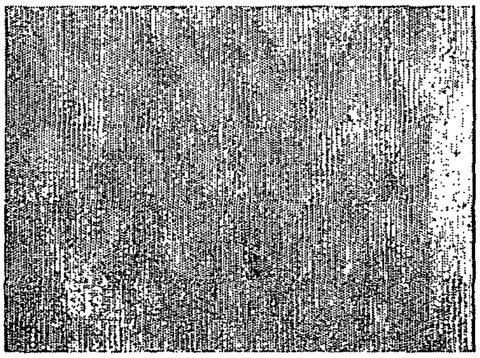

(a)

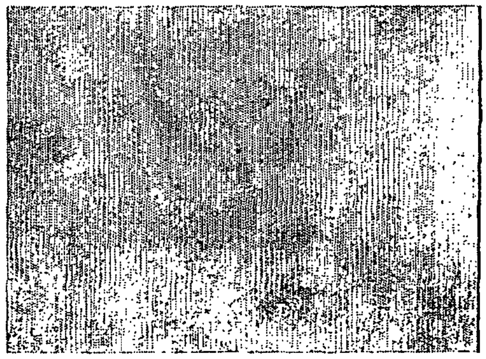

(b) PMSF 1 μ mol/mL

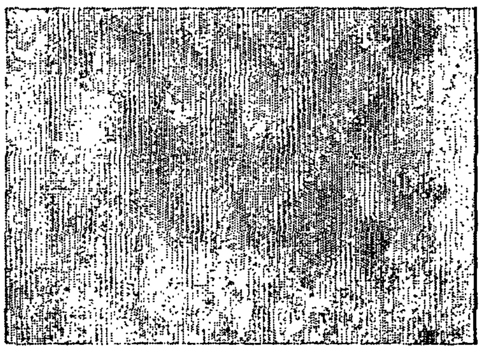

(c) PMSF 10 μ mol/mL

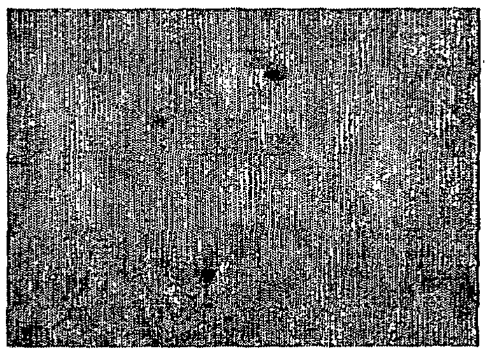

(d) PMSF 0.1mmol/mL

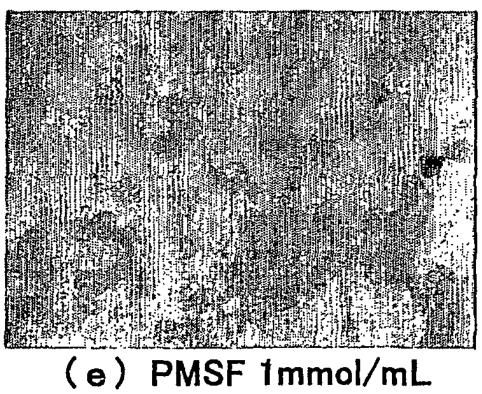

(e) PMSF 1mmol/mL

FIGURE 6

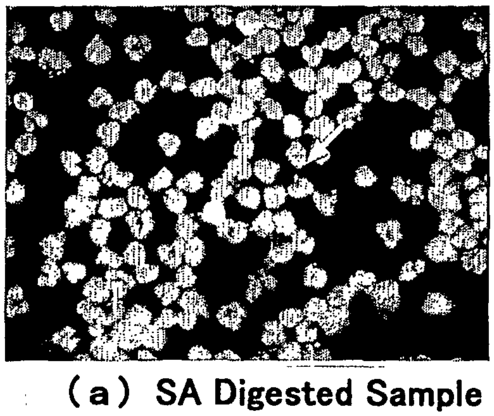

(a) SA Digested Sample

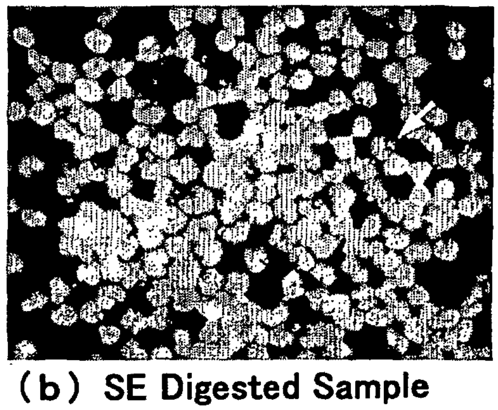

(b) SE Digested Sample

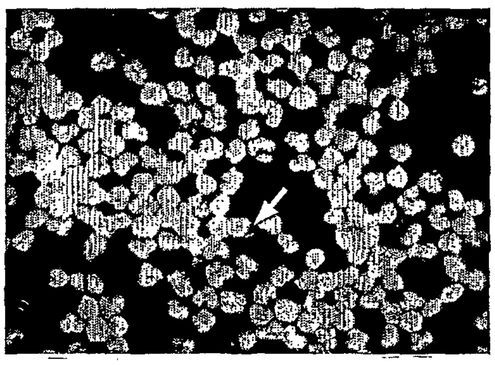

(c) PA Digested Sample

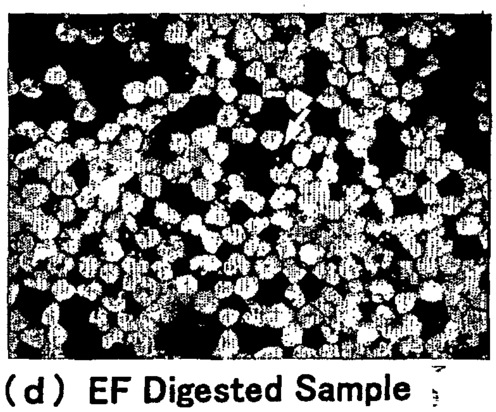

(d) EF Digested Sample

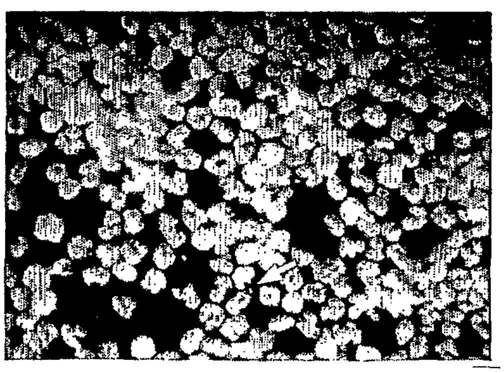

(e) EK Digested Sample

Arrow in the Photographs indicated the digested Bacteria.

FIGURE 7

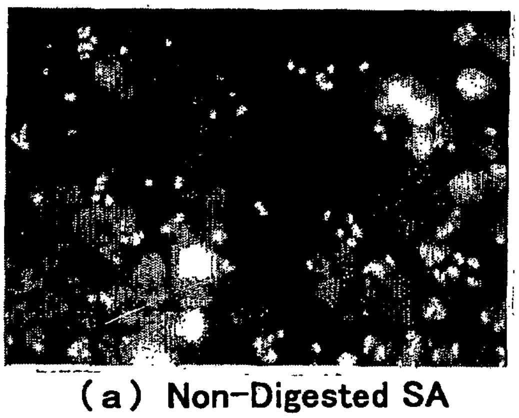

(a) Non-Digested SA

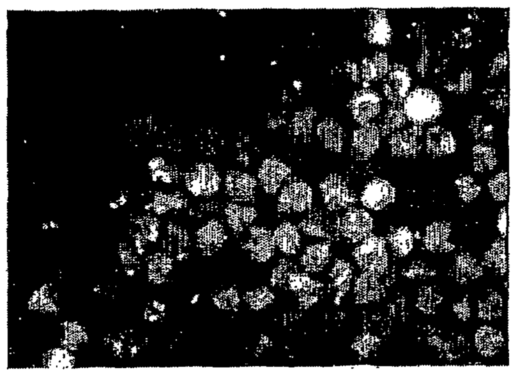

(b) Non-Digested EF

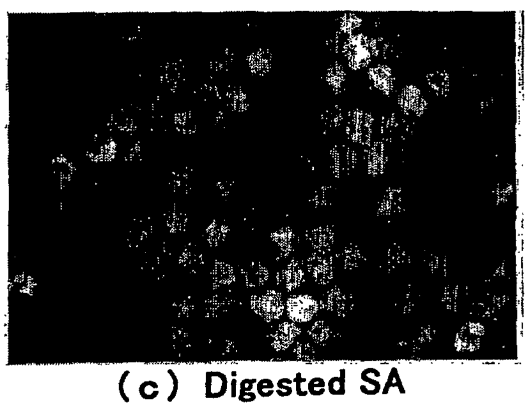

(c) Digested SA

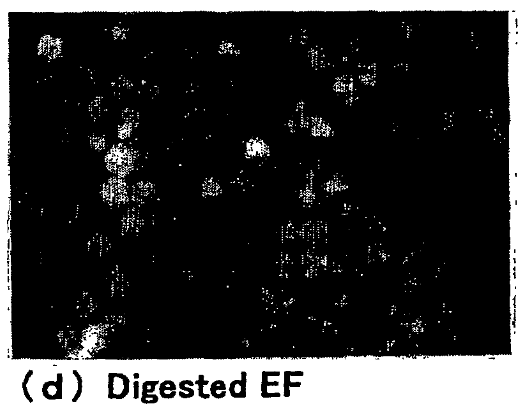

(d) Digested EF

FIGURE 8

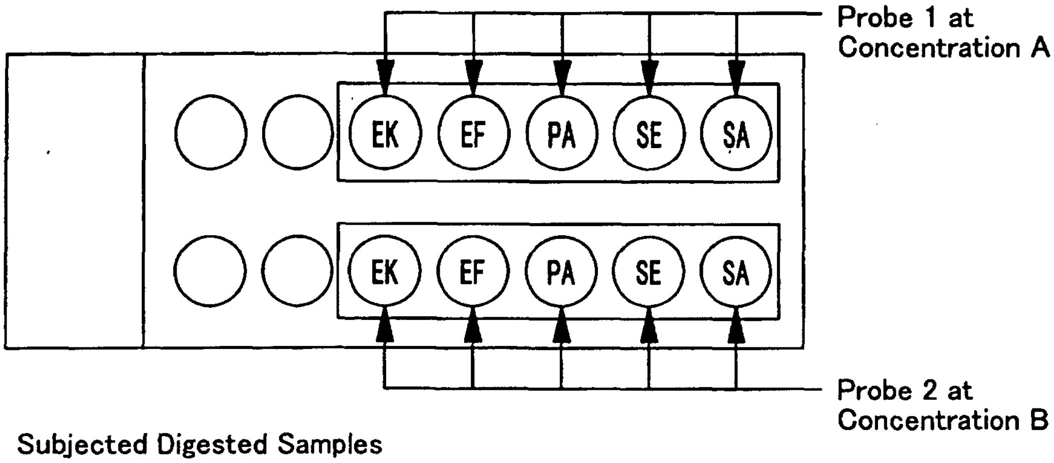


Subjected Digested Samples

SA : SA Digested Sample

SE : SE Digested Sample

PA : PA Digested Sample

EF : EF Digested Sample

EK : EK Digested Sample

FIGURE 9

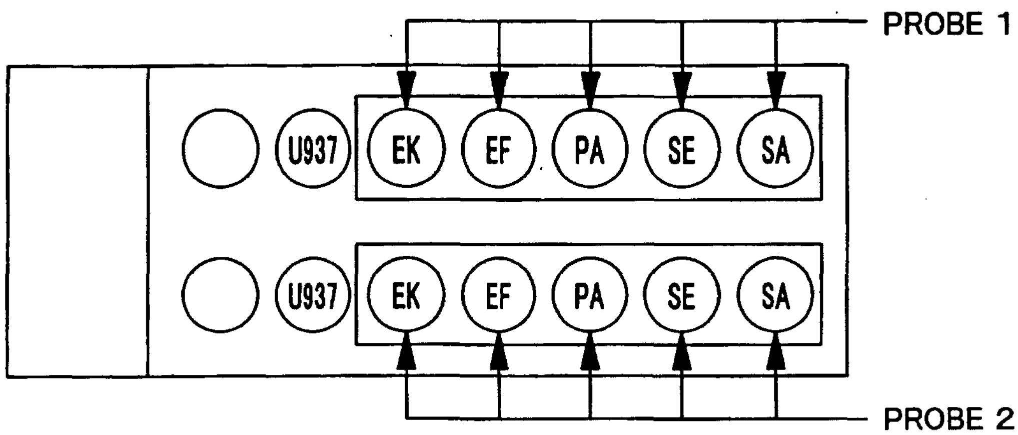


SA : SA Digested Sample
SE : SE Digested Sample
PA : PA Digested Sample
EF : EF Digested Sample
EK : EK Digested Sample

FIGURE 10

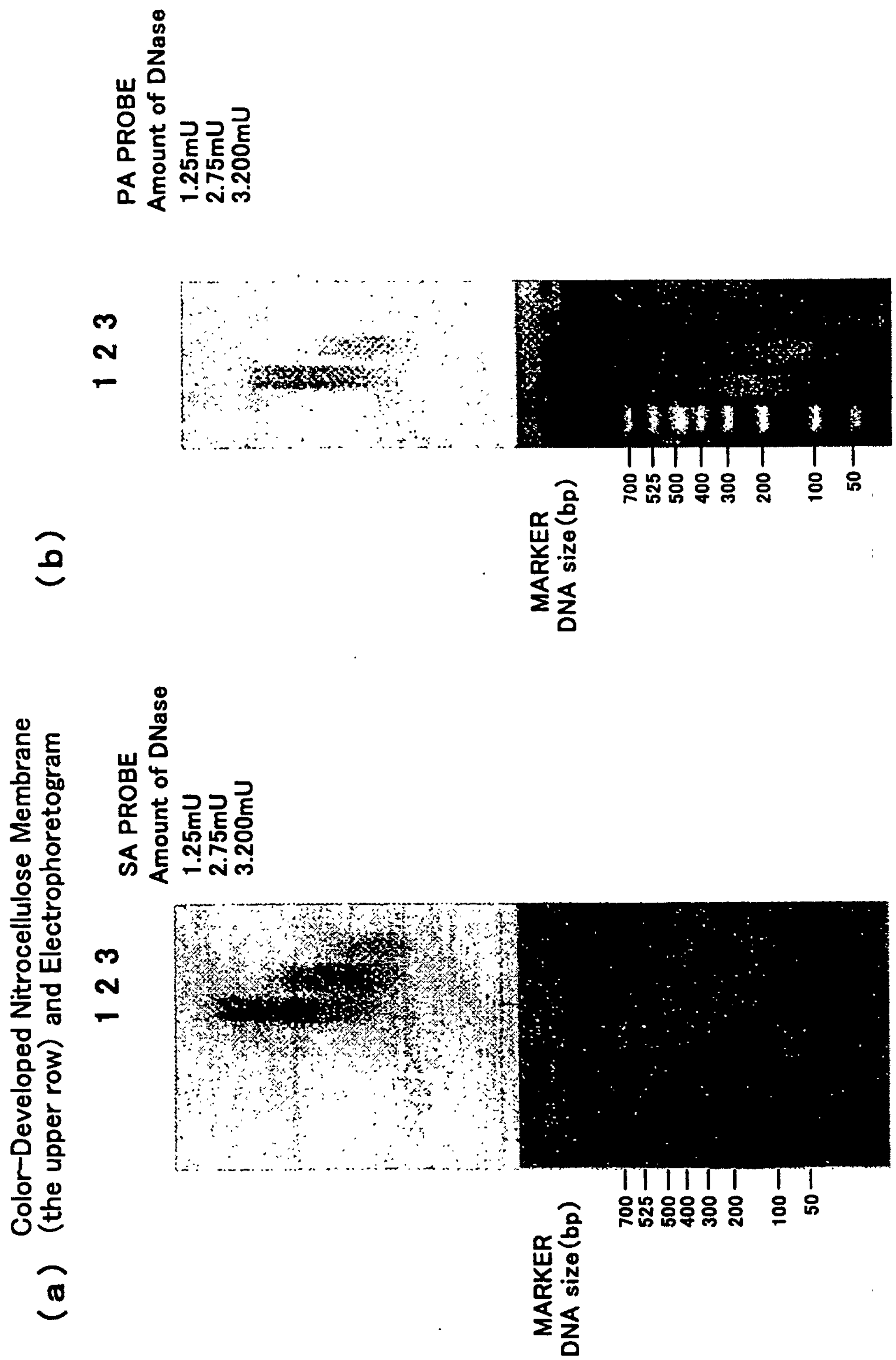
(a)
Color-Developed Nitrocellulose Membrane
(the upper row) and Electrophoretogram
1 2 3
SA PROBE
Amount of DNase
1.25mU
2.75mU
3.200mU
MARKER
DNA size (bp)
700
525
500
400
300
200
100
50
(b)
1 2 3
PA PROBE
Amount of DNase
1.25mU
2.75mU
3.200mU
MARKER
DNA size (bp)
700
525
500
400
300
200
100
50

FIGURE 11

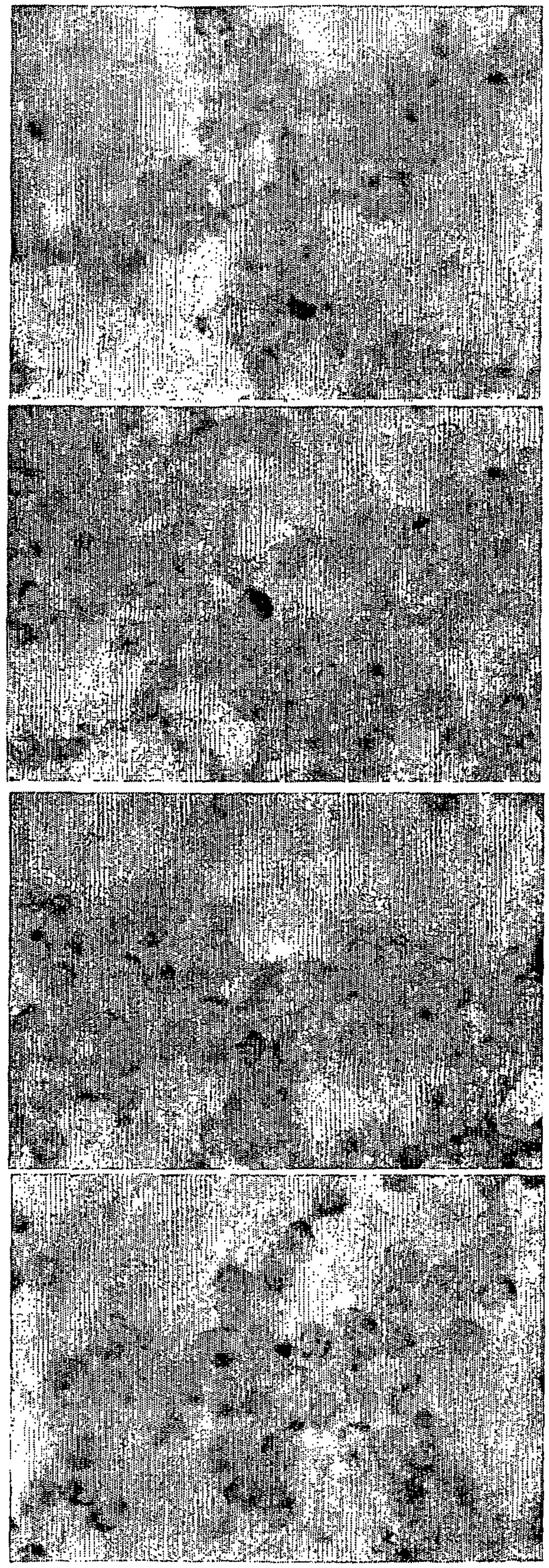

(a) EC-24

(b) EC-34

(c) EC-39

(d) Mix

FIGURE 12

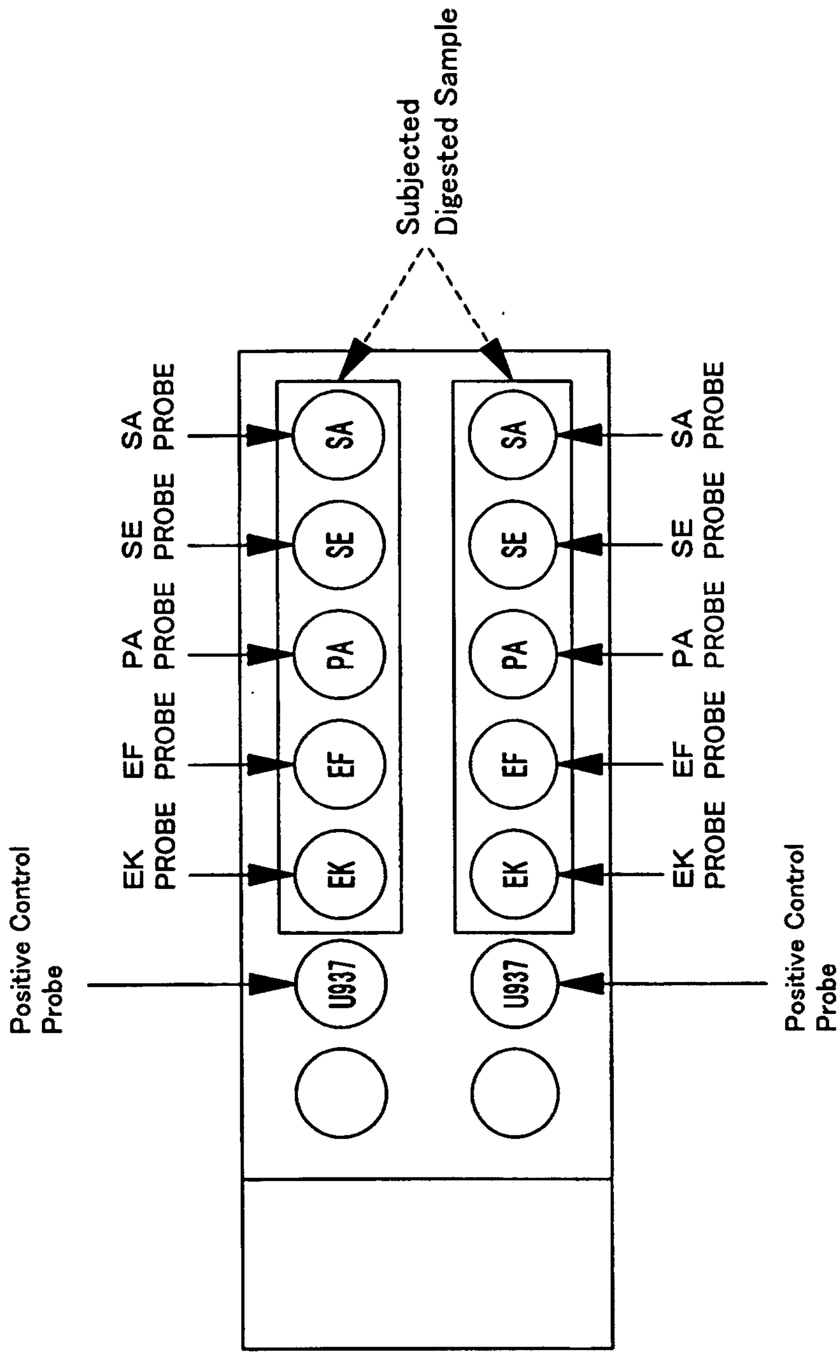
Subjected Digested Sample
SA PROBE
SE PROBE
PA PROBE
EF PROBE
EK PROBE
Positive Control Probe
SA
SE
PA
EF
EK
U937
SA
SE
PA
EF
EK
U937
SA PROBE
SE PROBE
PA PROBE
EF PROBE
EK PROBE
Positive Control Probe

FIGURE 13

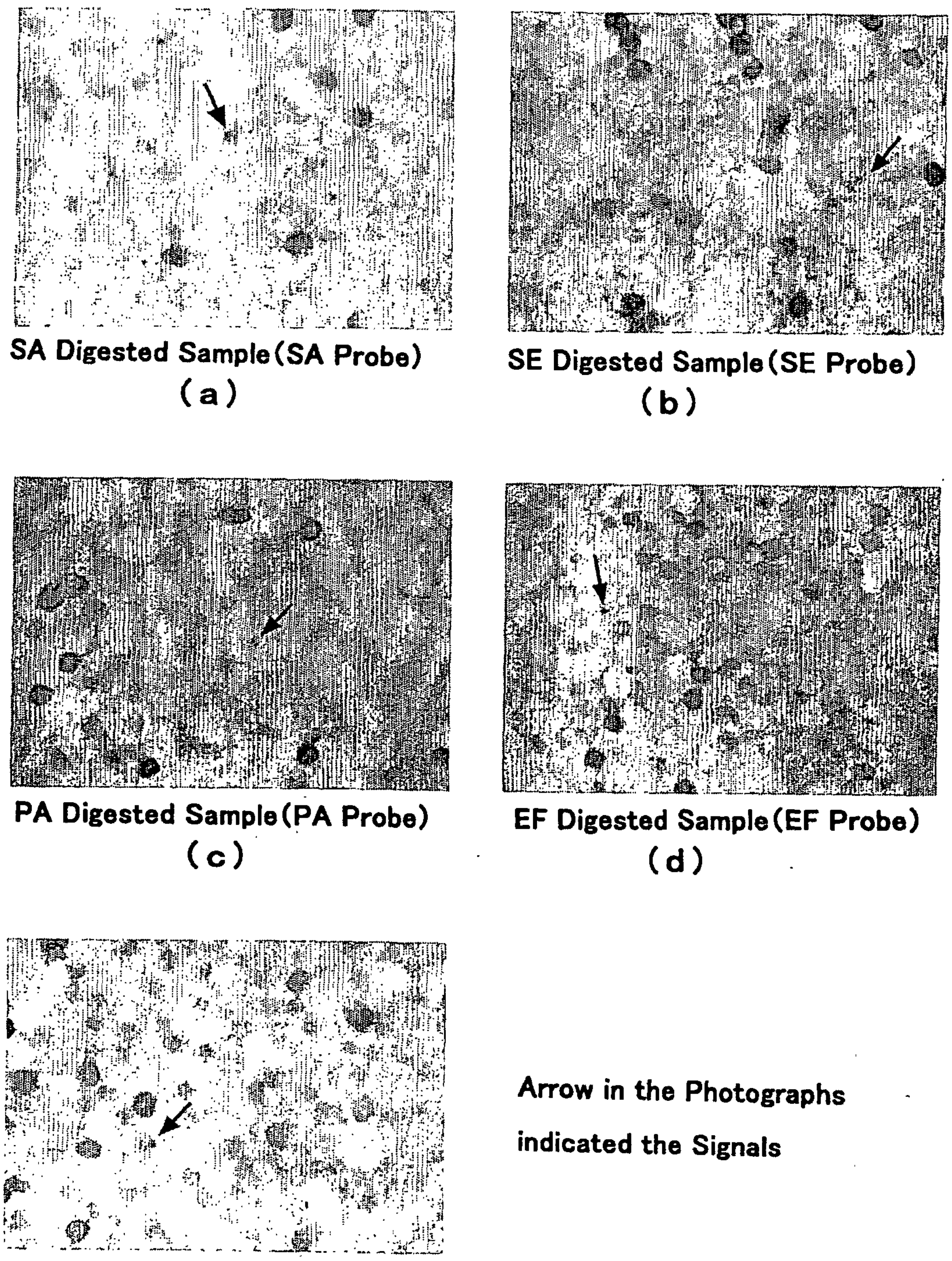

SA Digested Sample (SA Probe)
(a)

SE Digested Sample (SE Probe)
(b)

PA Digested Sample (PA Probe)
(c)

EF Digested Sample (EF Probe)
(d)

EK Digested Sample (EK Probe)
(e)

Arrow in the Photographs indicated the Signals

# FIGURE 14

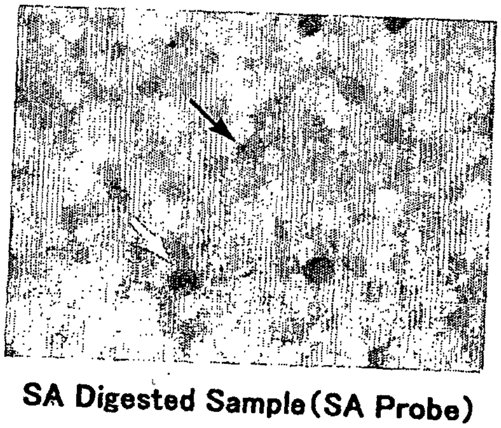

SA Digested Sample (SA Probe)
(a)

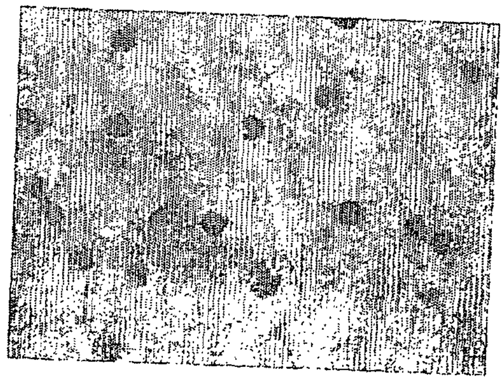

SE Digested Sample (SE Probe)
(b)

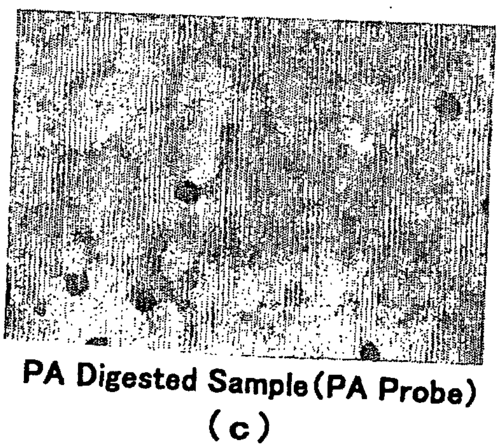

PA Digested Sample (PA Probe)
(c)

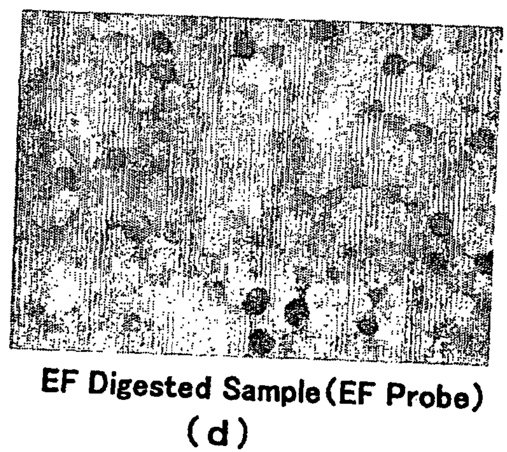

EF Digested Sample (EF Probe)
(d)

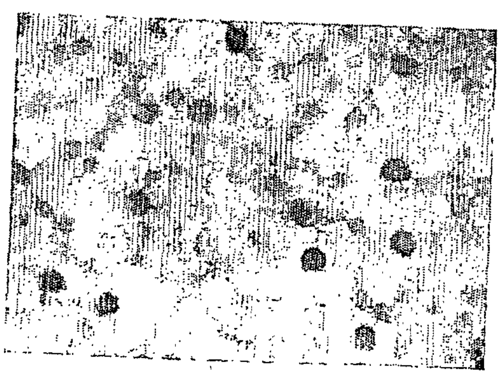

EK Digested Sample (EK Probe)
(e)

Black Arrow in the Photographs indicated the Signals.
Some Cells were well stained with Contrast Staining like those indicated by white Arrow in the Photographs.

## INTERNATIONAL SEARCH REPORT

International application No.
PCT/JP02/05106

A. CLASSIFICATION OF SUBJECT MATTER
Int.Cl[7] C12N15/09, C12N5/06, C12N5/10, C12Q1/68, G01N33/53

According to International Patent Classification (IPC) or to both national classification and IPC

B. FIELDS SEARCHED

Minimum documentation searched (classification system followed by classification symbols)
Int.Cl[7] C12N15/09, C12N5/06, C12N5/10, C12Q1/68, G01N33/53

Documentation searched other than minimum documentation to the extent that such documents are included in the fields searched

Electronic data base consulted during the international search (name of data base and, where practicable, search terms used)
BIOSIS(DIALOG), WPI(DIALOG)

C. DOCUMENTS CONSIDERED TO BE RELEVANT

| Category* | Citation of document, with indication, where appropriate, of the relevant passages | Relevant to claim No. |
|---|---|---|
| A | EP 159653 A1 (S.A. Innovi N.V.), 30 October, 1985 (30.10.85), & JP 60256377 A & US 4737455 A | 1-25,29-31, 33,40 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
20 August, 2002 (20.08.02)

Date of mailing of the international search report
03 September, 2002 (03.09.02)

Name and mailing address of the ISA/
Japanese Patent Office

Authorized officer

Facsimile No.

Telephone No.

Form PCT/ISA/210 (second sheet) (July 1998)

## INTERNATIONAL SEARCH REPORT

International application No.
PCT/JP02/05106

**Box I Observations where certain claims were found unsearchable (Continuation of item 1 of first sheet)**

This international search report has not been established in respect of certain claims under Article 17(2)(a) for the following reasons:

1. [X] Claims Nos.: 26-28, 32, 34-39
because they relate to subject matter not required to be searched by this Authority, namely:
Claims 26 to 28, 32 and 34 to 39 involve methods for treatment of the human body by surgery or therapy and thus relate to a subject matter which this International Searching Authority is not required, under the provisions of Article 17(2)(a)(i) of the PCT and Rule 39.1(iv) (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/05106

Continuation of Box No.I-1 of continuation of first sheet(1)

of the Regulations under the PCT, to search.

Form PCT/ISA/210 (extra sheet) (July 1998)